# EUROPEAN PATENT APPLICATION

(11) **EP 3 178 313 A1**
(43) Date of publication of application: **14.06.2017**
(21) Application number: 15198616.3
(22) Date of filing: 09.12.2015
(51) Int. Cl.: A01H 5/08, A01N 65/38, C12N 9/02, C12Q 1/68, C12N 15/82, A01N 27/00, A01N 31/04, A01N 43/20, C07C 13/23, C07D 303/14

(54) **GENE AND PROTEIN FOR THE SYNTHESIS OF OXIDIZED ZINGIBERENE DERIVATIVES**

(71) Applicant: Leibniz-Institut für Pflanzenbiochemie, 06120 Halle (DE)
(72) Inventor: The designation of the inventor has not yet been filed
(74) Representative: Blodig, Wolfgang

(57) **Abstract**

The invention provides a nucleic acid encoding an enzyme capable of producing 9-hydroxy-7-epi-zingiberene and 10,11-epoxy-9-hydroxy-7-epizingiberene from 7-epi-zingiberene. The invention further provides novel zingiberene derivatives that can be used for increasing pest resistance of plants.

SEQUENCE LISTING

<110>  Leibniz

<120>  Gene and protein for the synthesis of oxidized zingiberene
       derivatives

<130>  EAD-16911

<160>  36

<170>  PatentIn version 3.5

<210>  1
<211>  1398
<212>  DNA
<213>  Artificial Sequence

<220>
<223>  cDNA encoding zingiberene polyoxidase lacking membrane anchor

<400>  1
atgttgcctc ctggtccatg gaaattacct ttaattggaa gcatgtttca tttgttaggt      60

ggacttccac atcatgtttt tagagattta gcgaaaaaat atggaccaat tatgcacctt     120

caactaggtg aagtttctct agttgttatt acttctcctg acatggccaa acaaatacta     180

agaactcatg atctcgcttt tgcatctagg cccaagttcc tagttgggga gattctcttt     240

tataagtgga ctgatattct tttttctccc tatggtgatt actggaaaca aatgcgtaaa     300

atatgtctat tggaattgct tagcgcaaaa aatgttaagt cattcaactc tatacgacaa     360

gatgaagttc atcatatgat tgagtttttt cgatcatctt ctggtgaaac aattaatgta     420

acaaaaagga tttatcaatt ctcgagctct atgacatgta gatcagcatt tgggaaagta     480

ttcaaagagc aaaacaaact catagaactc gttaaagaag cgtcaggctt ttttaaagga     540

tttgatgtag ctgatatgtt tccatcacta aaatttcttc atgtgttatg tggaatgaag     600

ggtaaaatta tggatgctca ccatgagtta gatgccattc ttgaaaacat catcaatgag     660

cacaagaaca atggtgaatt aggaggcgaa ggtttacttg ttgcattgct aagattaatg     720

aaggagggag gctttcaatt tccaatcacc aatgacaata tcaaagctat tatttttgac     780

atgtttgctg ggggaacaga tacttcatca acattaattg attgggccat ggtggaaatg     840

atgaggaatc caagtgtact tttgaaagct caagttgaga taagaaatgc tttcacagga     900

aaagaaactt ttaatgaaaa cgatgttgaa gaattgaaat accttaagtt ggttgttaaa     960

gaaactttca gactccatcc tccaggaccc cttttgatgg caagggaatg tagagaagaa    1020

gtagacataa atggctataa tattcctttg aaaacaaaag ttatggttaa tgcctacgca    1080

atcggaagag atcctaaata ttggattgac gcagaatgtt tcaaacctga gagatttgag    1140

cagatttcag tagattttat tggcaataac tttgaatttc ttccgtttgg tagtggaagg    1200

aggagttgtc cgggaatatc atttggtcta gctaatattt ttttcccact agctaaatta    1260

ttgtatcact ttgattggaa acttcctatt ggaatcaatc caagtgattt ggacttaact 1320

gaatctgctg gaataacttg tcctagaaag agtaacttat acttgatcgc gactcctcat 1380

gaacctagtc aagaatga 1398

<210> 2
<211> 465
<212> PRT
<213> Artificial Sequence

<220>
<223> Solhab01g008670.2.1_translation zingiberene polyoxidase lacking
      membrane anchor

<400> 2

Met Leu Pro Pro Gly Pro Trp Lys Leu Pro Leu Ile Gly Ser Met Phe
1               5                   10                  15

His Leu Leu Gly Gly Leu Pro His His Val Phe Arg Asp Leu Ala Lys
            20                  25                  30

Lys Tyr Gly Pro Ile Met His Leu Gln Leu Gly Glu Val Ser Leu Val
        35                  40                  45

Val Ile Thr Ser Pro Asp Met Ala Lys Gln Ile Leu Arg Thr His Asp
    50                  55                  60

Leu Ala Phe Ala Ser Arg Pro Lys Phe Leu Val Gly Glu Ile Leu Phe
65                  70                  75                  80

Tyr Lys Trp Thr Asp Ile Leu Phe Ser Pro Tyr Gly Asp Tyr Trp Lys
                85                  90                  95

Gln Met Arg Lys Ile Cys Leu Leu Glu Leu Leu Ser Ala Lys Asn Val
            100                 105                 110

Lys Ser Phe Asn Ser Ile Arg Gln Asp Glu Val His His Met Ile Glu
        115                 120                 125

Phe Phe Arg Ser Ser Ser Gly Glu Thr Ile Asn Val Thr Lys Arg Ile
    130                 135                 140

Tyr Gln Phe Ser Ser Ser Met Thr Cys Arg Ser Ala Phe Gly Lys Val
145                 150                 155                 160

Phe Lys Glu Gln Asn Lys Leu Ile Glu Leu Val Lys Glu Ala Ser Gly
                165                 170                 175

Phe Phe Lys Gly Phe Asp Val Ala Asp Met Phe Pro Ser Leu Lys Phe
            180                 185                 190

Leu His Val Leu Cys Gly Met Lys Gly Lys Ile Met Asp Ala His His
        195                 200                 205

Glu Leu Asp Ala Ile Leu Glu Asn Ile Ile Asn Glu His Lys Asn Asn
    210                 215                 220

Gly Glu Leu Gly Gly Glu Gly Leu Leu Val Ala Leu Leu Arg Leu Met
225                 230                 235                 240

Lys Glu Gly Gly Phe Gln Phe Pro Ile Thr Asn Asp Asn Ile Lys Ala
                245                 250                 255

Ile Ile Phe Asp Met Phe Ala Gly Gly Thr Asp Thr Ser Ser Thr Leu
            260                 265                 270

Ile Asp Trp Ala Met Val Glu Met Met Arg Asn Pro Ser Val Leu Leu
        275                 280                 285

Lys Ala Gln Val Glu Ile Arg Asn Ala Phe Thr Gly Lys Glu Thr Phe
    290                 295                 300

Asn Glu Asn Asp Val Glu Glu Leu Lys Tyr Leu Lys Leu Val Val Lys
305                 310                 315                 320

Glu Thr Phe Arg Leu His Pro Pro Gly Pro Leu Leu Met Ala Arg Glu
                325                 330                 335

Cys Arg Glu Glu Val Asp Ile Asn Gly Tyr Asn Ile Pro Leu Lys Thr
            340                 345                 350

Lys Val Met Val Asn Ala Tyr Ala Ile Gly Arg Asp Pro Lys Tyr Trp
        355                 360                 365

Ile Asp Ala Glu Cys Phe Lys Pro Glu Arg Phe Glu Gln Ile Ser Val
    370                 375                 380

Asp Phe Ile Gly Asn Asn Phe Glu Phe Leu Pro Phe Gly Ser Gly Arg
385                 390                 395                 400

Arg Ser Cys Pro Gly Ile Ser Phe Gly Leu Ala Asn Ile Phe Phe Pro
                405                 410                 415

Leu Ala Lys Leu Leu Tyr His Phe Asp Trp Lys Leu Pro Ile Gly Ile
            420                 425                 430

Asn Pro Ser Asp Leu Asp Leu Thr Glu Ser Ala Gly Ile Thr Cys Pro
        435                 440                 445

Arg Lys Ser Asn Leu Tyr Leu Ile Ala Thr Pro His Glu Pro Ser Gln
    450                 455                 460

Glu
465

<210>  3
<211>  1488
<212>  DNA
<213>  Solanum habrochaites

<400>  3
atgcagttct tcaacatctt ttcccttttc ctttttatgt ctttcctctt tttgttaatt        60

aaatggaaga attccaatag caaaaccaaa agattgcctc ctggtccatg gaaattacct       120

ttaattggaa gcatgtttca tttgttaggt ggacttccac atcatgtttt tagagattta       180

gcgaaaaaat atggaccaat tatgcacctt caactaggtg aagtttctct agttgttatt       240

acttctcctg acatggccaa acaaatacta agaactcatg atctcgcttt tgcatctagg       300

cccaagttcc tagttgggga gattctcttt tataagtgga ctgatattct tttttctccc       360

tatggtgatt actggaaaca aatgcgtaaa atatgtctat tggaattgct tagcgcaaaa       420

aatgttaagt cattcaactc tatacgacaa gatgaagttc atcatatgat tgagtttttt       480

cgatcatctt ctggtgaaac aattaatgta acaaaaagga tttatcaatt ctcgagctct       540

atgacatgta gatcagcatt tgggaaagta ttcaaagagc aaaacaaact catagaactc       600

gttaaagaag cgtcaggctt ttttaaagga tttgatgtag ctgatatgtt tccatcacta       660

aaatttcttc atgtgttatg tggaatgaag ggtaaaatta tggatgctca ccatgagtta       720

gatgccattc ttgaaaacat catcaatgag cacaagaaca atggtgaatt aggaggcgaa       780

ggtttacttg ttgcattgct aagattaatg aaggagggag gctttcaatt tccaatcacc       840

aatgacaata tcaaagctat tatttttgac atgtttgctg ggggaacaga tacttcatca       900

acattaattg attgggccat ggtggaaatg atgaggaatc caagtgtact tttgaaagct       960

caagttgaga taagaaatgc tttcacagga aaagaaactt ttaatgaaaa cgatgttgaa      1020

gaattgaaat accttaagtt ggttgttaaa gaaactttca gactccatcc tccaggaccc      1080

cttttgatgg caagggaatg tagagaagaa gtagacataa atggctataa tattcctttg      1140

aaaacaaaag ttatggttaa tgcctacgca atcggaagag atcctaaata ttggattgac      1200

gcagaatgtt tcaaacctga gagatttgag cagatttcag tagattttat tggcaataac      1260

tttgaatttc ttccgtttgg tagtggaagg aggagttgtc cgggaatatc atttggtcta      1320

gctaatattt ttttcccact agctaaatta ttgtatcact ttgattggaa acttcctatt 1380

ggaatcaatc caagtgattt ggacttaact gaatctgctg gaataacttg tcctagaaag 1440

agtaacttat acttgatcgc gactcctcat gaacctagtc aagaatga 1488

<210> 4
<211> 495
<212> PRT
<213> Solanum habrochaites

<400> 4

Met Gln Phe Phe Asn Ile Phe Ser Leu Phe Leu Phe Met Ser Phe Leu
1               5                   10                  15

Phe Leu Leu Ile Lys Trp Lys Asn Ser Asn Ser Lys Thr Lys Arg Leu
            20                  25                  30

Pro Pro Gly Pro Trp Lys Leu Pro Leu Ile Gly Ser Met Phe His Leu
        35                  40                  45

Leu Gly Gly Leu Pro His His Val Phe Arg Asp Leu Ala Lys Lys Tyr
    50                  55                  60

Gly Pro Ile Met His Leu Gln Leu Gly Glu Val Ser Leu Val Val Ile
65                  70                  75                  80

Thr Ser Pro Asp Met Ala Lys Gln Ile Leu Arg Thr His Asp Leu Ala
                85                  90                  95

Phe Ala Ser Arg Pro Lys Phe Leu Val Gly Glu Ile Leu Phe Tyr Lys
            100                 105                 110

Trp Thr Asp Ile Leu Phe Ser Pro Tyr Gly Asp Tyr Trp Lys Gln Met
        115                 120                 125

Arg Lys Ile Cys Leu Leu Glu Leu Leu Ser Ala Lys Asn Val Lys Ser
    130                 135                 140

Phe Asn Ser Ile Arg Gln Asp Glu Val His His Met Ile Glu Phe Phe
145                 150                 155                 160

Arg Ser Ser Ser Gly Glu Thr Ile Asn Val Thr Lys Arg Ile Tyr Gln
                165                 170                 175

Phe Ser Ser Ser Met Thr Cys Arg Ser Ala Phe Gly Lys Val Phe Lys
            180                 185                 190

Glu Gln Asn Lys Leu Ile Glu Leu Val Lys Glu Ala Ser Gly Phe Phe

195                 200                 205

Lys Gly Phe Asp Val Ala Asp Met Phe Pro Ser Leu Lys Phe Leu His
    210                 215                 220

Val Leu Cys Gly Met Lys Gly Lys Ile Met Asp Ala His His Glu Leu
225                 230                 235                 240

Asp Ala Ile Leu Glu Asn Ile Ile Asn Glu His Lys Asn Asn Gly Glu
                245                 250                 255

Leu Gly Gly Glu Gly Leu Leu Val Ala Leu Leu Arg Leu Met Lys Glu
            260                 265                 270

Gly Gly Phe Gln Phe Pro Ile Thr Asn Asp Asn Ile Lys Ala Ile Ile
        275                 280                 285

Phe Asp Met Phe Ala Gly Gly Thr Asp Thr Ser Ser Thr Leu Ile Asp
    290                 295                 300

Trp Ala Met Val Glu Met Met Arg Asn Pro Ser Val Leu Leu Lys Ala
305                 310                 315                 320

Gln Val Glu Ile Arg Asn Ala Phe Thr Gly Lys Glu Thr Phe Asn Glu
                325                 330                 335

Asn Asp Val Glu Glu Leu Lys Tyr Leu Lys Leu Val Val Lys Glu Thr
            340                 345                 350

Phe Arg Leu His Pro Pro Gly Pro Leu Leu Met Ala Arg Glu Cys Arg
        355                 360                 365

Glu Glu Val Asp Ile Asn Gly Tyr Asn Ile Pro Leu Lys Thr Lys Val
    370                 375                 380

Met Val Asn Ala Tyr Ala Ile Gly Arg Asp Pro Lys Tyr Trp Ile Asp
385                 390                 395                 400

Ala Glu Cys Phe Lys Pro Glu Arg Phe Glu Gln Ile Ser Val Asp Phe
                405                 410                 415

Ile Gly Asn Asn Phe Glu Phe Leu Pro Phe Gly Ser Gly Arg Arg Ser
            420                 425                 430

Cys Pro Gly Ile Ser Phe Gly Leu Ala Asn Ile Phe Phe Pro Leu Ala
        435                 440                 445

Lys Leu Leu Tyr His Phe Asp Trp Lys Leu Pro Ile Gly Ile Asn Pro
    450                 455                 460

Ser Asp Leu Asp Leu Thr Glu Ser Ala Gly Ile Thr Cys Pro Arg Lys
465                 470                 475                 480

Ser Asn Leu Tyr Leu Ile Ala Thr Pro His Glu Pro Ser Gln Glu
                485                 490                 495

<210>  5
<211>  1488
<212>  DNA
<213>  Artificial Sequence

<220>
<223>  coding sequence of Solyc01g008670

<400>  5
atgcagttct tcaacatctt ttcccttttc ctttttatgt ctttcctctt tttgttaatt      60

aaatggaaga attccaatag caaaaccaaa agattgcctc ctggtccatg gaaattacct     120

ttaattggaa gcatgtttca tttgttaggt ggacttccac atcatgtttt tagagattta     180

gcgaaaaaat atggaccaat tatgcacctt caactaggtg aagtttctct agttgttatt     240

acttctcctg acatggccaa acaaatacta agaactcatg atctcgcttt tgcatctagg     300

cccaagttcc tagttgggga gattctcttt tataagtgga ctgatattct tttttctccc     360

tatggtgatt actggaaaca aatgcgtaaa atatgtctat tggaattgct tagcgcaaaa     420

aatgttaagt cattcaactc tatacgacaa gatgaagttc atcatatgat tgagtttttt     480

cgatcatctt ctggtgaaac aattaatgta acaaaaagga tttatcaatt ctcgagctct     540

atgacatgta gatcagcatt tgggaaagta ttcaaagagc aaaacaaact catagaactc     600

gttaaagaag cgtcaggctt ttttaaagga tttgatgtag ctgatatgtt tccatcacta     660

aaatttcttc atgtgttatg tggaatgaag ggtaaaatta tggatgctca ccatgagtta     720

gatgccattc ttgaaaacat catcaatgag cacaagaaca atggtgaatt aggaggcgaa     780

ggtttacttg ttgcattgct aagattaatg aaggagggag gctttcaatt tccaatcacc     840

aatgacaata tcaaagctat tatttttgac atgtttgctg ggggaacaga tacttcatca     900

acattaattg attgggccat ggtggaaatg atgaggaatc caagtgtact tttgaaagct     960

caagttgaga taagaaatgc tttcacagga aaagaaactt ttaatgaaaa cgatgttgaa    1020

gaattgaaat accttaagtt ggttgttaaa gaaactttca gactccatcc tccaggaccc    1080

cttttgatgg caagggaatg tagagaagaa gtagacataa atggctataa tattcctttg    1140

aaaacaaaag ttatggttaa tgcctacgca atcggaagag atcctaaata ttggattgac    1200

gcagaatgtt tcaaacctga gagatttgag cagatttcag tagattttat tggcaataac    1260

tttgaatttc ttccgtttgg tagtggaagg aggagttgtc cgggaatatc atttggtcta 1320

gctaatattt ttttcccact agctaaatta ttgtatcact ttgattggaa acttcctatt 1380

ggaatcaatc caagtgattt ggacttaact gaatctgctg gaataacttg tcctagaaag 1440

agtaacttat acttgatcgc gactcctcat gaacctagtc aagaatga 1488

<210> 6
<211> 36
<212> DNA
<213> Artificial Sequence

<220>
<223> P450iso008670-F

<400> 6
tttgaagaca aaatgcagtt cttcaacatc ttttcc 36

<210> 7
<211> 39
<212> DNA
<213> Artificial Sequence

<220>
<223> P450iso008670-R

<400> 7
tttgaagaca aaagctcatt cttgactagg ttcatgagg 39

<210> 8
<211> 23
<212> DNA
<213> Artificial Sequence

<220>
<223> P450-Fq Forward

<400> 8
catgtgttat gtggaatgaa ggg 23

<210> 9
<211> 24
<212> DNA
<213> Artificial Sequence

<220>
<223> P450-Rq Reverse

<400> 9
tcctaattca ccattgttct tgtg 24

<210> 10
<211> 30
<212> DNA
<213> Artificial Sequence

<220>

<223>  P450HRMC_For

<400>  10
atggctataa tattcctttg aaaacaaaag                                        30


<210>  11
<211>  24
<212>  DNA
<213>  Artificial Sequence

<220>
<223>  P450HRMC_Rev

<400>  11
gtttgaaaca ttctgcgtca atcc                                              24


<210>  12
<211>  45
<212>  DNA
<213>  Artificial Sequence

<220>
<223>  pGal_syn_for1


<220>
<221>  misc_feature
<222>  (19)..(28)
<223>  n is a, c, g, or t

<400>  12
tttggtctca acatggagnn nnnnnnnncg gattagaagc cgccg                        45


<210>  13
<211>  76
<212>  DNA
<213>  Artificial Sequence

<220>
<223>  pGal_syn_rev1


<220>
<221>  misc_feature
<222>  (38)..(39)
<223>  n is a, c, g, or t

<220>
<221>  misc_feature
<222>  (58)..(59)
<223>  n is a, c, g, or t

<400>  13
tttggtctca acaaattgtt cggagcagtg cggcgcgnnt cggagggctg tcgcccgnnc       60

ggcggcttct aatccg                                                       76


<210>  14
<211>  61

<212>  DNA
<213>  Artificial Sequence

<220>
<223>  pGal_syn_for2


<220>
<221>  misc_feature
<222>  (19)..(38)
<223>  n is a, c, g, or t

<400>  14
tttggtctca acatcaatnn nnnnnnnnnn nnnnnnnnca ccggcggtct ttcgtccgtg      60

c                                                                      61


<210>  15
<211>  60
<212>  DNA
<213>  Artificial Sequence

<220>
<223>  pGal_syn_rev2


<220>
<221>  misc_feature
<222>  (19)..(37)
<223>  n is a, c, g, or t

<400>  15
tttggtctca acaaatagnn nnnnnnnnnn nnnnnnngca cggacgaaag accgccggtg      60


<210>  16
<211>  61
<212>  DNA
<213>  Artificial Sequence

<220>
<223>  pGal_syn_for3

<400>  16
ttggtctcaa catctatata aatgcaaaaa ctgyatawmm acwwtaacta atactttcaa      60

c                                                                      61


<210>  17
<211>  58
<212>  DNA
<213>  Artificial Sequence

<220>
<223>  pGal_syn_rev3

<400>  17
tttggtctca acaagaatra rragyaatac aammcsaaaw tgttgaaagt attagtta        58


<210>  18

<211>  64
<212>  DNA
<213>  Artificial Sequence

<220>
<223>  pGal_syn_for4

<400>  18
tttggtctca acatattcwm aystwatwma aktatcawca araaatwgtt aatatacctc      60

tata                                                                  64


<210>  19
<211>  58
<212>  DNA
<213>  Artificial Sequence

<220>
<223>  pGal_syn_rev4


<220>
<221>  misc_feature
<222>  (40)..(41)
<223>  n is a, c, g, or t

<400>  19
tttggtctca acaacattkt wttttytyyw tkaygttaan ntatagaggt atattaac        58


<210>  20
<211>  2974
<212>  DNA
<213>  Artificial Sequence

<220>
<223>  pAGM2925

<400>  20
tcggtgagac catttccccg aaaagtgcca cctgacgtct aagaaaccat tattatcatg      60

acattaacct ataaaaatag gcgtatcacg aggccctttc gactcgcgcg tttcggtgat     120

gacggtgaaa acctctgaca catgcagctc ccggagatgg tcacagcttg tctgtaagcg     180

gatgccggga gcagacaagc ccgtcagggc gcgtcagcgg gtgttggcgg gtgtcggggc     240

tggcttaact atgcggcatc agagcagatt gtactgagag tgcaccatat gcggtgtgaa     300

ataccgcaca gatgcgtaag gagaaaatac cgcatcaggc gccattcgcc attcaggctg     360

cgcaactgtt gggaagggcg atcggtgcgg gcctcttcgc tattacgcca gctggcgaaa     420

gggggatgtg ctgcaaggcg attaagttgg gtaacgccag ggttttccca gtcacgacgt     480

tgtaaaacga cggccagtga attcgagctc ggtacccggg gatcctctag agtcgacctg     540

caggcatgca agcttggcgt aatcatggtc atagctgttt cctgtgtgaa attgttatcc     600

gctcacaatt ccacacaaca tacgagccgg aagcataaag tgtaaagcct ggggtgccta     660

atgagtgagc taactcacat taattgcgtt gcgctcactg cccgctttcc agtcgggaaa     720

cctgtcgtgc cagctgcatt aatgaatcgg ccaacgcgcg gggagaggcg gtttgcgtat 780

tgggcgctct tccgcttcct cgctcactga ctcgctgcgc tcggtcgttc ggctgcggcg 840

agcggtatca gctcactcaa aggcggtaat acggttatcc acagaatcag gggataacgc 900

aggaaagaac atgtgagcaa aaggccagca aaaggccagg aaccgtaaaa aggccgcgtt 960

gctggcgttt ttccataggc tccgcccccc tgacgagcat cacaaaaatc gacgctcaag 1020

tcagaggtgg cgaaacccga caggactata aagataccag gcgtttcccc ctggaagctc 1080

cctcgtgcgc tctcctgttc cgaccctgcc gcttaccgga tacctgtccg cctttctccc 1140

ttcgggaagc gtggcgcttt ctcatagctc acgctgtagg tatctcagtt cggtgtaggt 1200

cgttcgctcc aagctgggct gtgtgcacga accccccgtt cagcccgacc gctgcgcctt 1260

atccggtaac tatcgtcttg agtccaaccc ggtaagacac gacttatcgc cactggcagc 1320

agccactggt aacaggatta gcagagcgag gtatgtaggc ggtgctacag agttcttgaa 1380

gtggtggcct aactacggct acactagaag gacagtattt ggtatctgcg ctctgctgaa 1440

gccagttacc ttcggaaaaa gagttggtag ctcttgatcc ggcaaacaaa ccaccgctgg 1500

tagcggtggt ttttttgttt gcaagcagca gattacgcgc agaaaaaaag gatctcaaga 1560

agatcctttg atcttttcta cggggtctga cgctcagtgg aacgaaaact cacgttaagg 1620

gattttggtc atgagattat caaaaaggat cttcacctag atccttttaa attaaaaatg 1680

aagttttaaa tcaatctaaa gtatatatga gtaaacttgg tctgggtctc aacagcatga 1740

tatatctccc aatttgtgta gggcttatta tgcacgctta aaaataataa aagcagactt 1800

gacctgatag tttggctgtg agcaattatg tgcttagtgc atctaacgct tgagttaagc 1860

cgcgccgcga agcggcgtcg gcttgaacga atttctagct agacattatt tgccgactac 1920

cttggtgatc tcgcctttca cgtaatggac aaattcttcc aactgatctg cgcgcgaggc 1980

caagcgatct tcttcttgtc caagataagc ctgtctagct tcaagtatga cgggctgata 2040

ctgggccggc aggcgctcca ttgcccagtc ggcagcgaca tccttcggcg cgattttgcc 2100

ggttactgcg ctgtaccaaa tgcgggacaa cgtaagcact acatttcgct catcgccagc 2160

ccagtcgggc ggcgagttcc atagcgttaa ggtttcattt agcgcctcaa atagatcctg 2220

ttcaggaacc ggatcaaaga gttcctccgc cgctggacct accaaggcaa cgctatgttc 2280

tcttgctttt gtcagcaaga tagccagatc aatgtcgatc gtggctggct cgaagatacc 2340

tgcaagaatg tcattgcgct gccattctcc aaattgcagt tcgcgcttag ctggataacg 2400

ccacggaatg atgtcgtcgt gcacaacaat ggtgacttct acagcgcgga gaatctcgct 2460

ctctccaggg gaagccgaag tttccaaaag gtcgttgatc aaagctcgcc gcgttgtttc 2520

atcaagcctt acggtcaccg taaccagcaa atcaatatcg ctgtgtggct tcaggccgcc 2580

atccactgcg gagccgtaca aatgtacggc cagcaacgtc ggttcgagat ggcgctcgat 2640

gacgccaact acctctgata gttgagtcga tacttcggcg atcaccgctt cccccatgat 2700

gtttaacttt gttttagggc gactgccctg ctgcgtaaca tcgttgctgc tccataacat 2760

caaacatcga cccacggcgt aacgcgcttg ctgcttggat gcccgaggca tagactgtac 2820

cccaaaaaaa cagtcataac aagccatgaa aaccgccact gcgccgttac caccgctgcg 2880

ttcggtcaag gttctggacc agttgcgtga gcgcatacgc tacttgcatt acagcttacg 2940

aaccgaacag gcttatgtcc actgggttcg tgcc 2974

<210> 21
<211> 3196
<212> DNA
<213> Artificial Sequence

<220>
<223> pICH75721

<400> 21
tttggtctca tggtcactct gtggattttt ctttcaaagt ggaaacttgg gacttcagat 60

tccacccctt gcaagcgtgg aaagccttcc gaccaaggga agtgtcggat gtagaggaaa 120

tggaaagttt gttctcagat ggggacctgc ttgattgctt cacaagaatg ccagcttatg 180

cggtaaacgc agaggaagat ttagctgcaa tcaggaaaac gcccgagatg gatgtcggtc 240

aagaagttaa agagcctgca ggagacagaa atcaatactc aaaccctgca gaaactttcc 300

tcaacaagct ccacaggaaa cacagtaggg aggtgaccac gaagtggccg tgagaccaaa 360

gggtaccgag ctcgaattca ctggccgtcg ttttacaacg tcgtgactgg gaaaaccctg 420

gcgttaccca acttaatcgc cttgcagcac atcccccttt cgccagctgg cgtaatagcg 480

aagaggcccg caccgatcgc ccttcccaac agttgcgcag cctgaatggc gaatggcgcc 540

tgatgcggta ttttctcctt acgcatctgt gcggtatttc acaccgcata tggtgcactc 600

tcagtacaat ctgctctgat gccgcatagt taagccagcc ccgacacccg ccaacacccg 660

ctgacgcgcc ctgacgggct tgtctgctcc cggcatccgc ttacagacaa gctgtgacca 720

tctccgggag ctgcatgtgt cagaggtttt caccgtcatc accgaaacgc gcgagtcgaa 780

agggcctcgt gatacgccta tttttatagg ttaatgtcat gataataatg gtttcttaga 840

cgtcaggtgg cacttttcgg ggaaatgtgc gcggaacccc tatttgttta tttttctaaa 900

tacattcaaa tatgtatccg ctcatgagac aataaccctg ataaatgctt caataatatt 960

gaaaaaggaa gagtatgcgc tcacgcaact ggtccagaac cttgaccgaa cgcagcggtg 1020

gtaacggcgc agtggcggtt ttcatggctt gttatgactg tttttttggg gtacagtcta 1080

tgcctcgggc atccaagcag caagcgcgtt acgccgtggg tcgatgtttg atgttatgga 1140

gcagcaacga tgttacgcag cagggcagtc gccctaaaac aaagttaaac atcatgaggg 1200

aagcggtgat cgccgaagta tcgactcaac tatcagaggt agttggcgtc atcgagcgcc 1260

atctcgaacc gacgttgctg gccgtacatt tgtacggctc cgcagtggat ggcggcctga 1320

agccacacag cgatattgat ttgctggtta cggtgaccgt aaggcttgat gaaacaacgc 1380

ggcgagcttt gatcaacgac cttttggaaa cttcggcttc ccctggagag agcgagattc 1440

tccgcgctgt agaagtcacc attgttgtgc acgacgacat cattccgtgg cgttatccag 1500

ctaagcgcga actgcaattt ggagaatggc agcgcaatga cattcttgca ggtatcttcg 1560

agccagccac gatcgacatt gatctggcta tcttgctgac aaaagcaaga gaacatagcg 1620

ttgccttggt aggtccagcg gcggaggaac tctttgatcc ggttcctgaa caggatctat 1680

ttgaggcgct aaatgaaacc ttaacgctat ggaactcgcc gcccgactgg gctggcgatg 1740

agcgaaatgt agtgcttacg ttgtcccgca tttggtacag cgcagtaacc ggcaaaatcg 1800

cgccgaagga tgtcgctgcc gactgggcaa tggagcgcct gccggcccag tatcagcccg 1860

tcatacttga agctagacag gcttatcttg gacaagaaga agatcgcttg gcctcgcgcg 1920

cagatcagtt ggaagaattt gtccattacg taaaaggcga gatcaccaag gtagtcggca 1980

aataactgtc agaccaagtt tactcatata tactttagat tgatttaaaa cttcattttt 2040

aatttaaaag gatctaggtg aagatccttt ttgataatct catgaccaaa atcccttaac 2100

gtgagttttc gttccactga gcgtcagacc ccgtagaaaa gatcaaagga tcttcttgag 2160

atcctttttt tctgcgcgta atctgctgct tgcaaacaaa aaaaccaccg ctaccagcgg 2220

tggtttgttt gccggatcaa gagctaccaa ctctttttcc gaaggtaact ggcttcagca 2280

gagcgcagat accaaatact gtccttctag tgtagccgta gttaggccac cacttcaaga 2340

actctgtagc accgcctaca tacctcgctc tgctaatcct gttaccagtg gctgctgcca 2400

gtggcgataa gtcgtgtctt accgggttgg actcaagacg atagttaccg gataaggcgc 2460

agcggtcggg ctgaacgggg ggttcgtgca cacagcccag cttggagcga acgacctaca 2520

ccgaactgag atacctacag cgtgagctat gagaaagcgc cacgcttccc gaagggagaa 2580

aggcggacag gtatccggta agcggcaggg tcggaacagg agagcgcacg agggagcttc 2640

cagggggaaa cgcctggtat ctttatagtc ctgtcgggtt tcgccacctc tgacttgagc 2700

gtcgattttt gtgatgctcg tcaggggggc ggagcctatg gaaaaacgcc agcaacgcgg 2760

cctttttacg gttcctggcc ttttgctggc cttttgctca catgttcttt cctgcgttat 2820

cccctgattc tgtggataac cgtattaccg cctttgagtg agctgatacc gctcgccgca 2880

gccgaacgac cgagcgcagc gagtcagtga gcgaggaagc ggatgagcgc ccaatacgca 2940

aaccgcctct ccccgcgcgt tggccgattc attaatgcag ctggcacgac aggtttcccg 3000

actggaaagc gggcagtgag cgcaacgcaa ttaatgtgag ttagctcact cattaggcac 3060

cccaggcttt acactttatg cttccggctc gtatgttgtg tggaattgtg agcggataac 3120

aatttcacac aggaaacagc tatgaccatg attacgccaa gcttgcatgc ctgcaggtcg 3180

actctagagg atcccc 3196

<210> 22
<211> 3370
<212> DNA
<213> Artificial Sequence

<220>
<223> pICH75716

<400> 22
tggttgagac cacgaagtgg ctcttcagtg gacgaaaggg cctcgtgata cgcctatttt 60

tataggttaa tgtcatgata ataatggttt cttagacgtc aggtggcact tttcggggaa 120

atgtgcgcgg aacccctatt tgtttatttt tctaaataca ttcaaatatg tatccgctca 180

tgagacaata accctgataa atgcttcaat aatattgaaa aaggaagagt atgcgctcac 240

gcaactggtc cagaaccttg accgaacgca gcggtggtaa cggcgcagtg gcggttttca 300

tggcttgtta tgactgtttt tttggggtac agtctatgcc tcgggcatcc aagcagcaag 360

cgcgttacgc cgtgggtcga tgtttgatgt tatggagcag caacgatgtt acgcagcagg 420

gcagtcgccc taaaacaaag ttaaacatca tgagggaagc ggtgatcgcc gaagtatcga 480

ctcaactatc agaggtagtt ggcgtcatcg agcgccatct cgaaccgacg ttgctggccg 540

tacatttgta cggctccgca gtggatggcg gcctgaagcc acacagcgat attgatttgc 600

tggttacggt gaccgtaagg cttgatgaaa caacgcggcg agctttgatc aacgaccttt 660

tggaaacttc ggcttcccct ggagagagcg agattctccg cgctgtagaa gtcaccattg 720

ttgtgcacga cgacatcatt ccgtggcgtt atccagctaa gcgcgaactg caatttggag 780

aatggcagcg caatgacatt cttgcaggta tcttcgagcc agccacgatc gacattgatc 840

tggctatctt gctgacaaaa gcaagagaac atagcgttgc cttggtaggt ccagcggcgg 900

aggaactctt tgatccggtt cctgaacagg atctatttga ggcgctaaat gaaaccttaa 960

cgctatggaa ctcgccgccc gactgggctg gcgatgagcg aaatgtagtg cttacgttgt 1020

cccgcatttg gtacagcgca gtaaccggca aaatcgcgcc gaaggatgtc gctgccgact 1080

gggcaatgga gcgcctgccg gcccagtatc agcccgtcat acttgaagct agacaggctt 1140

atcttggaca agaagaagat cgcttggcct cgcgcgcaga tcagttggaa gaatttgtcc 1200

attacgtaaa aggcgagatc accaaggtag tcggcaaata actgtcagac caagtttact 1260

catatatact ttagattgat ttaaaacttc atttttaatt taaaaggatc taggtgaaga 1320

tcctttttga taatctcatg accaaaatcc cttaacgtga gttttcgttc cactgagcgt 1380

cagaccccgt agaaaagatc aaaggatctt cttgagatcc tttttttctg cgcgtaatct 1440

gctgcttgca aacaaaaaaa ccaccgctac cagcggtggt ttgtttgccg gatcaagagc 1500

taccaactct ttttccgaag gtaactggct tcagcagagc gcagatacca aatactgtcc 1560

ttctagtgta gccgtagtta ggccaccact tcaagaactc tgtagcaccg cctacatacc 1620

tcgctctgct aatcctgtta ccagtggctg ctgccagtgg cgataagtcg tgtcttaccg 1680

ggttggactc aagacgatag ttaccggata aggcgcagcg gtcgggctga acggggggtt 1740

cgtgcacaca gcccagcttg gagcgaacga cctacaccga actgagatac ctacagcgtg 1800

agctatgaga aagcgccacg cttcccgaag ggagaaaggc ggacaggtat ccggtaagcg 1860

gcagggtcgg aacaggagag cgcacgaggg agcttccagg gggaaacgcc tggtatcttt 1920

atagtcctgt cgggtttcgc cacctctgac ttgagcgtcg atttttgtga tgctcgtcag 1980

gggggcggag cctatggaaa aacgccagca acgcggcctt tttacggttc ctggcctttt 2040

gctggccttt tgctcacatg ttctttcctg cgttatcccc tgattctgtg gataaccgta 2100

ttaccgcctt tgagtgagct gataccgctc gccgcagccg aacgaccgag cgcagcgagt 2160

cagtgagcga ggaagcggaa gagcgcccaa tacgcaaacc gcctctcccc gcgcgttggc 2220

cgattcatta atcactctgt ggtctcacct gcctttttca atgggtaata actgatataa 2280

ttaaattgaa gctctaattt gtgagtttag tatacatgca tttacttata atacagtttt 2340

ttagttttgc tggccgcatc ttctcaaata tgcttcccag cctgcttttc tgtaacgttc 2400

accctctacc ttagcatccc ttccctttgc aaatagtcct cttccaacaa taataatgtc 2460

agatcctgta gataccacat catccacggt tctatactgt tgacccaatg cgtcaccctt 2520

gtcatctaaa cccactccgg gtgtcataat caaccaatcg taaccttcat ctcttccacc 2580

catgtctctt tgagcaataa agccgataac aaaatctttg tcgctcttgg caatgtcaac 2640

agtaccctta gtatattctc cagtagatag ggagcccttg catgacaatt ctgctaacat 2700

caaaaggcct ctaggttcct ttgttacttc ttctgccgcc tgcttcaaac cgctaacaat 2760

acctgggccc acgacaccgt gagcattcgt aatgtctgcc cattctgcta ttctgtatac 2820

acccgcagag tactgcaatt tgactgtatt accaatgtca gcaaattttc tgtcctcgaa 2880

gagtaaaaaa ttgtacttgg cggataatgc ctttagcggc ttaactgtgc cctccatgga 2940

aaaatcagtc aagatatcca catgtgtttt tagtaaacaa atttgggacc taatgcttc 3000

aactaactcc agtaattcct tggtggtacg aacatccaat gaagcacaca agtttgtttg 3060

cttttcgtgc atgatattaa atagcttggc agcaacagga ctaggatgag tagcagcacg 3120

ttccttatat gtagctttcg acatgattta tcttcgtttc ctgcaggttt ttgttctgtg 3180

cagttgggtt aagaatactg ggcaatttca tgtttcttca acactacata tgcgtatata 3240

taccaatcta agtctgtgct ccttccttcg ttcttccttc tgttcggaga ttaccgaatc 3300

aaaaaaattt caaagaaacc gaaatcaaaa aaaagaataa aaaaaaaatg atgaattgaa 3360

ttgaaaagct 3370

<210> 23
<211> 3723
<212> DNA
<213> Artificial Sequence

<220>
<223> pICH75603

<400> 23
ccgatgagac cacgaagtgg ctcttcagtg gacgaaaggg cctcgtgata cgcctatttt 60

tataggttaa tgtcatgata ataatggttt cttagacgtc aggtggcact tttcggggaa 120

atgtgcgcgg aacccctatt tgtttatttt tctaaataca ttcaaatatg tatccgctca 180

tgagacaata accctgataa atgcttcaat aatattgaaa aaggaagagt atgcgctcac 240

gcaactggtc cagaaccttg accgaacgca gcggtggtaa cggcgcagtg gcggttttca 300

tggcttgtta tgactgtttt tttggggtac agtctatgcc tcgggcatcc aagcagcaag 360

cgcgttacgc cgtgggtcga tgtttgatgt tatggagcag caacgatgtt acgcagcagg 420

gcagtcgccc taaaacaaag ttaaacatca tgagggaagc ggtgatcgcc gaagtatcga 480

ctcaactatc agaggtagtt ggcgtcatcg agcgccatct cgaaccgacg ttgctggccg 540

tacatttgta cggctccgca gtggatggcg gcctgaagcc acacagcgat attgatttgc 600

tggttacggt gaccgtaagg cttgatgaaa caacgcggcg agctttgatc aacgaccttt 660

tggaaacttc ggcttcccct ggagagagcg agattctccg cgctgtagaa gtcaccattg 720

ttgtgcacga cgacatcatt ccgtggcgtt atccagctaa gcgcgaactg caatttggag 780

aatggcagcg caatgacatt cttgcaggta tcttcgagcc agccacgatc gacattgatc 840

tggctatctt gctgacaaaa gcaagagaac atagcgttgc cttggtaggt ccagcggcgg 900

aggaactctt tgatccggtt cctgaacagg atctatttga ggcgctaaat gaaaccttaa 960

cgctatggaa ctcgccgccc gactgggctg gcgatgagcg aaatgtagtg cttacgttgt 1020

cccgcatttg gtacagcgca gtaaccggca aaatcgcgcc gaaggatgtc gctgccgact 1080

gggcaatgga gcgcctgccg gcccagtatc agcccgtcat acttgaagct agacaggctt 1140

atcttggaca agaagaagat cgcttggcct cgcgcgcaga tcagttggaa gaatttgtcc 1200

attacgtaaa aggcgagatc accaaggtag tcggcaaata actgtcagac caagtttact 1260

catatatact ttagattgat ttaaaacttc atttttaatt taaaaggatc taggtgaaga 1320

tcctttttga taatctcatg accaaaatcc cttaacgtga gttttcgttc cactgagcgt 1380

cagaccccgt agaaaagatc aaaggatctt cttgagatcc tttttttctg cgcgtaatct 1440

gctgcttgca aacaaaaaaa ccaccgctac cagcggtggt ttgtttgccg gatcaagagc 1500

taccaactct ttttccgaag gtaactggct tcagcagagc gcagatacca aatactgtcc 1560

ttctagtgta gccgtagtta ggccaccact tcaagaactc tgtagcaccg cctacatacc 1620

tcgctctgct aatcctgtta ccagtggctg ctgccagtgg cgataagtcg tgtcttaccg 1680

ggttggactc aagacgatag ttaccggata aggcgcagcg gtcgggctga acggggggtt 1740

cgtgcacaca gcccagcttg gagcgaacga cctacaccga actgagatac ctacagcgtg 1800

agctatgaga aagcgccacg cttcccgaag ggagaaaggc ggacaggtat ccggtaagcg 1860

gcagggtcgg aacaggagag cgcacgaggg agcttccagg gggaaacgcc tggtatcttt 1920

atagtcctgt cgggtttcgc cacctctgac ttgagcgtcg atttttgtga tgctcgtcag 1980

gggggcggag cctatggaaa aacgccagca acgcggcctt tttacggttc ctggcctttt 2040

gctggccttt tgctcacatg ttctttcctg cgttatcccc tgattctgtg gataaccgta 2100

ttaccgcctt tgagtgagct gataccgctc gccgcagccg aacgaccgag cgcagcgagt 2160

cagtgagcga ggaagcggaa gagcgcccaa tacgcaaacc gcctctcccc gcgcgttggc 2220

cgattcatta atcactctgt ggtctcagcc gttatcgatg ataagctgtc aaagatgaga 2280

attaattcca cggactatag actatactag atactccgtc tactgtacga tacacttccg 2340

ctcaggtcct tgtcctttaa cgaggcctta ccactctttt gttactctat tgatccagct 2400

cagcaaaggc agtgtgatct aagattctat cttcgcgatg tagtaaaact agctagaccg 2460

agaaagagac tagaaatgca aaggcacttc ctacaatggc tgccatcatt attatccgat 2520

gtgacgctgc agcttctcaa tgatattcga atacgctttg aggagataca gcctaatatc 2580

cgacaaactg ttttacagat ttacgatcgt acttgttacc catcattgaa ttttgaacat 2640

ccgaacctgg gagttttccc tgaaacagat agtatatttg aacctgtata ataatatata 2700

gtctagcgct ttacggaaga gaatgtatgt atttcggttc ctggagaaac tattgcatct 2760

attgcatagg taatcttgca cgtcgcatcc ccggttcatt ttctgcgttt ccatcttgca 2820

cttcaatagc atatctttgt taacgaagca tctgtgcttc attttgtaga acaaaaatgc 2880

aacgcgagag cgctaatttt tcaaacaaag aatctgagct gcatttttac agaacagaaa 2940

tgcaacgcga aagcgctatt ttaccaacga agaatctgtg cttcattttt gtaaaacaaa 3000

aatgcaacgc gacgagagcg ctaatttttc aaacaaagaa tctgagctgc atttttacag 3060

aacagaaatg caacgcgaga gcgctatttt accaacaaag aatctatact tcttttttgt 3120

tctacaaaaa tgcatcccga gagcgctatt tttctaacaa agcatcttag attacttttt 3180

ttctcctttg tgcgctctat aatgcagtct cttgataact ttttgcactg taggtccgtt 3240

aaggttagaa gaaggctact ttggtgtcta ttttctcttc cataaaaaaa gcctgactcc 3300

acttcccgcg tttactgatt actagcgaag ctgcgggtgc attttttcaa gataaaggca 3360

tccccgatta tattctatac cgatgtggat tgcgcatact ttgtgaacag aaagtgatag 3420

cgttgatgat tcttcattgg tcagaaaatt atgaacggtt tcttctattt tgtctctata 3480

tactacgtat aggaaatgtt tacattttcg tattgttttc gattcactct atgaatagtt 3540

cttactacaa tttttttgtc taaagagtaa tactagagat aaacataaaa aatgtagagg 3600

tcgagtttag atgcaagttc aaggagcgaa aggtggatgg gtaggttata tagggatata 3660

gcacagagat atatagcaaa gagatacttt tgagcaatgt ttgtggaagc ggtattcgca 3720

atg 3723

<210> 24
<211> 2344
<212> DNA
<213> Artificial Sequence

<220>
<223> pICH42100

<400> 24
cctgtgagac cgccttttgc tggccttttg ctcacatgtt ctttcctgcg ttatcccctg 60

attctgtgga taaccgtatt accgcctttg agtgagctga taccgctcgc cgcagccgaa 120

cgaccgagcg cagcgagtca gtgagcgagg aagcggaaga gcgcccaata cgcaaaccgc 180

ctctccccgc gcgttggccg attcattaat cactctgtgg atttttcttt caaagtggaa 240

acttgggact tcagattcca ccccttgcaa gcgtggaaag ccttccgacc aagggaagtg 300

tcggatgtag aggaaatgga aagtttgttc tcagatgggg acttgcttga ttgcttcaca 360

agaatgccag cttatgcggt aaacgcagag gaagatttag ctgcaatcag gaaaacgccc 420

gagatggatg tcggtcaaga agttaaagaa cctgcaggag acagaaatca atactcaaac 480

cctgcagaaa ctttcctcaa caagctccac aggaaacaca gtagggaggt gaccacgaag 540

tggctcttca gtggacgaaa gggcctcgtg atacgcctat ttttataggt taatgtcatg 600

ataataatgg tttcttagac gtcaggtggc acttttcggg gaaatgtgcg cggaacccct 660

atttgtttat ttttctaaat acattcaaat atgtatccgc tcatgagaca ataaccctga 720

taaatgcttc aataatattg aaaaaggaag agtatggcta aaatgagaat atcaccggaa 780

ttgaaaaaac tgatcgaaaa ataccgctgc gtaaaagata cggaaggaat gtctcctgct 840

aaggtatata agctggtggg agaaaatgaa aacctatatt taaaaatgac ggacagccgg 900

tataaaggga ccacctatga tgtggaacgg gaaaaggaca tgatgctatg gctggaagga 960

aagctgcctg ttccaaaggt cctgcacttt gaacggcatg atggctggag caatctgctc 1020

atgagtgagg ccgatggcgt cctttgctcg gaagagtatg aagatgaaca aagccctgaa 1080

aagattatcg agctgtatgc ggagtgcatc aggctctttc actccatcga catatcggat 1140

tgtccctata cgaatagctt agacagccgc ttagccgaat tggattactt actgaataac 1200

gatctggccg atgtggattg cgaaaactgg gaagaggaca ctccatttaa agatccgcgc 1260

gagctgtatg attttttaaa gacggaaaag cccgaagagg aacttgtctt ttcccacggc 1320

gacctgggag acagcaacat ctttgtgaaa gatggcaaag taagtggctt tattgatctt 1380

gggagaagcg gcagggcgga caagtggtat gacattgcct tctgcgtccg gtcgatcagg 1440

gaggatatcg gggaagaaca gtatgtcgag ctattttttg acttactggg gatcaagcct 1500

gattgggaga aaataaaata ttatatttta ctggatgaat tgttttagcg gtctcactgt 1560

cagaccaagt ttactcatat atactttaga ttgatttaaa acttcatttt taatttaaaa 1620

ggatctaggt gaagatcctt tttgataatc tcatgaccaa aatcccttaa cgtgagtttt 1680

cgttccactg agcgtcagac cccgtagaaa agatcaaagg atcttcttga gatccttttt 1740

ttctgcgcgt aatctgctgc ttgcaaacaa aaaaaccacc gctaccagcg gtggtttgtt 1800

tgccggatca agagctacca actctttttc cgaaggtaac tggcttcagc agagcgcaga 1860

taccaaatac tgttcttcta gtgtagccgt agttaggcca ccacttcaag aactctgtag 1920

caccgcctac atacctcgct ctgctaatcc tgttaccagt ggctgctgcc agtggcgata 1980

agtcgtgtct taccgggttg gactcaagac gatagttacc ggataaggcg cagcggtcgg 2040

gctgaacggg gggttcgtgc acacagccca gcttggagcg aacgacctac accgaactga 2100

gatacctaca gcgtgagcta tgagaaagcg ccacgcttcc cgaagggaga aaggcggaca 2160

ggtatccggt aagcggcagg gtcggaacag gagagcgcac gagggagctt ccagggggaa 2220

acgcctggta tctttatagt cctgtcgggt ttcgccacct ctgacttgag cgtcgatttt 2280

tgtgatgctc gtcagggggg cggagcctat ggaaaaacgc cagcaacgcg gcctttttac 2340

ggtt 2344

<210> 25
<211> 2529
<212> DNA
<213> Artificial Sequence

<220>
<223> pAGT864

<400> 25
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aggagagagt gtggtcggat tagaagccgc cgcacgggcg acagccctcc 2280

gacccgcgcc gcactgctcc gaacaatagg ggttgagaaa gccggcccac cggcggtctt 2340

tcgtccggcc aagttccact tgtccggtct atataaatgc aaaaactgca tataaacttt 2400

aactaatact ttcaacaatt tcggtttgta ttgctcctca ttctcatctt attaaagtat 2460

catcaagaaa ttgttaatat acctctatac attaacatca taaaaaaaaa acaaatgtga 2520

gaccacgaa 2529

<210> 26
<211> 2530
<212> DNA
<213> Artificial Sequence

<220>
<223> pAGT865

<400> 26
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aggagttgtg ttgttcggat tagaagccgc cgaccgggcg acagccctcc 2280

gactcgcgcc gcactgctcc gaacaatctg gagatgcact tgtggcccac cggcggtctt 2340

tcgtccgtgc ggcctgttgc gtagcagagc tatataaatg caaaaactgc atatccacta 2400

taactaatac tttcaacaat ttggtgttgt attgcttttc attctaatgt tattcaagta 2460

tcatcaagaa attgttaata tacctctata gcttaacgtc aaagaaaaaa aacaaatgtg 2520

agaccacgaa 2530

<210> 27
<211> 2530
<212> DNA
<213> Artificial Sequence

<220>
<223> pAGT866

<400> 27
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aggagggctt agcgtcggat tagaagccgc cgtacgggcg acagccctcc 2280

gaaccgcgcc gcactgctcc gaacaatctg cgccaaaaag ggcgtggcac cggcggtctt 2340

tcgtccgtgc cttcctttgt agattaagtc tatataaatg caaaaactgc ataaccacta 2400

taactaatac tttcaacaat ttcgggttgt attactcttt attctcatct tataaaagta 2460

tcaacaagaa atagttaata tacctctata ccttaacatc ataaaaaaaa aaaaaatgtg 2520

agaccacgaa 2530

<210> 28
<211> 2558
<212> DNA
<213> Artificial Sequence

<220>
<223> pAGT529

<400> 28
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aggagtcctt ttggaaagct atacttcgga gcactgttga gcgaaggctc 2280

attagatata ttttctgtca ttttccttaa cccaaaaata agggagaggg tccaaaaagc 2340

gctcggacaa ctgttgaccg tgatccgaag gactggctat acagtgttca caaaatagcc 2400

aagctgaaaa taatgtgtag cttttagcta tgttcagtta gtttggctag caaagatata 2460

aaagcaggtc ggaaatattt atgggcatta ttatgcagag catcaacatg ataaaaaaaa 2520

acagttgaat attccctcaa aaaatgtgag accacgaa 2558

<210> 29
<211> 2446
<212> DNA
<213> Artificial Sequence

<220>
<223> pAGT537

<400> 29
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240
cttgaccgaa cgcagcggtg gtaacggcgc agtggcggtt ttcatggctt gttatgactg 300
tttttttggg gtacagtcta tgcctcgggc atccaagcag caagcgcgtt acgccgtggg 360
tcgatgtttg atgttatgga gcagcaacga tgttacgcag cagggcagtc gccctaaaac 420
aaagttaaac atcatgaggg aagcggtgat cgccgaagta tcgactcaac tatcagaggt 480
agttggcgtc atcgagcgcc atctcgaacc gacgttgctg gccgtacatt tgtacggctc 540
cgcagtggat ggcggcctga agccacacag cgatattgat ttgctggtta cggtgaccgt 600
aaggcttgat gaaacaacgc ggcgagcttt gatcaacgac cttttggaaa cttcggcttc 660
ccctggagag agcgagattc tccgcgctgt agaagtcacc attgttgtgc acgacgacat 720
cattccgtgg cgttatccag ctaagcgcga actgcaattt ggagaatggc agcgcaatga 780
cattcttgca ggtatcttcg agccagccac gatcgacatt gatctggcta tcttgctgac 840
aaaagcaaga gaacatagcg ttgccttggt aggtccagcg gcggaggaac tctttgatcc 900
ggttcctgaa caggatctat ttgaggcgct aaatgaaacc ttaacgctat ggaactcgcc 960
gcccgactgg gctggcgatg agcgaaatgt agtgcttacg ttgtcccgca tttggtacag 1020
cgcagtaacc ggcaaaatcg cgccgaagga tgtcgctgcc gactgggcaa tggagcgcct 1080
gccggcccag tatcagcccg tcatacttga agctagacag gcttatcttg gacaagaaga 1140
agatcgcttg gcctcgcgcg cagatcagtt ggaagaattt gtccattacg taaaaggcga 1200
gatcaccaag gtagtcggca aataactgtc agaccaagtt tactcatata tactttagat 1260
tgatttaaaa cttcattttt aatttaaaag gatctaggtg aagatccttt ttgataatct 1320
catgaccaaa atcccttaac gtgagttttc gttccactga gcgtcagacc ccgtagaaaa 1380
gatcaaagga tcttcttgag atcctttttt tctgcgcgta atctgctgct tgcaaacaaa 1440
aaaaccaccg ctaccagcgg tggtttgttt gccggatcaa gagctaccaa ctctttttcc 1500
gaaggtaact ggcttcagca gagcgcagat accaaatact gtccttctag tgtagccgta 1560
gttaggccac cacttcaaga actctgtagc accgcctaca tacctcgctc tgctaatcct 1620
gttaccagtg gctgctgcca gtggcgataa gtcgtgtctt accgggttgg actcaagacg 1680
atagttaccg gataaggcgc agcggtcggg ctgaacgggg ggttcgtgca cacagcccag 1740
cttggagcga acgacctaca ccgaactgag atacctacag cgtgagctat gagaaagcgc 1800
cacgcttccc gaagggagaa aggcggacag gtatccggta agcggcaggg tcggaacagg 1860
agagcgcacg agggagcttc cagggggaaa cgcctggtat ctttatagtc ctgtcgggtt 1920
tcgccacctc tgacttgagc gtcgattttt gtgatgctcg tcaggggggc ggagcctatg 1980
gaaaaacgcc agcaacgcgg cctttttacg gttcctggcc ttttgctggc cttttgctca 2040
catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc agcttgacaa taaaaagatt cttgttttca agaacttgtc atttgtatag 2280

tttttttata ttgtagttgt tctattttaa tcaaatgtta gcgtgattta tatttttttt 2340

cgcctcgaca tcatctgccc agatgcgaag ttaagtgcgc agaaagtaat atcatgcgtc 2400

aatcgtatgt gaatgctggt cgctatactg cgcttgagac cacgaa 2446

<210> 30
<211> 2738
<212> DNA
<213> Artificial Sequence

<220>
<223> pAGT538

<400> 30
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc agcttctgct attctaaaac gggtactgta cagttagtac attgagtcga 2280

aatatacgaa attattgttc ataattttca tcctggctct ttttttcttc aaccatagtt 2340

aaatggacag ttcatatctt aactctaata atacttttct agttcttatc cttttccgtc 2400

gcaccgcaga ttttatcata gtattaaatt tatattttgt tcgtaaaaag aaaaatttgt 2460

gagcgttacc gctcgtttca ttacccgaag gctgtttcag tagaccactg attaagtaag 2520

tagatgaaaa aatttcatca ccatgaaaga gttcgatgag agctactttt tcaaatgctt 2580

aacagctaac cgccattcaa taatgttacg ctctcttcat tctgcggcta cgttatctaa 2640

caagaggttt tactctctca tatctcattc aaatagaaag aacataatca aaaagcttct 2700

acgtcaccct tctttcgatc cacgcttgag accacgaa 2738

<210> 31
<211> 2575
<212> DNA
<213> Artificial Sequence

<220>
<223> pAGT539

<400> 31
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc agcttgcgaa tttcttatga tttatgattt ttattattaa ataagttata 2280

aaaaaaataa gtgtatacaa attttaaagt gactcttagg ttttaaaacg aaaattctta 2340

ttcttgagta actctttcct gtaggtcagg ttgctttctc aggtatagca tgaggtcgct 2400

cttattgacc acacctctac cggcatgccg agcaaatgcc tgcaaatcgc tccccatttc 2460

acccaattgt agatatgcta actccagcaa tgagttgatg aatctcggtg tgtattttat 2520

gtcctcagag gacaacacct gttgtaatcg ttcttccacc gcttgagacc acgaa 2575

<210> 32
<211> 2712
<212> DNA
<213> Artificial Sequence

<220>
<223> pAGT540

<400> 32
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc agctttcgaa tttacgtagc ccaatctacc actttttttt ttcatttttt 2280

aaagtgttat acttagttat gctctaggat aatgaactac tttttttttt tttttttact 2340

gttatcataa atatatatac cttattgttg tttgcaaccg tcggttaatt ccttatcaag 2400

gttccccaag ttcggatcat taccatcaat ttccaacatt ttcatgagtt cttcttcttc 2460

attaccgtgt tttagggggc tgttcgcact tccaataggg ctatcaccaa gctgttctaa 2520

ttcgtccaaa agttcagtaa cacgatcttt atgctttagc tcgtcataat ctttcaattc 2580

ataaatattt acaatttcgt ctacgatatt aaattgcctc ttgtaggtgc ctatcttttc 2640

cttatgctct tcattttcac cgttttcttg aaaccaaaca ccgaactcac tacgcacgct 2700

tgagaccacg aa 2712

<210> 33

<211>  3025
<212>  DNA
<213>  Artificial Sequence

<220>
<223>  pAGT1372

<400>  33
gtggctcttc agtggacgaa agggcctcgt gatacgccta tttttatagg ttaatgtcat      60

gataataatg gtttcttaga cgtcaggtgg cacttttcgg ggaaatgtgc gcggaacccc     120

tatttgttta tttttctaaa tacattcaaa tatgtatccg ctcatgagac aataaccctg     180

ataaatgctt caataatatt gaaaaaggaa gagtatgcgc tcacgcaact ggtccagaac     240

cttgaccgaa cgcagcggtg gtaacggcgc agtggcggtt ttcatggctt gttatgactg     300

tttttttggg gtacagtcta tgcctcgggc atccaagcag caagcgcgtt acgccgtggg     360

tcgatgtttg atgttatgga gcagcaacga tgttacgcag cagggcagtc gccctaaaac     420

aaagttaaac atcatgaggg aagcggtgat cgccgaagta tcgactcaac tatcagaggt     480

agttggcgtc atcgagcgcc atctcgaacc gacgttgctg gccgtacatt tgtacggctc     540

cgcagtggat ggcggcctga agccacacag cgatattgat ttgctggtta cggtgaccgt     600

aaggcttgat gaaacaacgc ggcgagcttt gatcaacgac cttttggaaa cttcggcttc     660

ccctggagag agcgagattc tccgcgctgt agaagtcacc attgttgtgc acgacgacat     720

cattccgtgg cgttatccag ctaagcgcga actgcaattt ggagaatggc agcgcaatga     780

cattcttgca ggtatcttcg agccagccac gatcgacatt gatctggcta tcttgctgac     840

aaaagcaaga gaacatagcg ttgccttggt aggtccagcg gcggaggaac tctttgatcc     900

ggttcctgaa caggatctat ttgaggcgct aaatgaaacc ttaacgctat ggaactcgcc     960

gcccgactgg gctggcgatg agcgaaatgt agtgcttacg ttgtcccgca tttggtacag    1020

cgcagtaacc ggcaaaatcg cgccgaagga tgtcgctgcc gactgggcaa tggagcgcct    1080

gccggcccag tatcagcccg tcatacttga agctagacag gcttatcttg gacaagaaga    1140

agatcgcttg gcctcgcgcg cagatcagtt ggaagaattt gtccattacg taaaaggcga    1200

gatcaccaag gtagtcggca aataactgtc agaccaagtt tactcatata tactttagat    1260

tgatttaaaa cttcattttt aatttaaaag gatctaggtg aagatccttt ttgataatct    1320

catgaccaaa atcccttaac gtgagttttc gttccactga gcgtcagacc ccgtagaaaa    1380

gatcaaagga tcttcttgag atcctttttt tctgcgcgta atctgctgct tgcaaacaaa    1440

aaaaccaccg ctaccagcgg tggtttgttt gccggatcaa gagctaccaa ctctttttcc    1500

gaaggtaact ggcttcagca gagcgcagat accaaatact gtccttctag tgtagccgta    1560

gttaggccac cacttcaaga actctgtagc accgcctaca tacctcgctc tgctaatcct    1620

gttaccagtg gctgctgcca gtggcgataa gtcgtgtctt accgggttgg actcaagacg    1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aaatgcgtgg tctgaacaag atctcatgct ctctgagcct tcaaaccgag 2280

aaattgtgct atgaggacaa cgacaatgat ctggacgaag agttaatgcc gaaacacatt 2340

gccctgatta tggatggcaa tcgccgttgg gccaaagata aaggtctgga tgtaagtgaa 2400

gggcataaac acctctttcc gaaactgaaa gaaatctgcg atatcagttc gaaactcggc 2460

attcaggtga ttacagcgtt tgcattctcg acagaaaact ggaagcgcgc taaaggcgaa 2520

gtggattttc tgatgcagat gttcgaagag ctgtatgacg aatttagccg gtcaggggtt 2580

cgtgtgagca ttattggctg taaaaccgat ctgcctatga cccttcagaa gtgtatcgct 2640

ctgacggaag aaacgacgaa aggcaacaaa ggattgcatc tggtcattgc gctgaactat 2700

ggtgggtact atgacatctt acaggcgacc aaatccattg tcaacaaagc gatgaatggc 2760

ttgctggatg tggaggatat caacaagaac ctgttcgacc aggaattaga atccaagtgt 2820

ccaaatccag atctgctgat ccgcactggt ggagatcaac gcgtatcgaa tttcctcctg 2880

tggcaattgg cgtataccga attctacttc accaaaaccc tctttccgga ttttggcgaa 2940

gaggacctga aagaagccat tatcaatttt cagcaacgcc atcgccgttt tggtggtcat 3000

acgtactaag ctttgagacc acgaa 3025

<210> 34
<211> 4482
<212> DNA
<213> Artificial Sequence

<220>
<223> pAGT1041

<400> 34
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240
cttgaccgaa cgcagcggtg gtaacggcgc agtggcggtt ttcatggctt gttatgactg 300
tttttttggg gtacagtcta tgcctcgggc atccaagcag caagcgcgtt acgccgtggg 360
tcgatgtttg atgttatgga gcagcaacga tgttacgcag cagggcagtc gccctaaaac 420
aaagttaaac atcatgaggg aagcggtgat cgccgaagta tcgactcaac tatcagaggt 480
agttggcgtc atcgagcgcc atctcgaacc gacgttgctg gccgtacatt tgtacggctc 540
cgcagtggat ggcggcctga agccacacag cgatattgat ttgctggtta cggtgaccgt 600
aaggcttgat gaaacaacgc ggcgagcttt gatcaacgac cttttggaaa cttcggcttc 660
ccctggagag agcgagattc tccgcgctgt agaagtcacc attgttgtgc acgacgacat 720
cattccgtgg cgttatccag ctaagcgcga actgcaattt ggagaatggc agcgcaatga 780
cattcttgca ggtatcttcg agccagccac gatcgacatt gatctggcta tcttgctgac 840
aaaagcaaga gaacatagcg ttgccttggt aggtccagcg gcggaggaac tctttgatcc 900
ggttcctgaa caggatctat ttgaggcgct aaatgaaacc ttaacgctat ggaactcgcc 960
gcccgactgg gctggcgatg agcgaaatgt agtgcttacg ttgtcccgca tttggtacag 1020
cgcagtaacc ggcaaaatcg cgccgaagga tgtcgctgcc gactgggcaa tggagcgcct 1080
gccggcccag tatcagcccg tcatacttga agctagacag gcttatcttg gacaagaaga 1140
agatcgcttg gcctcgcgcg cagatcagtt ggaagaattt gtccattacg taaaaggcga 1200
gatcaccaag gtagtcggca aataactgtc agaccaagtt tactcatata tactttagat 1260
tgatttaaaa cttcattttt aatttaaaag gatctaggtg aagatccttt ttgataatct 1320
catgaccaaa atcccttaac gtgagttttc gttccactga gcgtcagacc ccgtagaaaa 1380
gatcaaagga tcttcttgag atcctttttt tctgcgcgta atctgctgct tgcaaacaaa 1440
aaaaccaccg ctaccagcgg tggtttgttt gccggatcaa gagctaccaa ctctttttcc 1500
gaaggtaact ggcttcagca gagcgcagat accaaatact gtccttctag tgtagccgta 1560
gttaggccac cacttcaaga actctgtagc accgcctaca tacctcgctc tgctaatcct 1620
gttaccagtg gctgctgcca gtggcgataa gtcgtgtctt accgggttgg actcaagacg 1680
atagttaccg gataaggcgc agcggtcggg ctgaacgggg ggttcgtgca cacagcccag 1740
cttggagcga acgacctaca ccgaactgag atacctacag cgtgagctat gagaaagcgc 1800
cacgcttccc gaagggagaa aggcggacag gtatccggta agcggcaggg tcggaacagg 1860
agagcgcacg agggagcttc cagggggaaa cgcctggtat ctttatagtc ctgtcgggtt 1920
tcgccacctc tgacttgagc gtcgattttt gtgatgctcg tcaggggggc ggagcctatg 1980
gaaaaacgcc agcaacgcgg cctttttacg gttcctggcc ttttgctggc cttttgctca 2040
catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aaatgtgcag ccacagtacc ccttcatcaa tgaatggttt cgaagatgca 2280

agggatagaa taagggaaag ttttgggaaa gtagagttat ctccttcttc ctatgacaca 2340

gcatgggtag ctatggtccc ttcaaaacat tcactaaatg agccatgttt tccacaatgt 2400

ttggattgga ttattgaaaa tcaaagagaa gatggatctt ggggactaaa ccctagccat 2460

ccattgcttc ttaaggactc actttcttcc actcttgcat gtttgcttgc actaaccaaa 2520

tggagagttg gagatgagca aatcaaaaga ggccttggct ttattgaaac ccagagttgg 2580

gcaattgata acaaggatca aatttcacct ctaggatttg aaattatatt tcccagtatg 2640

atcaagtctg cagaaaaact aaacttaaat ctagcaatta acaaaagaga ttcaacaatt 2700

aaaagagcat tacagaatga gttcacgagg aatattgaat atatgagtga aggatttggt 2760

gaattatgtg attggaagga aataataaag ttacatcaaa ggcaaaatgg ttcattattt 2820

gattcaccag ccactactgc agctgccttg atttaccatc agcatgataa aaaatgctat 2880

gaatatctta attcaatctt gcaacaacac aaaaattggg ttcccactat gtatccaaca 2940

aagatacatt cattgctttg cttggttgat acacttcaaa atcttggagt acatcggcat 3000

tttaaatcag aaataaagaa agccctagat gaaatataca ggctatggca acaaaagaat 3060

gaagaaattt tctcaaatgt cacccattgt gctatggctt ttcgacttct aaggataagc 3120

tactatgatg tctcctcaga tgaactagca gaatttgtgg atgaagaaca tttctttgca 3180

acaagtggga aatatacaag tcatgttgaa attcttgaac tccacaaagc atcacaattg 3240

gctattgatc atgagaaaga tgacattttg gataagatta acaattggac aagaacattt 3300

atggagcaaa aactcttaaa caatggcttc atagatagga tgtcaaaaaa ggaggtggaa 3360

cttgctttga ggaattttta tatcatatct gatctagcag aaaatagaag atatataaag 3420

tcatacgaag agaacaattt taaaatctta aaagcagctt ataggtcacc taacattaac 3480

aataaggact tgtttatatt ttcaatacgc gactttgaat tatgccaagc tcaacaccaa 3540

gaagaacttc aacaactcaa gaggtggttt gaagattgta gattggacca actcggactt 3600

tcggaacaat ttatatctgc tagttactta tgtgctattc ctattgtccc cgggcctgaa 3660

ttatccgatg ctcgtctcgt gtacgcgaaa tacgtcatgc tcttgactat tgtcgatgat 3720

catttcgaga gttttgcatc tacagatgaa tgtctcaaca tcattgaatt agtagaaagg 3780

tgggatgact atgcaagtgt aggttataaa tctgagaggg ttaaagtttt attttcaatg 3840

ttttacaaat caatagagga gattgcaaca attgctgaaa ttaaacaagg acgatctgtc 3900

aaaaatcacc ttattaattt gtggcttaaa gtgatgaagt tgatgttgat ggaacgagta 3960

gagtggtgtt ctggcaagac aataccaaga atagaagagt atttgtatgt tagttctata 4020

acatttggtt caagattgat tcctctcaca acacaatatt ttattggaat aaaaatatcc 4080

aaagatcttt tagaaagtga tgaaatttat ggtttatgca attttaccgg tatagtcttg 4140

aggctcctca atgatttaca agattccaag agagaacaaa aggagggctc aataaattta 4200

gtcacattac taatgaaaag tatctctgag gaagaagcta taatgaagat gaaggaaatc 4260

ttggaaatga aaagaagaga gttatttaaa atggttttag ttcaaaaaaa gggaagccaa 4320

ttgcctcaat tatgcaaaga aatattttgg aggacatgca aatgggctca tttcacttat 4380

tcacaaactg atagatatag atttccagag gaaatggaga atcacattga tgaagtcttt 4440

tacaaactac tcaatcatgg gctttagctt tgagaccacg aa 4482

<210> 35
<211> 3736
<212> DNA
<213> Artificial Sequence

<220>
<223> pAGT938

<400> 35
gtggctcttc agtggacgaa agggcctcgt gatacgccta tttttatagg ttaatgtcat 60

gataataatg gtttcttaga cgtcaggtgg cacttttcgg ggaaatgtgc gcggaacccc 120

tatttgttta tttttctaaa tacattcaaa tatgtatccg ctcatgagac aataaccctg 180

ataaatgctt caataatatt gaaaaaggaa gagtatgcgc tcacgcaact ggtccagaac 240

cttgaccgaa cgcagcggtg gtaacggcgc agtggcggtt ttcatggctt gttatgactg 300

tttttttggg gtacagtcta tgcctcgggc atccaagcag caagcgcgtt acgccgtggg 360

tcgatgtttg atgttatgga gcagcaacga tgttacgcag cagggcagtc gccctaaaac 420

aaagttaaac atcatgaggg aagcggtgat cgccgaagta tcgactcaac tatcagaggt 480

agttggcgtc atcgagcgcc atctcgaacc gacgttgctg gccgtacatt tgtacggctc 540

cgcagtggat ggcggcctga agccacacag cgatattgat ttgctggtta cggtgaccgt 600

aaggcttgat gaaacaacgc ggcgagcttt gatcaacgac cttttggaaa cttcggcttc 660

ccctggagag agcgagattc tccgcgctgt agaagtcacc attgttgtgc acgacgacat 720

cattccgtgg cgttatccag ctaagcgcga actgcaattt ggagaatggc agcgcaatga 780

cattcttgca ggtatcttcg agccagccac gatcgacatt gatctggcta tcttgctgac 840

aaaagcaaga gaacatagcg ttgccttggt aggtccagcg gcggaggaac tctttgatcc 900

ggttcctgaa caggatctat ttgaggcgct aaatgaaacc ttaacgctat ggaactcgcc 960

gcccgactgg gctggcgatg agcgaaatgt agtgcttacg ttgtcccgca tttggtacag 1020

cgcagtaacc ggcaaaatcg cgccgaagga tgtcgctgcc gactgggcaa tggagcgcct 1080

gccggcccag tatcagcccg tcatacttga agctagacag gcttatcttg gacaagaaga 1140

agatcgcttg gcctcgcgcg cagatcagtt ggaagaattt gtccattacg taaaaggcga 1200

gatcaccaag gtagtcggca aataactgtc agaccaagtt tactcatata tactttagat 1260

tgatttaaaa cttcattttt aatttaaaag gatctaggtg aagatccttt ttgataatct 1320

catgaccaaa atcccttaac gtgagttttc gttccactga gcgtcagacc ccgtagaaaa 1380

gatcaaagga tcttcttgag atcctttttt tctgcgcgta atctgctgct tgcaaacaaa 1440

aaaaccaccg ctaccagcgg tggtttgttt gccggatcaa gagctaccaa ctctttttcc 1500

gaaggtaact ggcttcagca gagcgcagat accaaatact gtccttctag tgtagccgta 1560

gttaggccac cacttcaaga actctgtagc accgcctaca tacctcgctc tgctaatcct 1620

gttaccagtg gctgctgcca gtggcgataa gtcgtgtctt accgggttgg actcaagacg 1680

atagttaccg gataaggcgc agcggtcggg ctgaacgggg ggttcgtgca cacagcccag 1740

cttggagcga acgacctaca ccgaactgag atacctacag cgtgagctat gagaaagcgc 1800

cacgcttccc gaagggagaa aggcggacag gtatccggta agcggcaggg tcggaacagg 1860

agagcgcacg agggagcttc cagggggaaa cgcctggtat ctttatagtc ctgtcgggtt 1920

tcgccacctc tgacttgagc gtcgattttt gtgatgctcg tcaggggggc ggagcctatg 1980

gaaaaacgcc agcaacgcgg cctttttacg gttcctggcc ttttgctggc cttttgctca 2040

catgttcttt cctgcgttat cccctgattc tgtggataac cgtattaccg cctttgagtg 2100

agctgatacc gctcgccgca gccgaacgac cgagcgcagc gagtcagtga gcgaggaagc 2160

ggaagagcgc ccaatacgca aaccgcctct ccccgcgcgt tggccgattc attaatcact 2220

ctgtggtctc aaatgcagtt cttcaacatc ttttcccttt tcctttttat gtctttcctc 2280

tttttgttaa ttaaatggaa gaattccaat agcaaaacca aaagattgcc tcctggtcca 2340

tggaaattac ctttaattgg aagcatgttt catttgttag gtggacttcc acatcatgtt 2400

tttagagatt tagcgaaaaa atatggacca attatgcacc ttcaactagg tgaagtttct 2460

ctagttgtta ttacttctcc tgacatggcc aaacaaatac taagaactca tgatctcgct 2520

tttgcatcta ggcccaagtt cctagttggg gagattctct tttataagtg gactgatatt 2580

cttttttctc cctatggtga ttactggaaa caaatgcgta aaatatgtct attggaattg 2640

cttagcgcaa aaaatgttaa gtcattcaac tctatacgac aagatgaagt tcatcatatg 2700

attgagtttt ttcgatcatc ttctggtgaa acaattaatg taacaaaaag gatttatcaa 2760

ttctcgagct ctatgacatg tagatcagca tttgggaaag tattcaaaga gcaaaacaaa 2820

ctcatagaac tcgttaaaga agcgtcaggc ttttttaaag gatttgatgt agctgatatg 2880

tttccatcac taaaatttct tcatgtgtta tgtggaatga agggtaaaat tatggatgct 2940

caccatgagt tagatgccat tcttgaaaac atcatcaatg agcacaagaa caatggtgaa 3000

ttaggaggcg aaggtttact tgttgcattg ctaagattaa tgaaggaggg aggctttcaa 3060

tttccaatca ccaatgacaa tatcaaagct attatttttg acatgtttgc tgggggaaca 3120

gatacttcat caacattaat tgattgggcc atggtggaaa tgatgaggaa tccaagtgta 3180

cttttgaaag ctcaagttga gataagaaat gctttcacag gaaaagaaac ttttaatgaa 3240

aacgatgttg aagaattgaa ataccttaag ttggttgtta aagaaacttt cagactccat 3300

cctccaggac cccttttgat ggcaagggaa tgtagagaag aagtagacat aaatggctat 3360

aatattcctt tgaaaacaaa agttatggtt aatgcctacg caatcggaag agatcctaaa 3420

tattggattg acgcagaatg tttcaaacct gagagatttg agcagatttc agtagatttt 3480

attggcaata actttgaatt tcttccgttt ggtagtggaa ggaggagttg tccgggaata 3540

tcatttggtc tagctaatat ttttttccca ctagctaaat tattgtatca ctttgattgg 3600

aaacttccta ttggaatcaa tccaagtgat ttggacttaa ctgaatctgc tggaataact 3660

tgtcctagaa agagtaactt atacttgatc gcgactcctc atgaacctag tcaagaatga 3720

gctttgagac cacgaa 3736

<210> 36
<211> 4327
<212> DNA
<213> Artificial Sequence

<220>
<223> pAGM1011

<400> 36
gctttgagac cacgaagtgg ctcttcagtg gacgaaaggg cctcgtgata cgcctatttt 60

tataggttaa tgtcatgata ataatggttt cttagacgtc aggtggcact tttcggggaa 120

atgtgcgcgg aacccctatt tgtttatttt tctaaataca ttcaaatatg tatccgctca 180

tgagacaata accctgataa atgcttcaat aatattgaaa aaggaagagt atgcgctcac 240

gcaactggtc cagaaccttg accgaacgca gcggtggtaa cggcgcagtg gcggttttca 300

tggcttgtta tgactgtttt tttggggtac agtctatgcc tcgggcatcc aagcagcaag 360

cgcgttacgc cgtgggtcga tgtttgatgt tatggagcag caacgatgtt acgcagcagg 420

gcagtcgccc taaaacaaag ttaaacatca tgagggaagc ggtgatcgcc gaagtatcga 480

ctcaactatc agaggtagtt ggcgtcatcg agcgccatct cgaaccgacg ttgctggccg 540

tacatttgta cggctccgca gtggatggcg gcctgaagcc acacagcgat attgatttgc 600

tggttacggt gaccgtaagg cttgatgaaa caacgcggcg agctttgatc aacgaccttt 660

tggaaacttc ggcttcccct ggagagagcg agattctccg cgctgtagaa gtcaccattg 720

ttgtgcacga cgacatcatt ccgtggcgtt atccagctaa gcgcgaactg caatttggag 780

aatggcagcg caatgacatt cttgcaggta tcttcgagcc agccacgatc gacattgatc 840

tggctatctt gctgacaaaa gcaagagaac atagcgttgc cttggtaggt ccagcggcgg 900

aggaactctt tgatccggtt cctgaacagg atctatttga ggcgctaaat gaaaccttaa 960

cgctatggaa ctcgccgccc gactgggctg gcgatgagcg aaatgtagtg cttacgttgt 1020

cccgcatttg gtacagcgca gtaaccggca aaatcgcgcc gaaggatgtc gctgccgact 1080

gggcaatgga gcgcctgccg gcccagtatc agcccgtcat acttgaagct agacaggctt 1140

atcttggaca agaagaagat cgcttggcct cgcgcgcaga tcagttggaa gaatttgtcc 1200

attacgtaaa aggcgagatc accaaggtag tcggcaaata actgtcagac caagtttact 1260

catatatact ttagattgat ttaaaacttc atttttaatt taaaaggatc taggtgaaga 1320

tcctttttga taatctcatg accaaaatcc cttaacgtga gttttcgttc cactgagcgt 1380

cagaccccgt agaaaagatc aaaggatctt cttgagatcc tttttttctg cgcgtaatct 1440

gctgcttgca aacaaaaaaa ccaccgctac cagcggtggt ttgtttgccg gatcaagagc 1500

taccaactct ttttccgaag gtaactggct tcagcagagc gcagatacca aatactgtcc 1560

ttctagtgta gccgtagtta ggccaccact tcaagaactc tgtagcaccg cctacatacc 1620

tcgctctgct aatcctgtta ccagtggctg ctgccagtgg cgataagtcg tgtcttaccg 1680

ggttggactc aagacgatag ttaccggata aggcgcagcg gtcgggctga acggggggtt 1740

cgtgcacaca gcccagcttg gagcgaacga cctacaccga actgagatac ctacagcgtg 1800

agctatgaga aagcgccacg cttcccgaag ggagaaaggc ggacaggtat ccggtaagcg 1860

gcagggtcgg aacaggagag cgcacgaggg agcttccagg gggaaacgcc tggtatcttt 1920

atagtcctgt cgggtttcgc cacctctgac ttgagcgtcg atttttgtga tgctcgtcag 1980

gggggcggag cctatggaaa aacgccagca acgcggcctt tttacggttc ctggcctttt 2040

gctggccttt tgctcacatg ttctttcctg cgttatcccc tgattctgtg gataaccgta 2100

ttaccgcctt tgagtgagct gataccgctc gccgcagccg aacgaccgag cgcagcgagt 2160

cagtgagcga ggaagcggaa gagcgcccaa tacgcaaacc gcctctcccc gcgcgttggc 2220

cgattcatta atcactctgt ggtctcaaat gacttctgct ttgtatgctt ccgatttgtt 2280

taagcagctc aagtcaatta tggggacaga ttcgttatcc gacgatgttg tacttgtgat 2340

tgcaacgacg tctttggcac tagtagctgg atttgtggtg ttgttatgga agaaaacgac 2400

ggcggatcgg agcggggagc tgaagccttt gatgatccct aagtctctta tggctaagga 2460

cgaggatgat gatttggatt tgggatccgg taagactaga gtctctatct tcttcggtac 2520

gcagactgga acagctgagg gatttgctaa ggcattatcc gaagaaatca aagcgagata 2580

tgaaaaagca gcagtcaaag tcattgactt ggatgactat gctgccgatg atgaccagta 2640

tgaagagaaa ttgaagaagg aaactttggc atttttctgt gttgctactt atggagatgg 2700

agagcctact gacaatgctg ccagatttta caaatggttt acggaggaaa atgaacggga 2760

tataaagctt caacaactag catatggtgt gtttgctctt ggtaatcgcc aatatgaaca 2820

ttttaataag atcgggatag ttcttgatga agagttatgt aagaaaggtg caaagcgtct 2880

tattgaagtc ggtctaggag atgatgatca gagcattgag gatgatttta atgcctggaa 2940

agaatcacta tggtctgagc tagacaagct cctcaaagac gaggatgata aaagtgtggc 3000

aactccttat acagctgtta ttcctgaata ccgggtggtg actcatgatc ctcggtttac 3060

aactcaaaaa tcaatggaat caaatgtggc caatggaaat actactattg acattcatca 3120

tccctgcaga gttgatgttg ctgtgcagaa ggagcttcac acacatgaat ctgatcggtc 3180

ttgcattcat ctcgagttcg acatatccag gacgggtatt acatatgaaa caggtgacca 3240

tgtaggtgta tatgctgaaa atcatgttga aatagttgaa gaagctggaa aattgcttgg 3300

ccactcttta gatttagtat tttccataca tgctgacaag gaagatggct ccccattgga 3360

aagcgcagtg ccgcctcctt tccctggtcc atgcacactt gggactggtt tggcaagata 3420

cgcagacctt ttgaaccctc ctcgaaagtc tgcgttagtt gccttggcgg cctatgccac 3480

tgaaccaagt gaagccgaga aacttaagca cctgacatca cctgatggaa aggatgagta 3540

ctcacaatgg attgttgcaa gtcagagaag tcttttagag gtgatggctg cttttccatc 3600

tgcaaaaccc ccactaggag tattttttgc tgcaatagct cctcgtctac aacctcgtta 3660

ctactccatc tcatcctcgc caagattggc gccaagtaga gttcatgtta catccgcact 3720

agtatatggt ccaactccta ctggtagaat ccacaagggt gtgtgttcta cgtggatgaa 3780

gaatgcagtt cctgcggaga aaagtcatga atgtagtgga gccccaatct ttattcgagc 3840

atctaatttc aagttaccat ccaacccttc aactccaatc gttatggtgg gacctgggac 3900

tgggctggca ccttttagag gttttctgca ggaaaggatg gcactaaaag aagatggaga 3960

agaactaggt tcatctttgc tgttctttgg gtgtagaaat cgacagatgg actttatata 4020

cgaggatgag ctcaataatt ttgttgatca aggcgtaata tctgagctca tcatggcatt 4080

ctcccgtgaa ggagctcaga aggagtatgt tcaacataag atgatggaga aggcagcaca 4140

agtttgggat ctaataaagg aagaaggata tctctatgta tgcggtgatg ctaagggcat 4200

ggcgagggac gtccaccgaa ctctacacac cattgttcag gagcaggaag gtgtgagttc 4260

gtcagaggca gaggctatag ttaagaaact tcaaaccgaa ggaagatacc tcagagatgt 4320

ctggtga 4327

## Description

### FIELD OF THE INVENTION

The present invention provides a nucleic acid or gene encoding a protein producing novel compounds in plants that provide plants with improved pest resistance. The invention also provides the protein. Further provided are a recombinant construct comprising the nucleic acid, a vector or plasmid comprising the recombinant construct, and prokaryotic and eukaryotic cells and organisms such as plants containing the nucleic acid or gene. The invention further provides processes of generating a plant or plant cell capable of producing the novel compounds, e.g. for increasing insect pest resistance of the plant. The invention also provides a method of selecting a plant having increased pest resistance. Moreover, the invention provides the novel compounds, the use of the compounds for increasing pest resistance of a plant, a process of producing the novel compounds, and pesticidal compositions containing the compounds.

### BACKGROUND OF THE INVENTION

Some wild tomato species display a significantly stronger resistance to a variety of insect pests than the cultivated tomato (*Solanum lycopersicum*). In many cases, this resistance can be traced to compounds produced on the leaf surface in glandular trichomes (Glas et al., 2012). One species that has been particularly investigated is *Solanum habrochaites.* Because *S*. *habrochaites* can be crossed with *S*. *lycopersicum,* it is possible to introduce these insect resistance traits into the cultivated tomato. For this, however, knowledge of which genes are necessary and sufficient for the biosynthesis of these compounds would be of great value, since molecular markers could then be developed to assist and accelerate the breeding process. Furthermore, availability of the genes required for the biosynthesis of these compounds would allow their production in other unrelated species by transgenic routes. Within *S*. *habrochaites,* there are different sesquiterpene chemotypes and some of them have been associated with insect resistance. The sesquiterpenes produced by *S*. *habrochaites* can be derived from either *trans,trans-*farnesyl diphosphate (*E,E*-FPP) or *cis*,*cis*-farnesyl diphosphate (*Z,Z*-FPP). The *E,E*-FPP-derived compounds are essentially of the germacrene type (germacrene B and D) and the sesquiterpene synthases responsible for this have been characterized (van Der Hoeven et al., 2000).

Sesquiterpenes derived from *Z,Z*-FPP can be classified in two types. The santalene/ bergamotene type and the zingiberene type. Santalene and bergamotene are typically oxidized to carboxylic acids which are the major compounds (Coates et al., 1988). 7-epizingiberene was identified as a major sesquiterpene in certain *S*. *habrochaites* accessions (Breeden and Coates, 1994). Its biosynthesis was described in a recent publication (Bleeker et al., 2012, Schuurink, 2012). In a more recent study, a survey of the different sesquiterpene chemotypes of *S*. *habrochaites* was published (Gonzales-Vigil et al., 2012).

It is an object of the present invention to identify the origin or reason of the stronger resistance to pests such as insect pests in some wild tomato species. It is a further object to identify nucleic acids involved in the pest resistance. It is a further object to provide methods of providing the pest resistance trait to other plants, and to provide plants having this trait. It is a further object to provide markers useful for selecting plants having the pest resistance trait. A further object is to provide a method of increasing pest resistance of a plant.

### SUMMARY OF THE INVENTION

For solving the above objects, the present invention provides:
(1) A nucleic acid comprising
   (i) a nucleotide sequence as defined in SEQ ID NO: 1 or in SEQ ID NO:3, optionally with inserted intron(s);
   (ii) a nucleotide sequence having a sequence identity of at least 85% to the nucleotide sequence as defined in SEQ ID NO: 1 or in SEQ ID NO:3, optionally with inserted intron(s);
   (iii) a nucleotide sequence encoding a protein comprising an amino acid sequence as defined in SEQ ID NO: 2 or in SEQ ID NO:4;
   (iv) a nucleotide sequence encoding a protein having an amino acid sequence identity to the amino acid sequence as defined in SEQ ID NO: 2 or in SEQ ID NO:4 of at least 85%;
   (v) a nucleotide sequence encoding a protein having an amino acid sequence similarity to the amino acid sequence as defined in SEQ ID NO: 2 or in SEQ ID NO:4 of at least 90%;
   (vi) a nucleotide sequence encoding a protein having from 1 to 30 amino acid substitutions, insertions or additions to the amino acid sequence as defined in SEQ ID NO: 2.
(2) A nucleic acid comprising
   (i) a nucleotide sequence as defined in SEQ ID NO: 1 or in SEQ ID NO:3;
   (ii) a nucleotide sequence having a sequence identity of at least 97% to the nucleotide sequence as defined in SEQ ID NO: 1 or in SEQ ID NO:3;
   (iii) a nucleotide sequence encoding a protein comprising an amino acid sequence as defined in SEQ ID NO: 2 or in SEQ ID NO:4;
   (iv) a nucleotide sequence encoding a protein having an amino acid sequence identity to the amino acid sequence as defined in SEQ ID NO: 2 or in SEQ ID NO:4 of at least 94.5 %;
   (v) a nucleotide sequence encoding a protein having an amino acid sequence similarity to the amino acid sequence as defined in SEQ ID NO: 2 or in SEQ ID NO:4 of at least 97.0%;
   (vi) a nucleotide sequence encoding a protein having from 1 to 25 amino acid substitutions to the amino acid sequence as defined in SEQ ID NO: 2, and/or having from 0 to 15 non-conservative amino acid substitutions to the amino acid sequence as defined in SEQ ID NO: 2.
(3) The nucleic acid according to item (1) or (2), encoding a protein functional for the conversion of 7-epizingiberene to 9-hydroxy-7-epi-zingiberene.
(4) A protein encoded by the nucleic acid of any one of item (1) to (3).
(5) The protein according to item (4) that is functional for the conversion of 7-epizingiberene to 9-hydroxy-7-epi-zingiberene.
(6) A recombinant construct comprising the nucleic acid according to any one items (1) to (3).
(7) The recombinant construct according to item (6), wherein the nucleic acid is operably linked to an upstream promoter active in prokaryotic or eukaryotic cells, and/or the nucleic is operably linked to a downstream terminator sequence.
(8) A vector or plasmid comprising the nucleic acid according to any one of items (1) to (3), or the recombinant construct according to item (6) or (7).
(9) The vector or plasmid according to item (8), wherein said recombinant construct is flanked by a T-DNA border sequence on at least one side for permitting the Agrobacterium-mediated transfer of said recombinant construct into cells of said plant.
(10) The vector or plasmid according to item (8), further comprising a selectable marker allowing selecting for the presence of said vector or plasmid in eukaryotic or prokaryotic cells.
(11) A prokaryotic cell or a eukaryotic non-plant cell, cell line or strain comprising the nucleic acid according to any one of items (1) to (3).
(12) A prokaryotic cell or a eukaryotic cell, cell line or strain comprising the recombinant construct according to item (6) or (7), or a vector or plasmid according to any one of items (8) to (10).
(13) The prokaryotic or eukaryotic cell, cell line or strain according to item 11 or 12, further comprising a gene encoding a Z,Z-FPP synthase and/or a gene encoding an 7-epizingiberene synthase.
(14) The eukaryotic cell, cell line or strain according to any one of items (11) to (13), which is a yeast cell, cell line or strain.
(15) A use of the prokaryotic or eukaryotic cell, cell line or strain according to any one of items (11) to (14) for producing 9-hydroxy-zingiberene and/or 9-hydroxy-10,11-epoxy-zingiberene; and/or 9-hydroxy-curcumene and/or 9-hydroxy-10,11-epoxy-curcumene.
(16) A plant or material of said plant comprising the recombinant construct according to item (6) or (7), or a vector or plasmid according to any one of items (8) to (10).
(17) A process of generating a plant or plant cell capable of producing 9-hydroxy-zingiberene and/or 9-hydroxy-10,11-epoxy-zingiberene, comprising inserting the recombinant construct according to item (6) or (7), or a vector or plasmid according to any one of items (8) to (10) into a plant or plant cell.
(18) The process according to item (17), further comprising inserting a gene construct expressibly encoding a Z,Z-farnesyl diphosphate synthase and/or a gene expressibly encoding a 7-epi-zingiberene synthase.
(19) A plant, plant cell, or material of said plant, obtainable according to the process of any item (17) or (18).
(20) A process of generating a plant capable of producing 9-hydroxy-zingiberene and/or 9-hydroxy-10,11-epoxy-zingiberene, comprising crossing a plant (A) not capable of producing 9-hydroxy-zingiberene and/or 9-hydroxy-10,11-epoxy-zingiberene or producing insufficient amounts of 9-hydroxy-zingiberene and/or 9-hydroxy-10,11-epoxy-zingiberene with a plant (B) capable of producing 9-hydroxy-zingiberene and/or 9-hydroxy-10,11-epoxy-zingiberene, and selecting progeny capable of producing 9-hydroxy-zingiberene and/or 9-hydroxy-10,11-epoxy-zingiberene.
(21) The process according to item (20), wherein plant (A) does not have a nucleic acid according to item 1, 2 or 3, and plant (b) comprises a nucleic acid according to item (1), (2) or (3) in a chromosome; and/or said selection step may comprise testing progeny for the presence of a nucleic acid according to any one of items (1) to (3) in a chromosome or testing progeny for the expression of mRNA encoded by or being a nucleic acid according to any one of items (1) to (3).
(22) The process according to item (20), wherein plant (A) is a *Solanum lycopersicum* plant and plant (B) is a Solanum habrochaites plant.
(23) The process according to any one of items (20) to (22), wherein any one or more of the following positions of SEQ ID NO: 3 is used as a marker for selecting progeny capable of producing 9-hydroxy-zingiberene and/or 9-hydroxy-10,11-epoxy-zingiberene: 59, 85, 168, 243, 247, 295, 307, 309, 337, 349, 408, 444, 445, 557, 590, 602, 616, 624, 708, 776, 804, 824, 904, 965, 1060, 1076, 1153, 1218, 1295, 1376, 1404, 1405, 1432, and/or 1447.
(24) A plant or plant material thereof, obtainable according to the process of any one of items (20) to (23).
(25) A *Solanum lycopersicum* plant comprising a nucleic acid according to item (2).
(26) The Solanum *lycopersicum* plant according to item (25), further comprising a gene construct expressibly encoding a *Z,Z*-farnesyl diphosphate synthase and/or a gene expressibly encoding a 7-epizingiberene synthase.
(27) A use of a nucleotide sequence as defined in SEQ ID NO: 1 or a part thereof for identifying a plant capable of producing 9-hydroxy-zingiberene and/or 9-hydroxy-10,11-epoxy-zingiberene.
(28) A method of determining the capability of a plant for producing 9-hydroxy-zingiberene and/or 9-hydroxy-10,11-epoxy-zingiberene, comprising:
   obtaining a sample from said plant and analyzing said sample for the presence or absence of a nucleic acid as defined in item (2); or
   obtaining a sample from said plant and analyzing said sample for the presence or absence, or the amount of, expression of an mRNA encoded by a nucleic acid as defined in item (1) or (2); and/or
   obtaining a sample from said plant and analyzing said sample for its content of 9-hydroxy-zingiberene and/or 9-hydroxy-10,11-epoxy-zingiberene, and/or 9-hydroxy curcumene and/or 9-hydroxy-10,11-epoxy curcumene; and/or
   analyzing a plurality of plants in said set of plants for one or more polymorphisms at any one or more of positions 59, 85, 168, 243, 247, 295, 307, 309, 337, 349, 408, 444, 445, 557, 590, 602, 616, 624, 708, 776, 804, 824, 904, 965, 1060, 1076, 1153, 1218, 1295, 1376, 1404, 1405, 1432, and/or 1447 of SEQ ID NO: 3, and selecting a plant or plants having one or more of the nucleotides of SEQ ID NO: 3 at any one or more of these positions of SEQ ID NO:3.
(29) A method of selecting, from a set of plants, a plant having increased pest resistance compared to another plant or other plants in said set, comprising:
   analyzing a plurality of plants in said set of plants for the presence of a nucleic acid as defined in any one of item (1) to (3), and selecting a plant or plants containing said nucleic acid; or
   analyzing a plurality of plants in said set of plants for 9-hydroxy-zingiberene and/or 9-hydroxy-10,11-epoxy-zingiberene, and selecting a plant or plants having higher content or production of 9-hydroxy-zingiberene and/or 9-hydroxy-10,11-epoxy-zingiberene than one or more other plants in said set.
(30) A zingiberene derivative selected from 9-hydroxy-zingiberene and 9-hydroxy-10,11-epoxy-zingiberene.
(31) A method of selecting, from a set of plants, a plant having increased pest resistance compared to another plant or other plants in said set, comprising analyzing a plurality of plants in said set of plants for the production of 9-hydroxy-zingiberene and/or 9-hydroxy-10,11-epoxy-zingiberene, and selecting a plant or plants having higher production of 9-hydroxy-zingiberene and/or 9-hydroxy-10,11-epoxy-zingiberene than one or more other plants in said set.
(32) A use of the compound 9-hydroxy-zingiberene and/or 9-hydroxy-10,11-epoxy-zingiberene for increasing pest resistance of a plant.
(33) A method of increasing pest resistance of a plant, comprising spraying said plant or the vicinity where it grows with a composition comprising 9-hydroxy-zingiberene and/or 9-hydroxy-10,11-epoxy-zingiberene.
(34) Pesticidal composition comprising a zingiberene derivative selected from 9-hydroxy-zingiberene and 9-hydroxy-10,11-epoxy-zingiberene.
(35) A use of a protein according to item (4) for producing 9-hydroxy zingiberene and/or 9-hydroxy-10,11-epoxy-zingiberene, and/or 9-hydroxy curcumene, and/or 9-hydroxy-10,11-epoxy curcumene.
(36) A process of producing 9-hydroxy zingiberene, and/or 9-hydroxy-10,11-epoxy-zingiberene, and/or 9-hydroxy curcumene, and/or 9-hydroxy-10,11-epoxy curcumene, comprising incubating zingiberene in the presence of the protein according to item (4) and a system capable of providing reducing equivalents to said protein.

### BRIEF DESCRIPTON OF THE FIGURES

Fig. 1 GC-MS chromatograms of leaf surface extracts from *S*. *habrochaites* LA2167 (I.), *S*. *lycopersicum* LA4024 (II.) and a F1 cross between these two lines (III.). A: monoterpenoids (accumulate mostly in *S*. *lycopersicum*); B: 7-epi-zingiberene; C: germacrene D; D: 9-hydroxy zingiberene; E: 9-hydroxy-10,11-epoxy-zingiberene.
Fig. 2 Biosynthesis pathway of the zingiberene derivatives from *Solanum habrochaites* LA2167. zFPS: *Z,Z*-FPP synthase, ShZS: zingiberene synthase (also referred to herein as ShZIS), ShZPO, zingiberene polyoxidase.
Fig. 3 Electrospray (EI) mass spectra of zingiberene (A), 9-hydroxyzingiberene (B) and 9-hydroxy-10,11-epoxy-zingiberene (C).

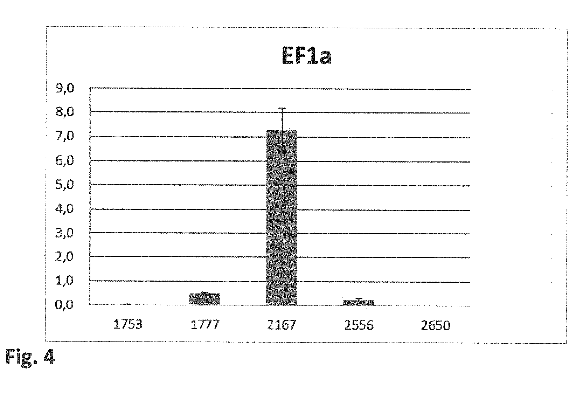

Fig. 4 Expression level (vertical axis) of Solhab01g008670 in various *S*. *habrochaites* accessions. Expression was measured by quantitative real-time RT-PCR for the Solhab01g008670 using the EF1a gene as a reference.
Fig. 5 transient expression in *N. benthamiana.* I. empty vector, II: zFPS+ ShZIS, III: FPS + ShZIS + ZPO; IV: zFPS + ShZIS + AtCPR (cytochrome P450 reductase) + ZPO. A: *ar*-curcumene B: 7-epizingiberene; C: 9-hydroxy curcumene; D: 9-hydroxy zingiberene; E: Z,Z-farnesol (dephosphorylation product of *Z,Z*-FPP); F: 9-hydroxy-10,11-epoxy curcumene; G: 9-hydroxy-10,11-epoxy-zingiberene.
Fig. 6 Mass spectra of *ar*-curcumene (A), 9-hydroxy-curcumene (B) and 9-hydroxy-10,11-epoxy-curcumene (C).
Fig. 7 Expression in yeast. I: empty vector, II: trc-zFPS + trc-ShZIS, III: trc-zFPS + trc-ShZIS + ZPO; IV: trc-zFPS + trc-ShZIS + AtCPR + ZPO. A: 7-epi-zingiberene; B: 9-hydroxy zingiberene; C: *Z,Z*-farnesol; D: 9-hydroxy-10,11-epoxy-zingiberene. In the presence of the cytochrome reductase (AtCPR) in addition to the biosynthesis genes, the zingiberene derivatives are visible.
Fig. 8 Sequence alignment of the coding sequences from Solhab01g008670 (SEQ ID NO:3) and Solyc01g008670 (SEQ ID NO:5). The sequence polymorphisms are marked in grey or white. Any of these polymorphisms can be used for genotyping. The polymorphism indicated by an inverted triangle was used for mapping using an HRM marker (see materials and methods).
Fig. 9 overview over the construction of the backbone vector for yeast expression level M vectors.
Fig. 10 Overview over the construction of yeast level M vectors. As for the level 1 vectors, the inserts from the plant level M vectors (pAGM7961, pAGM7973, pAGM7985, pAGM7997, pAGM8000, pAGM8011, pAGM8023) are cloned into the backbone vector pAGT522 using a DralII restriction/ligation reaction to give the yeast level M vectors. The end-linkers are the same as described in Weber et al. (2012).
Fig. 11 construction of a library of synthetic gal inducible promoters. The four fragments were amplified by primer extension using the primers designated in green and cloned in a level -1 vector. The four fragment were assembled in a level 0 vector to generate the library of promoters. The nucleotide sequences of the primers used are given in SEQ ID NOs: 12 to 19.

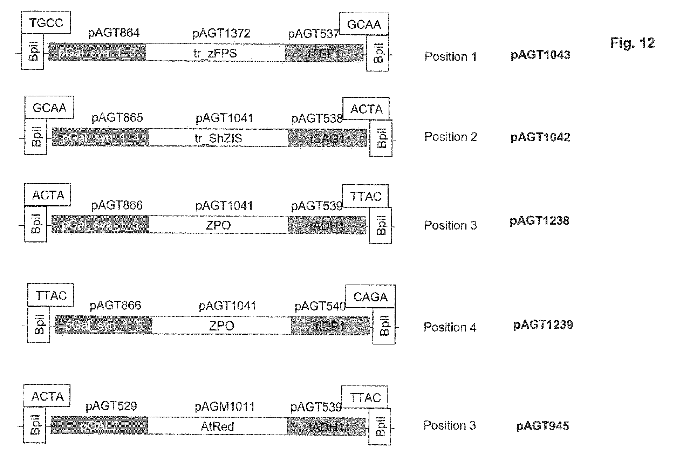

Fig. 12 shows schematically level 1 vectors for expression of zFPS, ShZIS and ZPO in yeast.

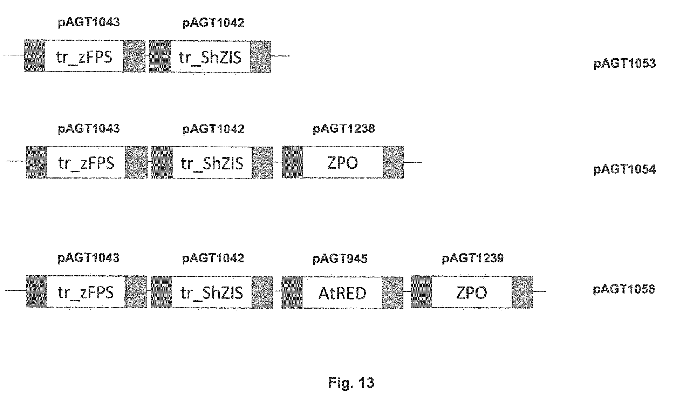

Fig. 13 Level M vectors for production of zingiberene and oxidized derivatives in yeast (all cloned in the level M vector pAGT564).

### DETAILED DESCRIPTION OF THE INVENTION

The inventors have surprisingly identified two major zingiberene derivatives in *S*. *habrochaites* accession LA2167 as 9-hydroxy-7-epi-zingiberene (herein sometimes referred to as "ZG9OH") and 10,11-epoxy-9-hydroxy-7-epizingiberene (herein sometimes referred to as "ZG9OH10Epoxy") In accession LA2167, these two compounds are the most abundant zingiberene derivatives, at levels equal to or higher than the precursor 7-epizingiberene. The invention describes inter alia the identification of a gene encoding a cytochrome P450 oxygenase (referred to herein as "zingiberene polyoxidase" or "ZPO") which is responsible for the biosynthesis of these newly identified compounds, as well as for the biosynthesis of the corresponding compounds from *ar*-curcumene. The invention provides evidence for these findings based on gene expression levels, genetic evidence, transient expression in *Nicotiana benthamiana* and expression in yeast. The gene can be used for the production of the zingiberene derivatives of the invention in transgenic organisms (e.g. microbial or plant) and the sequence polymorphisms between the *S*. *habrochaites* and *S*. *lycopersicum* alleles of this gene can be used as or to design molecular markers for the breeding of cultivated tomato plants producing the zingiberene derivatives of the invention. Other applications of the invention are described below. The inventors have also found that 9-hydroxy curcumene (C9OH) and/or 9-hydroxy-10,11-epoxy curcumene (C9OH10Epoxy) can also be biosynthesized involving ZPO.

The nucleic acid of the invention comprises, in one embodiment, the nucleotide sequence as defined in SEQ ID NO: 1 or the nucleotide sequence as defined in SEQ ID NO: 3. SEQ ID NO: 3 is the cDNA encoding ZPO in *S*. *habrochaites* accession LA2167; this cDNA encodes a ZPO protein having the amino acid sequence of SEQ ID NO: 4. SEQ ID NO: 1 is the cDNA encoding ZPO of *S*. *habrochaites* accession LA2167 truncated at the N-terminus to remove the membrane anchor. SEQ ID NO: 1 encodes a protein having the amino acid sequence of SEQ ID NO: 2 The invention also provides a nucleic acid comprising the nucleotide sequence as defined in SEQ ID NO: 1 and/or 3 with inserted intron(s). In the following, several variants of the nucleic acids of SEQ ID NO: 1 and 3 are described.

In a second embodiment, the nucleic acid of the invention comprises a nucleotide sequence having a sequence identity of at least 85%, preferably at least 90%, more preferably at least 94%, even more preferably at least 97%, and most preferably at least 98% to the nucleotide sequence as defined in SEQ ID NO: 1, optionally with inserted intron(s). In a further embodiment, the nucleic acid comprises a nucleotide sequence having a sequence identity of at least 85%, preferably at least 90%, more preferably at least 93%, even more preferably at least 97%, and most preferably at least 98% to the nucleotide sequence as defined in SEQ ID NO: 3, optionally with inserted intron(s).

In a further embodiment, the nucleic acid of the invention comprises a nucleotide sequence encoding a protein comprising an amino acid sequence as defined in SEQ ID NO: 2 or an amino acid sequence as defined in SEQ ID NO: 4.

In a further embodiment, the nucleic acid comprises a nucleotide sequence encoding a protein having an amino acid sequence identity to the amino acid sequence as defined in SEQ ID NO: 2 of at least 85%, preferably of at least 90%, more preferably of at least 92%, even more preferably of at least 94.5%, even more preferably of at least 96%, and most preferably of at least 98%. In a further embodiment, the nucleic acid comprises a nucleotide sequence encoding a protein having an amino acid sequence identity to the amino acid sequence as defined in SEQ ID NO: 4 of at least 85%, preferably of at least 90%, more preferably of at least 92%, even more preferably of at least 94.5%, even more preferably of at least 96%, and most preferably of at least 98%.

In a further embodiment, the nucleic acid comprises a nucleotide sequence encoding a protein having an amino acid sequence similarity to the amino acid sequence as defined in SEQ ID NO: 2 of at least 90%, preferably of at least 94%, more preferably of at least 97%, even more preferably of at least 98%, and most preferably of 99%. In a further embodiment, the nucleic acid comprises a nucleotide sequence encoding a protein having an amino acid sequence similarity to the amino acid sequence as defined in SEQ ID NO: 4 of at least 90%, preferably of at least 94%, more preferably of at least 97%, even more preferably of at least 98%, and most preferably of 99%.

Herein, sequence identities of nucleotide sequences are determined with the alignment software CLUSTALW (Thompson et al., 1994) with the following parameters. The cost matrix was IUB, gap open cost set to 15 and the gap extend cost set to 6.66. Sequence identities and similarities between amino acid sequences are determined with CULSTALW (Thompson et al., 1994). The BLOSUM cost matrix was used with a gap open cost of 10 and a gap extend cost of 0.1.

In a further embodiment, the nucleic acid comprises a nucleotide sequence encoding a protein having from 1 to several amino acid residue substitutions, insertions, additions or deletions, preferably substitutions, insertions, or additions, to the amino acid sequence as defined in SEQ ID NO: 2 or as defined in SEQ ID NO: 4. In a further embodiment, the nucleic acid comprises a nucleotide sequence encoding a protein having from 1 to 30, preferably from 1 to 25, more preferably from 1 to 20, even more preferably from 1 to 15, even more preferably from 1 to 10, and most preferably from 1 to 5 amino acid residue substitutions, insertions, additions or deletions, preferably substitutions, insertions, or additions, to the amino acid sequence as defined in SEQ ID NO: 2. In a further embodiment, the nucleic acid comprises a nucleotide sequence encoding a protein having from 1 to 30, preferably from 1 to 25, more preferably from 1 to 20, even more preferably from 1 to 15, even more preferably from 1 to 10, and most preferably from 1 to 5 amino acid residue substitutions, insertions, additions or deletions, preferably substitutions, insertions, or additions, to the amino acid sequence as defined in SEQ ID NO: 4. Amino acid residue addition(s) means an addition of one or more amino acid residues to an end of the amino acid sequence of SEQ ID NO: 2 or 4, while insertion(s) means insertion of one or more amino acid residues within the amino acid sequence of SEQ ID NO: 2 or 4. In one embodiment, one or more additions or substitutions to the amino acid sequence of SEQ ID NO: 2 or 4 are allowed, whereby the upper limit of additions or substitutions may be 30, preferably 25, more preferably 20, even more preferably 15, even more preferably 10, and most preferably 5 amino acid residues. The total number of additions, substitutions, additions, and deletions together determine the number of "amino acid residue substitutions, insertions, additions, or deletions" or, as the case may be, "substitutions, insertions, or additions", or "substitutions or additions". Thus, these operations may be combined. In a further embodiment, the nucleic acid of the invention comprises a nucleotide sequence encoding a protein having from 1 to 15 non-conservative amino acid substitutions or additions to the amino acid sequence as defined in SEQ ID NO: 2.

The nucleic acid of the invention in the embodiments described above may consist of the nucleotide sequences defined above.

The nucleic acids of the invention may be an isolated nucleic acid.

The variants of a nucleic acid of SEQ ID NO: 1 or SEQ ID NO:3 described above preferably encode a ZPO, i.e. a protein functional for the conversion of 7-epizingiberene to ZG9OH. This functionality of a variant can be tested for example by co-expressing the variant with the *Z,Z*-farnesyl diphosphate synthase from *Solanum habrochaites* (Uniprot accession N° B8XA40) and the 7-epizingiberene synthase from *S*. *habrochaites* (GenBank accession N°AFJ67807) transiently in *Nicotiana benthamiana.* For such transient assays, the cDNA encoding the proteins may be cloned in an *Agrobacterium* T-DNA vector and placed under the control of a strong constitutive promoters such as the 35S promoter from the Cauliflower Mosaic Virus (CaMV) and terminator such as the nos terminator. *Agrobacterium* strains containing these T-DNA vectors can then be (co-)infiltrated in the leaves of *N. benthamiana* and, after 4 to 6 days, the presence of a product such as ZG9OH can be detected by hexane extraction of the leaves and analysis by GC-MS. A content of ZG9OH in leaves higher than in control leaves not transiently transfected with a T-DNA vector encoding the variant means that the variant encodes a protein that is functional for the conversion of 7-epi-zingiberene to 9-hydroxy-7-epizingiberene. Analogously, such test may be performed making use of the capability of ZPO to convert 7-epizingiberene to ZG9OH10Epoxy. In this case, a content of ZG9OH10Epoxy in leaves higher than in control leaves not transiently transfected with a T-DNA vector encoding the variant nucleic acid means that the variant encodes a protein that is functional for the conversion of 7-epi-zingiberene to ZG9OH10Epoxy.

The invention provides a recombinant construct comprising the nucleic acid of the invention. The recombinant construct may comprise the nucleic acid of the invention such that the latter is expressible in desired prokaryotic or eukaryotic cells or in plants. For this purpose, the recombinant construct may contain a promoter upstream of the nucleic acid such that the nucleic acid is under the control of the promoter for expression in the cells or the plant. The promoter should be active or functional in the prokaryotic or eukaryotic cells where the nucleic acid should be expressed. The recombinant construct may further comprise other sequences such as regulatory sequences for expression of the nucleic acid, such as a 3' UTR (3' non-translated sequence) or a terminator downstream of the nucleic acid of the invention. Both prokaryotic and eukaryotic expression of nucleic acids is known to the skilled person. The recombinant construct may further comprise other desired genes or gene constructs that are desired to be co-expressed with the nucleic acid of the invention. For example, the recombinant construct may further comprise a gene construct expressibly encoding a Z,Z-farnesyl diphosphate synthase and/or a gene construct expressibly encoding a 7-epi-zingiberene synthase, as done in the examples. The recombinant construct may additionally comprise a gene construct for co-expression of a cytochrome P-450 reductase functional with the ZPO of the invention. The reductase should be functional with the ZPO and should therefore be a plant cytochrome P-450 reductase, such as from *Arabidopsis.* In one embodiment, a recombinant construct comprises a gene construct encoding a ZPO, a gene construct encoding a Z,Z-farnesyl diphosphate synthase, a gene construct encoding a 7-epi-zingiberene synthase, and a gene construct encoding a cytochrome P-450 reductase (see examples).

The nucleic acid construct may comprise a further nucleic acid or gene to be expressed in the cells or in the plants, e.g., for expression in plants, a gene encoding a suppressor of gene silencing such as the P19 protein may be included. Such use of the P19 protein is described in WO 2014/187571. Expression of such further gene may be under the control of the same or a different promoter as the promoter used for expressing the nucleic acid of the invention. The recombinant construct may further comprise a selectable marker gene for selecting cells containing the recombinant construct, e.g. during cloning or when transforming the prokaryotic or eukaryotic cells.

For expression in plants or plant cells using *Agrobacterium*-mediated transformation, the recombinant construct may be flanked by a T-DNA border sequence on at least one side, which permits the transfer of said recombinant construct into cells of the plant by *Agrobacterium*-mediated transformation.

The term "gene" means a DNA sequence comprising a region (transcribed region), which is transcribed into a RNA molecule (e.g. a mRNA) in a cell, operably linked to suitable regulatory regions (e.g. a promoter). A gene may thus comprise several operably linked sequences, such as a promoter, a 5' leader sequence comprising e.g. sequences involved in translation initiation, a (protein) coding region (cDNA or genomic DNA), introns, and a 3'non- translated sequence comprising e.g. transcription termination sites. For bacterial gene expression, several coding sequences may be combined into an operon. Genes and operons and their use for gene expression in different organisms including prokaryotes and eukaryotes are known to the skilled person.

As used herein, the term "promoter" means a transcription promoter, i.e. a DNA sequence that is capable of controlling (initiating) transcription in a cell or organism where the nucleic acid is to be expressed. For expression in prokaryotic cells such as in *Escherichia choli,* promoters generally known from prokaryotic gene expression may be used. Promoters for expression of a nucleic acid in yeast are also generally known. Promoters for expression in plants or plant cells, any promoter of plant origin, but also any promoter of non-plant origin which is capable of directing transcription in a plant cell, i.e., certain promoters of viral or bacterial origin such as the cauliflower mosaic virus 35S promoter (CaMV35S promoter) (Harpster et al. (1988) Mol Gen Genet. 212(1):182-90, the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al. (1996) Plant Cell 8(1):15-30), stem-specific promoters (Keller et al., (1988) EMBO J. 7(12): 3625-3633), leaf specific promoters (Hudspeth et al. (1989) Plant Mol Biol. 12: 579-589), mesophyl-specific promoters (such as the light-inducible Rubisco promoters), root-specific promoters (Keller et al. (1989) Genes Dev. 3: 1639-1646), tuber-specific promoters (Keil et al. (1989) EMBO J. 8(5): 1323-1330), vascular tissue specific promoters (Peleman et al. (1989) Gene 84: 359-369), stamen-selective promoters (WO 89/10396, WO 92/13956), dehiscence zone specific promoters (WO 97/13865) etc. For transient expression in plants cells or plants, constitutive promoters, i.e. promoters that are not developmentally regulated, are preferably used. However, constitutive promoters may be tissue-specific or organ-specific.

The invention further provides a vector or plasmid comprising the nucleic acid of the invention or the recombinant construct. The vector and plasmid may comprise a selectable marker allowing selecting for the presence of said vector or plasmid in eukaryotic or prokaryotic cells. For expression in plants or plant cells, the recombinant construct may be flanked by a T-DNA border sequence on at least one side, which permits the transfer of said recombinant construct into cells of said plant, notably by *Agrobacterium-mediated* transformation.

The invention further provides a prokaryotic or eukaryotic cell, cell line or strain comprising a nucleic acid or recombinant construct according to the invention. The nucleic acid may be introduced into prokaryotic or eukaryotic cells by generally known methods, e.g. by transformation or crossing of cells or organisms containing the nucleic acid. In case of transformation, cells are generally transformed with the vector of plasmid of the invention. The prokaryotic or eukaryotic cell, cell line or strain may comprise a gene encoding a *Z,Z-*farnesyl diphosphate synthase (*Z,Z*-FPP synthase, also referred to herein as "zFPS") and/or a gene encoding a zingiberene synthase (also referred to herein as ShZIS). The ShZIS as well as its use and expression is described in WO 2012/165961 A1.

Prokaryotic cells may be any prokaryotic cells usable for cloning the recombinant construct, plasmid and/or vector, or prokaryotic cells used for expressing the nucleic acid of the invention. An example of a usable prokaryotic cell is *Escherichia coli.* Other examples are *Bacillus subtilis,* photosynthetic cyanobacteria such as *Synechocystis sp.* PCC6803, or bacteria that grow alternative on substrates such as methanol, e.g. *Methylobacterium extorquens, Methylobacillus sp., Methylophyllus sp., Ancylobacter sp., Hyphomicrobium sp., Xanthobacter sp., Thiobacillus sp., Mycobacterium sp., Paracococcus sp., Methylophaga sp. and Acidomonas sp.* Methods of transforming a construct or vector into prokaryotic cells are well-known to the skilled person.

Examples of eukaryotic cells, cell lines or strains are yeast cells, cell lines or strains, such as of genus *Saccharomyces* (e.g. *Saccharomyces cerevisiae)* or *Schizosaccharomyces* (e.g. *Schizosaccharomyces pombe), Yarrowia lipolytica,* or *Pichia pastoris.* Other fungi such as molds, e.g. *Fusarium fujikuroi* could also be used in the invention. Yeast cells may be transformed according to generally known methods, cf. Borts et al., 1996. Methods for nucleic acid expression in *S*. *pombe,* including vectors, promoters, terminators, transformation methods, integration into a target region e.g. using homologous recombination are known in the prior art, e.g. from EP 2 468 860 A1.
Examples of microalgae are microalgae such as *Chlamydomonas reinhardtii,* various species of *Scenedesmus* (e.g. *S*. *obliquus,* S*. vacuolatus, S*. *rubescens), Chlorella vulgaris* or *C*. *kessleri, Haematococcus pluvialis, or Dunaliella salina.* Methods for nucleic acid expression in *C*. *reinhardtii* and other microalgae are known in the art as reviewed by Scaife et al. (2015) or Rasala and Mayfield (2015).

In one embodiment, the eukaryotic cells, cell lines or strains are plant cells, plant cell lines or strain of plant cells, preferably of multicellular plants, more preferably of dicot plants, even more preferably of *Solanum* plants, and even more preferably *Solanum lycopersicum* plants. Further plants usable in the invention are listed below. The invention further provides a plant, or material of a plant, comprising the recombinant construct, or comprising a vector or plasmid according to the invention. The plants may be the same as listed above in this paragraph or those listed below. A material of a plant may be plant parts such as embryos, pollen, ovules, fruit (e.g. harvested tomatoes), flowers, leaves, seeds, roots, and root tips.

The invention provides a process of generating a plant or plant cell, comprising inserting the recombinant construct of the invention or a vector or plasmid containing the recombinant construct into a plant or into a plant cell. The plant or plant cell produced are preferably capable of producing ZG9OH and/or ZG9OH10Epoxy or are capable of producing increased amounts of ZG9OH and/or ZG9OH10Epoxy compared to a plant or call before the inserting step. Plants or cells thereof may be stably transformed or transiently transfected with the nucleic acid, the recombinant construct or vector according to the invention. In stable transformation, a nucleic acid is integrated into a chromosome such as a nuclear or plastid chromosome of a plant cell, such that the nucleic acid can be transferred to progeny cell or progeny plants. Integration into a nuclear chromosome is preferred. Selection methods using a selectable marker gene in the recombinant construct or vector allows selecting transformed plant cells or plants out of the large background of non-transformed cells or plants. From transformed cells or tissue (such as callus tissue), transgenic plants containing the nucleic acid can be regenerated using generally known methods.

Alternatively, in a process of generating a plant or plant cell capable of producing ZG9OH and/or ZG9OH10Epoxy parts of a plant are transiently transfected with said nucleic acid molecule (vector) for transient expression of said nucleotide sequence of interest. Transient transfection has the advantage that a decision on when the nucleotide sequence of interest should be expressed in plants, and which nucleotide sequence of interest to be expressed, can be delayed until shortly before the point of transfection. The term "transient" means that no selection methods are used for selecting cells or plants containing the nucleic acid, recombinant construct or vector or from non-transfected cells or plants using, e.g. a selectable agent and a selectable marker gene capable of detoxifying the selectable agent. As a result, the transfected nucleic acid is generally not stably introduced into plant chromosomal DNA. Instead, transient expression methods make use of the effect of transfection in the very plant cells transfected.

A possible way of achieving expression of the nucleic acid of the invention is the use of self-replicating (viral) replicons containing a nucleotide sequence encoding said nucleic acid of the invention. Plant viral expression systems have been described in many publications both for the use of DNA viral vectors and for the use of RNA viral vectors, such as in WO2008028661, WO2006003018, WO2005071090, WO2005049839, WO2006012906, WO02101006 or WO02068664 and many more publications are cited in these documents.

Various methods for introducing a nucleic acid molecule such as a DNA molecule, into a plant or plant part for transient expression are known. One widely used method is *Agrobacterium*-mediated transfection/transformation for introducing into plants or plant cells a nucleic acid molecule (vector) or recombinant construct e.g. by agroinfiltration (see e.g. Marillonnet et al., Proc. Natl. Acad. Sci. USA 101, 6852-6857; EP2184363 A1). In another embodiment, plants or plant parts are sprayed with a suspension containing cells of an *Agrobacterium* strain, e.g. as described in WO2012/019660. For stable transformation, transient transfection is followed by selection steps using a selectable marker as mentioned above. *Agrobacterium-mediated* gene transfer and vectors therefor are known to the skilled person, e.g. from the references cited above or from text books on plant biotechnology such as Slater, Scott and Fowler, Plant Biotechnology, second edition, Oxford University Press, 2008.

Plants usable for this purpose are not particularly limited. Examples are *Solanum* plants such as *Solanum lycopersicum, Solanum tuberosum,* and *Solanum melongena; Capsicum* plants such as *Capsicum annuum,* and other crop plants of the Solanaceae family; *Helianthus* plants such as *Helianthus annuus* and *Helianthus tuberosus; Zea mays; Oryza sativa; Triticum sp.; Hordeum vulgare; Glycine max,* and *Brassica* plants such as *Brassica rapa.*

The *Agrobacterium* strain may belong to the species *Agrobacterium tumefaciens* or *Agrobacterium rhizogenes* that are commonly used for plant transformation and transfection and which are known to the skilled person from general knowledge. The *Agrobacterium* strain to be used in the processes of the invention may comprises a DNA molecule (Ti-plasmid) comprising a recombinant construct containing a nucleic acid of the invention. The recombinant construct is typically present in T-DNA of a Ti-plasmid for introduction of the recombinant construct into plant cells by the secretory system of the *Agrobacterium* strain. On at least one side or on both sides, the nucleic acid construct is flanked by a T-DNA border sequence for allowing transfection of plant(s) or plant cells and introduction into cells of said plant of said nucleic acid construct. Preferably, said nucleic acid construct is present in T-DNA and flanked on both sides by T-DNA border sequences.

Most preferably, the recombinant construct is present in T-DNA of a Ti-plasmid of the *Agrobacterium* strain. Ti-plasmids may contain a selectable marker outside of said T-DNA for allowing cloning and genetic engineering in bacteria. The T-DNA that is transferred into cells of a plant preferably may or may not contain a selectable marker. If present, the selectable marker may be used for selecting plant tissue or plant cells containing the T-DNA. Examples of selectable marker genes that may or may not be present in T-DNA of the Ti-plasmid are an antibiotic resistance gene or a herbicide resistance gene. In transient transfection and expression of a nucleic acid (or said protein), a step of selecting for plant cells having incorporated the nucleic acid by using such antibiotic resistance gene or a herbicide resistance gene is not necessary. Accordingly, no antibiotic resistance gene or a herbicide resistance gene needs to be incorporated into plants.

*Agrobacterium* strains usable for transfection or transformation of plants are those that are generally used in the art for transfecting or transforming plants. Generally, binary vector systems and binary strains are used, i.e. the *vir* genes required for transfer of T-DNA into plant cells on the one hand and the T-DNA on the other hand are on separate plasmids. Examples of usable *Agrobacterium* strains are given in the article of Hellens et al., Trends in Plant Science 5 (2000) 446-451 on binary *Agrobacterium* strains and vector systems. In the context of a binary *Agrobacterium* strain, the plasmid containing the *vir* genes is referred to as "vir plasmid" or "vir helper plasmid". The plasmid containing the T-DNA to be transfected is the so-called binary vector that is also referred to herein as "vector". The term "strain" or *"Agrobacterium* strain" relates to components of the *Agrobacterium* other than the binary vector. Thus, herein, a binary *Agrobacterium* strain not containing a binary vector and after introduction of a binary vector are referred to by the same strain name.

The process of generating a plant or plant cell capable of producing ZG9OH and/or ZG9OH10Epoxy may further comprise inserting a gene or recombinant construct expressibly encoding a *Z,Z*-FPP synthase and/or a gene or recombinant construct expressibly encoding a zingiberene synthase. The latter two enzymes may be useful in cells or plants that do not naturally produce the substrate 7-epizingiberene of the protein of the invention. Alternatively or additionally, the process may further comprise inserting a gene or recombinant construct encoding a cytochrome P450 reductase, notably from plants, for providing ZPO with reducing equivalents. Vectors or constructs encoding these enzymes may be inserted into the cell simultaneously e.g. by co-transformation or consecutively.

The invention also provides a plant, plant cell, or material of the plant, obtainable according to the process of generating a plant or plant cell. Material of the plant may be as defined above.

The invention further provides a process of generating a plant capable of producing ZG9OH and/or ZG9OH10Epoxy or capable of producing higher amounts of ZG9OH and/or ZG9OH10Epoxy the process comprising crossing a plant (A) not capable of producing ZG9OH and/or ZG9OH10Epoxy or producing insufficient or low amounts of ZG9OH and/or ZG9OH10Epoxy with a plant (B) capable of producing ZG9OH and/or ZG9OH10Epoxy and selecting progeny capable of producing ZG9OH and/or ZG9OH10Epoxy. In one embodiment, the plant (A) may not have a nucleic acid according to the invention encoding a protein capable of producing these compounds, and plant (B) comprises a nucleic acid according to the invention in a (nuclear or plastidic) chromosome; and/or the selection step may comprise testing progeny for the presence of a nucleic acid according to the invention in a chromosome or testing progeny for the expression of mRNA encoded by or being a nucleic acid according to the invention. In one embodiment, the plant (A) is a *Solanum lycopersicum* plant. In another embodiment, plant (B) is a *Solanum habrochaites* plant. In a preferred embodiment, the plant (A) is a *Solanum lycopersicum* plant and plant (B) is a *Solanum habrochaites* plant.

The term "insufficient or low amounts of ZG9OH and/or ZG9OH10Epoxy" is not particularly limited and is considered fulfilled if the amount of ZG9OH and/or ZG9OH10Epoxy produced in a plant can be increased by the process.

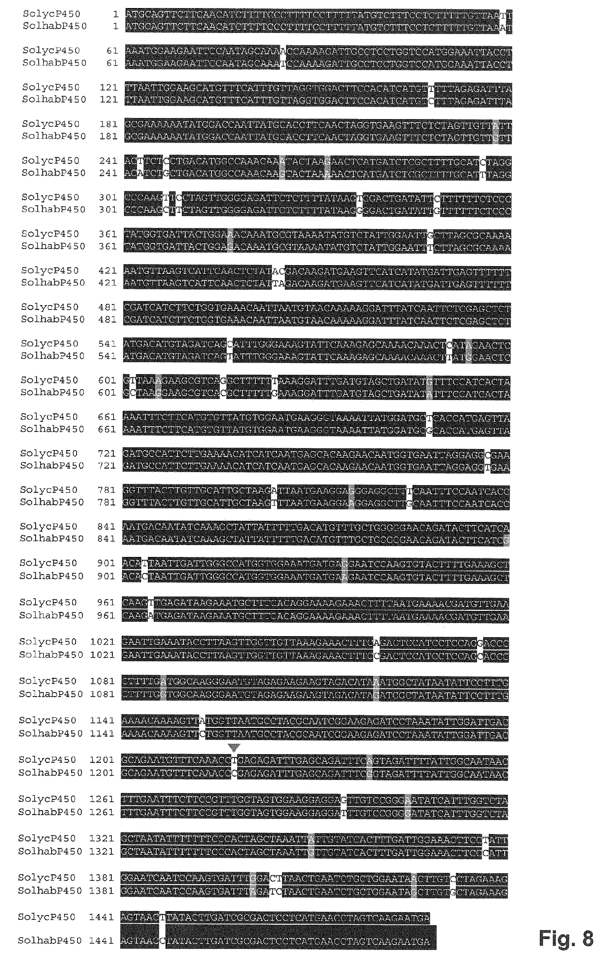

The nucleic acid defined in SEQ ID NO: 1 or SEQ ID NO: 3 or a part thereof may be used for identifying a plant capable of producing ZG9OH and/or ZG9OH10Epoxy e.g. in the above process of generating a plant. As a result, a breeding process may be simplified in that progeny plants from crosses may be genetically analyzed for the presence of the nucleic acid of the invention, as opposed to a more cumbersome or time-consuming phenotypic analysis. Any one or more of the positions where the two nucleotide sequences shown in Fig. 8 deviate may be used as a marker position for identifying whether a nucleic acid of the invention is present in a given plant. The presence of one or more nucleotides characteristic for the SohabP450 sequence in Fig. 8 at such positions indicates that a nucleic acid according to the invention is present in the plant. As a control, the base at the corresponding position in plant (A) above may be determined. Identification of one or more polymorphisms may be done by any known method including sequencing the nucleic acid of the plant. In this way, the nucleic acid of SEQ ID NO: 1 or 3 may be used as a marker for selecting progeny capable of producing ZG9OH and/or ZG9OH10Epoxy. The bases usable are at the following positions in SEQ ID NO: 3: 59, 85, 168, 243, 247, 295, 307, 309, 337, 349, 408, 444, 445, 557, 590, 602, 616, 624, 708, 776, 804, 824, 904, 965, 1060, 1076, 1153, 1218, 1295, 1376, 1404, 1405, 1432, and/or 1447. In a given plant, the presence or absence of a polymorphism may be determined by conventional means such as sequencing of the respective nucleic acids or a part thereof.

Further, the invention provides a method of selecting, from a set of plants, a plant having increased pest resistance compared to another plant or other plants in said set, comprising:
(a) analyzing a plurality of plants in said set of plants for a nucleic acid of the invention, preferably a nucleic acid of claim 2, and selecting a plant or plants having the nucleic acid; or
(b) analyzing a plurality of plants in said set of plants for ZG9OH and/or ZG9OH10Epoxy and selecting a plant or plants having higher content or production of ZG9OH and/or ZG9OH10Epoxy than one or more other plants in said set, or
(c) analyzing a plurality of plants in said set of plants for one or more polymorphisms at the positions listed above in a nucleic acid of SEQ ID NO: 3, and selecting a plant or plants having the polymorphism of SEQ ID NO: 3.
Also this method can support a breeding process wherein a plant capable of producing ZG9OH and/or ZG9OH10Epoxy is generated. Option (a) above generally comprises isolating genomic DNA from the plurality of plants, and determining the presence of a nucleic acid of the invention, notably a nucleic acid according to claim 2. In step (b), ZG9OH and/or ZG9OH10Epoxy may be determined by leaf surface extraction as mentioned above and comparing the amounts of ZG9OH and/or ZG9OH10Epoxy among the plurality of plants. The amounts of ZG9OH and/or ZG9OH10Epoxy in an extract can be determined using GC-MS as described in the examples.

As a further analytical method, the capability of a plant for producing ZG9OH and/or ZG9OH10Epoxy may be determined, e.g. by any one of the following methods:
(a) obtaining a sample from said plant and analyzing said sample for the presence or absence of a nucleic acid of the invention, preferably of that defined in claim 2; or
(b) obtaining a sample from said plant and analysing said sample for the presence or absence, or the amount of, expression of an mRNA encoded by a nucleic acid as defined in claim 1, 2 or 3; and/or
(c) obtaining a sample from said plant and analyzing said sample for its content of ZG9OH and/or ZG9OH10Epoxy and/or C9OH and/or C9OH10Epoxy, preferably by analyzing said sample for its content of ZG9OH and/or ZG9OH10Epoxy; the sample may be one or more leaves.
The nucleic acid of item (a) may be genomic DNA (gDNA), and said sample may be a sample containing gDNA.

The prokaryotic or eukaryotic cell, cell line or strain of the invention may be used in a process of producing ZG9OH and/or ZG9OH10Epoxy. In one embodiment, ZG9OH and/or ZG9OH10Epoxy may be produced in prokaryotic or eukaryotic cells described above and isolated therefrom. Isolation of these compounds may be made by homogenizing cells for example in the presence of an aqueous buffer, and extracting the obtained homogenized product with a solvent with low solubility in water (apolar solvent). Examples of suitable solvents are diethyl ether and hydrocarbon solvents having from 5 to 12 carbon atoms. Saturated hydrocarbon are preferred. Examples of saturated hydrocarbons as solvents are pentane, hexane, heptane, octane, nonane and decane or mixtures of two or more thereof. Alternatively or additionally, culture supernatant may be extracted with the before-mentioned solvents.

Accordingly, the invention provides a novel zingiberene derivative selected from ZG9OH and ZG9OH10Epoxy. Preferably, the novel compounds of the invention may be produced in plants containing a nucleic acid of the invention. The novel compounds may be extracted by leaf surface extraction using apolar solvents as listed above. If desired, ZG9OH and/or ZG9OH10Epoxy may be separated and purified by e.g. distillation. C9OH and/or C9OH10Epoxy may be produced using the same method. C9OH and/or C9OH10Epoxy may analogously be isolated from extracts and, if desired, be isolated e.g. by distillation. Thus, the invention also provides C9OH and/or C9OH10Epoxy.

The invention further provides a protein encoded by the nucleic acid of the invention. The protein is a cytochrome P450 oxygenase. It has 7-epizingiberene or curcumene, and oxygen as substrates. Reducing equivalents may be provided by cytochrome P450 reductase, as conventionally known by the skilled person for cytochrome P450 oxygenases. The protein may be expressed from the nucleic acid of the invention in prokaryotic or eukaryotic cells or in plants. For simplifying purification of the protein, the protein may be expressed with a tag for affinity purification fused to the protein. In this way, conventional affinity purification may be used to purify the protein. If it is desired to obtain the protein in water soluble form, it is preferred to delete the membrane anchor that is generally present on cytochrome P450 proteins before expression. Expression in yeast (such as *S*. *cerevisiae*) can be achieved with the full length protein and carried out as described in Urban et al. (1997). For expression in bacteria, replacement of the N-terminal membrane anchor (such as 5 to 35 N-terminal amino acids) by a sequence facilitating expression such as described in Swaminathan et al. (2009) or in Scott et al. (2001) is preferable. Alternatively, the full length protein can also be expressed in insect cells, for example as described in Jennewein et al. (2001).

The invention also provides a use of the protein of the invention for producing ZG9OH and/or ZG9OH10Epoxy and/or C9OH, and/or C9OH10Epoxy. Moreover, the invention provides a process of producing ZG9OH, and/or ZG9OH10Epoxy and/or C9OH and/or C9OH10Epoxy, comprising incubating 7-epizingiberene in the presence of the protein of the invention, or cells or tissue having expressed the protein, and, as the case requires, a system capable of providing reducing equivalents to the protein. The process may be conducted *in vitro* in a liquid medium. The liquid medium may be an aqueous medium that may contain a buffer. Methods of conducting chemical transformations using cytochrome P-450-oxygenase enzymes are known in the art, as well as ways of providing reducing equivalents via a cytochrome P-450 reductase. Such methods are known and reviewed in Urlacher et al. (2004) or in Kumar (2010). The reaction may be carried out in a buffered aqueous medium. The pH may be between 6 and 8, preferably between 6.5 and 7.5. The liquid medium may contain an additive such as dimethylsulfoxide (DMSO) or nonionic detergents such as Tween 80^{®}, Silwet 77^{®}, or Brij-35^{®} that help to solubilize the substrate and/or products. The main substrate of the protein is 7-epizingiberene that may be provided according to WO 2012/165961. Another substrate is ar-curcumene. The substrate(s) may be added to the reaction medium. Alternatively, the substrate may be produced *in situ* in a coupled enzymatic reaction. For example, the substrate may be produced by including ShZIS (zingiberene synthase) and its substrate *Z,Z*-FPP into the solution. Alternatively, cells or tissue having expressed these proteins may be included in a reaction mixture. The desired products may be isolated from the medium following the reaction e.g. by extraction with an apolar solvent as given above, or by distillation.

Additionally, the invention provides a pesticidal composition comprising a zingiberene derivative selected from ZG9OH and/or ZG9OH10Epoxy. The composition may be a liquid composition containing ZG9OH and/or ZG9OH10Epoxy a liquid carrier medium and optionally suitable additives. The liquid carrier medium may be aqueous. Suitable additives may be compounds that help to solubilize the compound(s) of the invention in the medium, such as surfactants. The pesticidal composition preferably contains an agricultural spray adjuvant. The spray adjuvant may be a surfactant. The surfactant has multiple advantages in the present invention. It reduces the surface tension of water of the aqueous suspension and makes the waxy surface of plant leaves more permeable for the active ingredients (ZG9OH and/or ZG9OH10Epoxy) Surfactants used in the present invention are not particularly limited, and examples of the surfactants include the following (A), (B), and (C). These may be used singly or in combination.
(A) Nonionic surfactants: A measurement frequently used to describe surfactants is the HLB (hydrophilic/lipophilic balance). The HLB describes the ability of the surfactant to associate with hydrophilic and lipophilic compounds. Surfactants with a high HLB balance associate better with water soluble compounds than with oil soluble compounds. Herein, the HLB value should be 12 or greater, preferably at least 13. As nonionic surfactants, organo-silicone surfactants such as polyalkyleneoxide-modified heptamethyltrisiloxane are most preferred in the present invention. A commercial product is Silwet L77™ spray adjuvant from GE Advanced Materials.
   (A-1) Polyethylene glycol type surfactants: examples of polyethylene glycol type surfactants include polyoxyethylene alkyl (C12-18) ether, ethylene oxide adduct of alkylnaphthol, polyoxyethylene (mono or di) alkyl (C8-12) phenyl ether, formaldehyde condensation product of polyoxyethylene (mono or di) alkyl (C8-12) phenyl ether, polyoxyethylene (mono, di, or tri) phenyl phenyl ether, polyoxyethylene (mono, di, or tri) benzyl phenyl ether, polyoxypropylene (mono, di, or tri) benzyl phenyl ether, polyoxyethylene (mono, di, or tri) styryl phenyl ether, polyoxypropylene (mono, di or tri) styryl phenyl ether, a polymer of polyoxyethylene (mono, di, or tri) styryl phenyl ether, a polyoxyethylene polyoxypropylene block polymer, an alkyl (C12-18) polyoxyethylene polyoxypropylene block polymer ether, an alkyl (C8-12) phenyl polyoxyethylene polyoxypropylene block polymer ether, polyoxyethylene bisphenyl ether, polyoxyethylene resin acid ester, polyoxyethylene fatty acid (C12-18) monoester, polyoxyethylene fatty acid (C12-18) diester, polyoxyethylene sorbitan fatty acid (C12-18) ester, ethylene oxide adduct of glycerol fatty acid ester, ethylene oxide adduct of castor oil, ethylene oxide adduct of hardened caster oil, ethylene oxide adduct of alkyl (C12-8) amine and ethylene oxide adduct of fatty acid (C12-18) amide;
   (A-2) Polyvalent alcohol type surfactants: examples of polyvalent alcohol type surfactants include glycerol fatty acid ester, polyglycerin fatty acid ester, pentaerythritol fatty acid ester, sorbitol fatty acid (C12-18) ester, sorbitan fatty acid (C12-8) ester, sucrose fatty acid ester, polyvalent alcohol alkyl ether, and fatty acid alkanol amide;
   (A-3) Acetylene-type surfactants: examples of acetylene type surfactants include acetylene glycol, acetylene alcohol, ethylene oxide adduct of acetylene glycol and ethylene oxide adduct of acetylene alcohol.
(B) Anionic surfactants:
   (B-1) Carboxylic acid type surfactants: examples of carboxylic acid type surfactants include polyacrylic acid, polymethacrylic acid, polymaleic acid, a copolymer of maleic acid and olefin (for example, isobutylene and diisobutylene), a copolymer of acrylic acid and itaconic acid, a copolymer of methacrylic acid and itaconic acid, a copolymer of maleic acid and styrene, a copolymer of acrylic acid and methacrylic acid, a copolymer of acrylic acid and methyl acrylate, a copolymer of acrylic acid and vinyl acetate, a copolymer of acrylic acid and maleic acid, N-methyl-fatty acid (C12-18) sarcosinate, carboxylic acids such as resin acid and fatty acid (C12-18) and the like, and salts of these carboxylic acids.
   (B-2) Sulfate ester type surfactants: examples sulfate ester type surfactants include alkyl (C12-18) sulfate ester, polyoxyethylene alkyl (C12-18) ether sulfate ester, polyoxyethylene (mono or di) alkyl (C8-12) phenyl ether sulfate ester, sulfate ester of a polyoxyethylene (mono or di) alkyl (C8-12) phenyl ether polymer, polyoxyethylene (mono, di, or tri) phenyl phenyl ether sulfate ester, polyoxyethylene (mono, di, or tri) benzyl phenyl ether sulfate ester, polyoxyethylene (mono, di, or tri) styryl phenyl ether sulfate ester, sulfate ester of a polyoxyethylene (mono, di, or tri) styryl phenyl ether polymer, sulfate ester of a polyoxyethylene polyoxypropylene block polymer, sulfated oil, sulfated fatty acid ester, sulfated fatty acid, sulfate ester of sulfated olefin and the like, and salts of these sulfate esters.
   (B-3) Sulfonic acid type surfactants: examples of sulfonic acid type surfactants include paraffin (C12-22) sulfonic acid, alkyl (C8-12) benzene sulfonic acid, formaldehyde condensation product of alkyl (C8 - 12) benzene sulfonic acid, formaldehyde condensation product of cresol sulfonic acid, -olefin (C14-16) sulfonic acid, dialkyl (C8-12) sulfosuccinic acid, lignin sulfonic acid, polyoxyethylene (mono or di) alkyl (C8-12) phenyl ether sulfonic acid, polyoxyethylene alkyl (C12-18) ether sulfosuccinate half ester, naphthalene sulfonic acid, (mono, or di) alkyl (C1-6) naphthalene sulfonic acid, formaldehyde condensation product of naphthalene sulfonic acid, formaldehyde condensation product of (mono, or di) alkyl (C1-6) naphthalene sulfonic acid, formaldehyde condensation product of creosote oil sulfonic acid, alkyl (C8-12) diphenyl ether disulfonic acid, Igepon T (tradename), polystyrene sulfonic acid, sulfonic acids of a styrene sulfonic acid - methacrylic acid copolymer and the like, and salts of these sulfonic acids.
   (B-4) Phosphate ester type surfactants: examples of phosphate ester type surfactants include alkyl (C8-12) phosphate ester, polyoxyethylene alkyl (C12-18) ether phosphate ester, polyoxyethylene (mono or di) alkyl (C8-12) phenyl ether phosphate ester, phosphate ester of a polyoxyethylene (mono, di, or tri) alkyl (C8-12) phenyl ether polymer, polyoxyethylene (mono, di, or tri) phenyl phenyl ether phosphate ester, polyoxyethylene (mono, di, or tri) benzyl phenyl ether phosphate ester, polyoxyethylene (mono, di, or tri) styryl phenyl ether phosphate ester, phosphate ester of a polyoxyethylene (mono, di, or tri) styryl phenyl ether polymer, phosphate ester of a polyoxyethylene polyoxypropylene block polymer, phosphatidyl choline, phosphate ester of phosphatidyl ethanolimine and condensed phosphoric acid (for example, such as tripolyphosphoric acid) and the like, and salts of these phosphate esters.
   Salts of above-mentioned (B-1) to (B-4) include alkaline metals (such as lithium, sodium and potassium), alkaline earth metals (such as calcium and magnesium), ammonium and various types of amines (such as alkyl amines, cycloalkyl amines and alkanol amines).
(C) Amphoteric surfactants: Examples of amphoteric surfactants include betaine type surfactants and amino acid type surfactants.

The total concentration of surfactants in the aqueous suspension of the invention may be easily tested by conducting comparative spraying experiments on plants infested with an insect pest. However, in general, the total concentration of surfactants may be between 0.005 and 2 volume-%, preferably between 0.01 and 0.5 volume-%, more preferably between 0.025 and 0.2 volume-% of said suspension. Since the density of surfactants is generally close to 1.0 g/ml, the total concentration of surfactants may be defined as being between 0.05 and 20 g per liter of said suspension, preferably between 0.1 and 5.0 g, more preferably between 0.25 and 2.0 g per liter of said suspension (including abrasive). If the above organo-silicone surfactants such as polyalkyleneoxide-modified heptamethyltrisiloxane are used, the concentration of the organo-silicone surfactant in the agrobacterial suspension used for spraying may be between 0.01 and 0.5 volume-%, preferably between 0.05 and 0.2 volume-%. Alternatively, the concentration of the organo-silicone surfactant in the agrobacterial suspension used for spraying may be defined as being between 0.1 and 5.0 g, preferably between 0.5 and 2.0 g per liter of said suspension.

Additional additives that may be used as the case requires, there may be mentioned alcohols such as methyl alcohol or ethylene glycol; ketones such as acetone, methyl ethyl ketone or N-methyl-2 -pyrrolidone; ethers such as dioxane or tetrahydrofuran; aliphatic hydrocarbons such as kerosine, gas oil or the like; aromatic hydrocarbons such as xylene, trimethylbenzene, tetramethylbenzene, cyclohexane or solvent naphtha; halogenated hydrocarbons such as chloroform or chlorobenzene; acid amides such as dimethylformamide; esters such as ethyl acetate or glycerine ester of a fatty acid; nitriles such as acetonitrile; sulfur-containing compounds such as dimethyl sulfoxide; and vegetable oils such as soybean oil or corn oil.

The concentration of ZG9OH and/or ZG9OH10Epoxy in the pesticidal composition may be from 10 ppm to 100 000 ppm, preferably 50 to 10 000 ppm, more preferably, 500 to 5 000 ppm by weight.

The invention further provides a use of the compound ZG9OH and/or ZG9OH10Epoxy for increasing pest resistance of a plant, and a method of increasing pest resistance of a plant, comprising spraying said plant or the vicinity where the plant grows with a composition comprising ZG9OH and/or ZG9OH10Epoxy. The compounds of the invention can act as a repellent of the pest.

The pest may be an insect pest notably insect pests of plans. Examples of pests are the two-spotted spider mite (Tetranychus urticae), carmine spider mite (Tetranychus cinnabarinus), kanzawa spider mite (Tetranychus kanzawai), citrus red mite (Panonychus citri), European red mite (Panonychus ulmi), broad mite (Polyphagotarsonemus latus), pink citrus rust mite (Aculops pelekassi) and bulb mite (Rhizoglyphus echinopus); agricultural insect pests such as diamondback moth (Plutella xylostella), cabbage armyworm (Mamestra brassicae), common cutworm (Spodoptera litura), codling moth (Laspeyresia pomonella), bollworm (Heliothis zea), tobacco budworm (Heliothis virescens), gypsy moth (Lymantria dispar), rice leafroller (Cnaphalocrocis medinalis), smaller tea tortrix (Adoxophyes sp.), summer fruit tortrix (Adoxophyes orana fasciata), peach fruit moth (Carposina niponensis), oriental fruit moth (Grapholita molesta), black cutworm (Agrotis ipsilon), cutworm (Agrotis segetum), Colorado potato beetle (Leptinotarsa decemlineata), cucurbit leaf beetle (Aulacophora femoralis), boll weevil (Anthonomus grandis), the greenhouse whitefly (*Trialeurodes vaporariorum*), the silverleaf whitefly (Bemisia tabacii), or the cabbage whitefly (*Aleyrodes proletella*), the tomato leaf miner (*Tuta absoluta*), aphids, planthoppers, leafhoppers, scales, bugs, whiteflies, thrips, grasshoppers, anthomyiid flies, scarabs, ants, or leafminer flies. Other pests are those given in WO 2012/165961 such as the sap-sucking insect pests given therein.

Further, the compound(s) of the invention may be mixed with or may be used in combination with other agricultural chemicals, fertilizers or phytotoxicity-reducing agents, whereby synergistic effects or activities may sometimes be obtained. Such other agricultural chemicals include, for example, a herbicide, an antivirus agent, an attractant, a plant hormone and a plant growth regulating agent.

As the pesticidal composition is liquid, it is preferably applied to plants or to an area where they grow by spraying. The sprayer to be used mainly depends on the number of plants or the area to be sprayed. For one or a small number of plants to be sprayed, pump sprayers as widely used in household and gardening can be used. These may have volumes of the spray tank of between 0.5 and 2 liters. For applications on a medium scale, manually operated hydraulic sprayers such as lever-operated knapsack sprayers or manually operated compression sprayers may be used. On the large scale such as on a farm field, power-operated hydraulic sprayers such as tractor-mounted hydraulic sprayers equipped with spray booms can be used. Aerial application techniques using helicopters or airplanes are also possible for large fields. All these types of sprayers are known in the art and are described for example in the book "Pesticide Application Methods" by G.A. Matthews, third edition, Blackwell Science, 2000.

In the method of increasing pest resistance of the invention, at least parts of plants such as leaves are sprayed. Preferably, most leaves are sprayed or entire plants. Such spraying is applicable to a wide range of species, including tomato (*S. lycopersicum*), potato (*Solanum tuberosum*), Aubergine *(Solanum melongena),* Sweet pepper (*Capsicum annuum),* and other crops of the Solanaceae family, sunflower (*Helianthus annuus*), Jerusalem artichoke (*Helianthus tuberosus*), corn (*Zea mays*), rice (*Oryza sativa*), wheat (*Triticum sp*.), barley (*Hordeum vulgare*), soja (*Glycine max*), various crops of the crucifer family such as *Brassica rapa, Brassica oleracea, Brassica nigra,* etc. *Solanum lycopersicum* is a preferred plant.

### EXAMPLES

### Materials and methods

### Bacteria

For transformation and propagation of plasmid DNA, the chemocompetent *Escherichia coli* strains NovaBlue, DH10B respectively XL-1 were used and for transient gene expression the electrocompetent *Agrobacterium tumefaciens* strain GV3101::pmP90.

### Electroporation

Electrocompetent bacteria like the *A. tumefaciens* strain GV3101::pmP90 were thawed on ice for 5 min. For one single transformation, a 50 µL aliquot of bacterial suspension was mixed in an Eppendorff tube with 2-3 µL ligation product (10 ng/µL) and transferred in a pre-cooled electroporation cuvette from VWR (Leuven, Belgium). After the electroshock (U = 2.5 kV; C = 25 µF; R < 200 Ω; t(impulse) = 5 ms), 950 µL of SOC-medium was added. This suspension was then transferred back into an Eppendorff tube and incubated in a thermomixer at 30°C for 2 h. At the end, 50 µL were plated on an LB agar plate with the appropriate antibiotics for selection of the transformed plasmid and grown at 37°C 2-3 days.

### Yeast

For transformation of plasmid DNA and gene expression in yeast, the chemocompetent *Saccharomyces cerevisiae* INVSc1 strain from Invitrogen (Carlsbad, USA) was used.

### Chemical Transformation

An aliquot of one-strand of salmon sperm DNA (D9156) and S. *cerevisiae* strain InvSC1 were thawn on ice for 5 min and mixed with 100 ng yeast plasmid. After addition of 600 µL plate buffer the transformation solution was vortexed and incubated at 30°C 30 min. Then 10 µL dimethylsulfoxid (DMSO) was added and heat-shocked for 15 min at 42°C. At the end the cells were centrifuged, the upper phase removed, the pellet re-solved in 500 µL ddH₂O and 100 µL transferred on synthetic Drop-out media agar plates for incubation at 30°C for 2-3 d.

### Oligonucleotides

All oligonucleotide primer pairs used for amplification of DNA fragments, Colony-PCR, qRT-PCR experiments and sequencing were designed using the software Geneious or Primer3 and ordered from Eurofins MWG Operon (Ebersberg, Germany) HPLC purified. The following oligonucleotides were used in this work:

**Table 1: List of oligonucleotides**

| **Use** | **Name** | **sequence (5' - 3')** | **SEQ ID NO:** |
|---|---|---|---|
| Cloning | P450iso008670-F | TTTGAAGACAAAATGCAGTTCTTCAACATCTTTT CC | 6 |
| Cloning | P450iso008670-R | TTTGAAGACAAAAGCTCATTCTTGACTAGGTTCA TGAGG | 7 |
| Sequencing | P450-Fq Forward | CATGTGTTATGTGGAATGAAGGG | 8 |
| Sequencing | P450-Rq Reverse | TCCTAATTCACCATTGTTCTTGTG | 9 |
| HRM marker | P450HRMC_For | ATGGCTATAATATTCCTTTGAAAACAAAAG | 10 |
| HRM marker | P450HRMC_Rev | GTTTGAAACATTCTGCGTCAATCC | 11 |

### Polymerase-chain reaction

For DNA amplification from gDNA respectively cDNA or plasmid DNA for later cloning procedures, different polymerase-chain reaction (PCR) methods were used. KOD Hot Start DNA Polymerase (KOD), Phusion high fidelity DNA polymerase (Phusion) and the DreamTaq DNA-Polymerase (DreamTaq) were used following manufacturer's recommendations. For the PCR reaction the following temperature program was applied including an initial denaturation step (thermal separation of the DNA double helix) of 2 min at 95 °C and then 30-35 cycles of 30 s denaturation at 95°C, a 30 s annealing step (hybridization of the primer with the single stranded DNA) at 55°C and elongation (DNA strand synthesis) for 0.5 min/kb (KOD) or 1 min/kb (DreamTaq) gene length at 72°C with 5 min final extension step at 72°C. Oligonucleotide primer pairs used for PCR are listed in Table 1.

### qRT-PCR - expression analysis + HRM mapping

qRT-PCR was done with a Bio-Rad Connect 96x Real-Time PCR system and the my-Budget 5x EvaGreen QPCR Mix II from Bio&Sell. Input of each reaction was 2-3 µL diluted cDNA (1ng/µL). The qRT-PCR reaction had the following program applied including an initial denaturation step of 15 min at 95°C and then 2 steps á 40 cycles of 10 s denaturation at 95°C and a 20 s annealing + elongation step at 55°C. The melt curve was recorded in parallel to additional denaturing at 95°C for 30 s, cooling down to 65°C for 30s and heating up again to 95°C 0.1°C/s. The analysis was done with the Bio-Rad CFX manager software. In case of doing a HRM mapping, the temperature program was adjusted to denaturation at 95°C for 15 min followed by 3 steps at 40 cycles 10s denaturation at 95°C, annealing for 20s at 60°C and elongation at 72°C for 30s. In this case extended melt curve was obtained by heating up 95°C for 1 min, cooling down to 60 °C h 1 min hold with followed heating from 70 to 90°C 0.01°C/s. The data was analyzed with the Bio-Rad Precision Melt Analysis program.

### Electrophoretic DNA separation

Amplified cDNA respectively plasmid-DNA were separated after addition of 1/6 volume of DNA loading dye [10mM Tris/HCl (pH 7.6), 0.03% bromphenolblue (w/v), 0.03% xylencyanol-FF (w/v), 60% glycerol (w/v) and 60 mM EDTA] with 1x TAE buffer [40 mM Tris/HCl (pH 7.0), 20 mM HOAc, 1 mM EDTA (pH 8.0)] containing 1% agarose (w/v) using Gene Ruler 1 kb/1kB+ DNA Ladder as a size marker and ethidium bromide for later visualization using the UV-imager Fusion FX7 from Fluke (Glottertal, Germany). For purification of PCR amplified DNA fragments the MiniElute PCR Purification Kit and for purification of digested plasmid-DNA from agarose gel pieces the MiniElute Gel Extraction Kit (both Qiagen) were used following manufacturer's recommendations. The purified DNA was stored at -20°C.

### Preparation of constructs

For cloning of DNA vector constructs, around 40 fmol PCR amplified DNA fragments and plasmids with Bpi I/Bsa I restriction sites were ligated by incubating a mixture of both for 2-3 h at 37°C with a T4 DNA ligase. An optional incubation step of 5 min at 50°C can be applied. The reaction was stopped by heating the mixture to 80°C for 5 min. By combination of more than 4-5 fragments and plasmids the temperature program was changed to 37°C 4 min + 20°C 2 min for 25-50 cycles followed by 37°C, 50°C and 80°C for 5 min each. The restriction enzymes were either BpiI or Bsal as part of the Golden Gate cloning system (Engler et al., 2014). Further subcloning of PCR fragments generated by the KOD or Phusion polymerase without 3'-dA overhangs into a blunt end vector-system like pUC18 ligation was done for 2-3 h at 20°C with Smal and the T4 DNA Ligase. If using a pGEM-Teasy vector an additional incubation step was performed. The A-Tailing of the PCR fragment was done by incubation of the PCR fragments together with the DreamTaq polymerase 15-30 min at 70°C. Further ligation was done according to manufacturer's recommendations.

### Isolation of plasmid DNA

For plasmid isolation from bacterial or yeast cultures the QIAprep Spin MiniPrep (Qiagen) or NucleoSpin Plasmid Easy Pure Kit (Machery-Nagel) was used following the manufacturer's recommendations.

### Sequencing

The sequencing of purified DNA fragments from PCR reaction or vector constructs were done by Eurofins MWG Operon by mixing up of either 15 µL of 50-100 ng/µL plasmid DNA or 2-10 ng/µL PCR fragment solved in ddH₂O with 2 µL oligonucleotide (10 uM).

### Yeast Expression

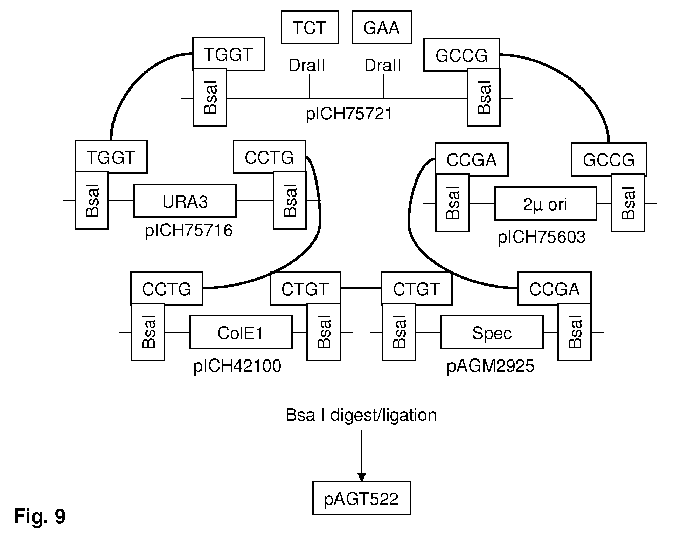

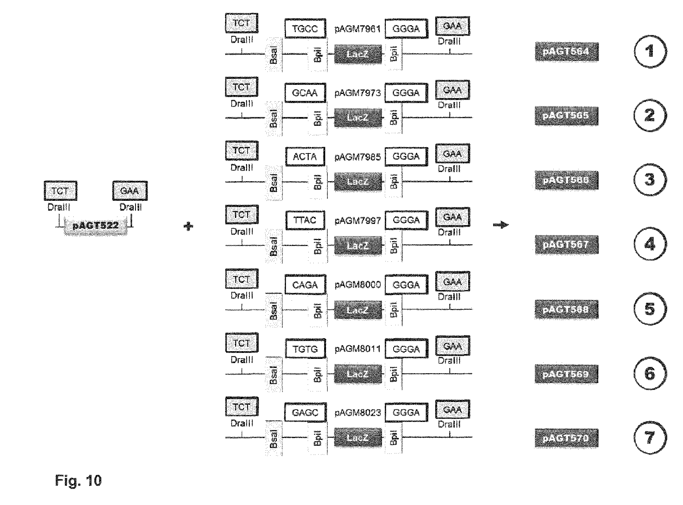

Yeast peptone dextrose medium (YPD-medium) [2% peptone (w/v), 1% yeast extract (w/v)] was used for propagation of yeast like *S*. *cerevisiae* strain INVSc1. The YPD-medium was prepared like the bacterial medium but without any selective antibiotics. Addition of sterile filtrated glucose for cultivation or galactose to induce gene-expression was done shortly before usage up to a final concentration of 2% (w/v). Yeast cultures were incubated under constant shaking at 250 rpm at 30°C 16-24 h. Freshly transformed cells were grown on Drop-out medium agar [0.01% synthetic drop-out medium without uracil (w/v), 0.67% yeast basic medium with amino acids (w/v) and 2% micro agar (w/v)] at 30°C for 4-5 days. The cloning system is described in WO 2011/154147. Yeast expression vectors were developed that are compatible with this cloning system. The level 1 vectors are as described in WO 2011/154147 and in Weber et al. (2011). New level M vectors for replication and selection in yeast were made as described in Werner et al. (2012) except that they contain a selection marker (the URA3 gene) and a replication origin for yeast (2 µ). An overview of the assembly of these plasmids is shown in Figures 9 and 10. Here, only the level M vector starting at position 1 (pAGT564 in Figure 10) was used. The sequences of plasmids required to build these level M vectors are provided in the appended sequence listing.

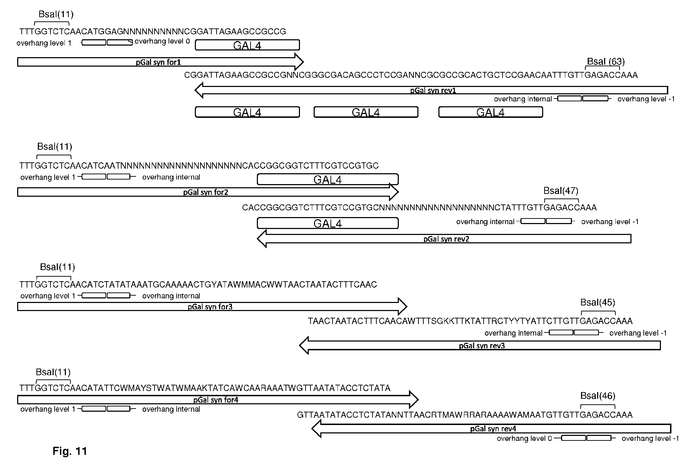

For the expression in yeast a library of synthetic galactose inducible promoters were made based on the design shown in Figure 11. Sequences of primers used for the cloning of synthetic galactose inducible promoters were as follows:
SEQ ID NO: 12: pGal_syn_for1 5'TTTGGTCTCAACATGGAGNNNNNNNNNNCGGATTAGAAGCCGCCG
SEQ ID NO: 13: pGal_syn_rev1 
5'TTTGGTCTCAACAAATTGTTCGGAGCAGTGCGGCGCGNNTCGGAGGGCTGTCGCCC
GNNCGGCGGCTTCTAATCCG
SEQ ID NO: 14: pGal_syn_for2 
5'- TTTGGTCTCAACATCAATNNNNNNNNNNNNNNNNNNNNCACCGGCGGTCTTTCGT
CCGTGC
SEQ ID NO: 15: pGal_syn_rev2 
5'-TTTGGTCTCAACAAATAGNNNNNNNNNNNNNNNNNNNNGCACGGACGAAAGACCGC
CGGTG
SEQ ID NO: 16: pGal_syn_for3 
5'-TTGGTCTCAACATCTATATAAATGCAAAAACTGYATAWMMACWWTAACTAATACTTT
CAAC
SEQ ID NO: 17: pGal_syn_rev3 5'TTTGGTCTCAACAAGAATRARRAGYAATACAAMMCSAAAWTGTTGAAAGTATTAGTTA
SEQ ID NO: 18: pGal_syn_for4 
5'TTTGGTCTCAACATATTCWMAYSTWATWMAAKTATCAWCAARAAATWGTTAATATAC
CTCTATA
SEQ ID NO: 19: pGal_syn_rev4 5' TTTGGTCTCAACAACATTKTWTTTTYTYYWTKAYGTTAANNTATAGAGGTATATTAAC

These promoters were then tested with a reporter gene (eYFP) and the strongest were used for expression of transgenes in yeast. Three of them were used: pGal_Syn1_3 (pAGT864), pGal-Syn1_4 (pAGT865) and pGal_Syn1_5 (pAGT866). In addition the promoter of the GAL7 gene was used to drive the expression of the *Arabidopsis thaliana* P450 reductase gene. The sequence of the plasmids containing these inserts is also provided in appended sequence listing.

Various yeast transcription terminators were also cloned in level 0 vectors, including tTEF1 (pAGT537), tSAG1 (pAGT538), tADH1 (pAGT539) and tIDP1 (pAGT540). The sequence of these plasmids is provided in appended sequence listing.

### Transient Expression in Nicotiana benthamiana

To express proteins in *N. benthamiana,* the full length sequence of the already described zFPS (FJ194969.1) and ZIS (JN990661.1) together with the cDNA from Solhab01g008670.2.1 from *Solanum habrochaites* accession LA2167 were cloned into the binary T-DNA vector pL1F-1 under fusion to a CaMV35S promotor and NosT terminator by using a single restriction-ligation reaction using 10 units Bsal and 10 units T4-HC-Ligase The *A. tumefaciens* strain GV3101::pMP90 was then transformed with the recombinant plasmids by electroporation. Afterwards, the cells were grown on LB medium agar plates (Duchefa - Haarlem, Netherlands) supplemented with the antibiotics rifampicillin (50 µg/mL), gentamycin (50 µg/mL) and carbenicillin (50 µg/mL) dilution 1:1000 (v/v) for 48 h at 28°C. After selection on solid LB medium agar plates single colonies of *A. tumefaciens* cells were grown in a 5 mL LB medium pre-culture for 24 h at 28°C under constant shaking with 180 rpm. The pre-culture was then used for inoculation of the 50 mL LB medium main culture 1:100 with the same antibiotics dilution 1:1000 (v/v) with continued growth for 16 h at 28°C at 180 rpm. The cell density was then determined at OD with a Beckmann Coulter DU800 photometer (Krefeld, Germany). The final OD was adjusted to 0.6 in 20 mL infiltration medium. The *A. tumefaciens* cells were then mixed up in different combinations of expression constructs and harvested by centrifugation for 30 min at 1800 x g at 4°C. Resuspension was done in 0.25 volumes per LB broth and sterile ddH₂O together with 0.5 volumes infiltration buffer containing 20 mM glucose (Novagen, Darmstadt, Germany), 10% sucrose (w/v) and 8.6 g/L Murashige and Skoog basal salt mixture. All recombinant constructs were co-infiltrated in different combinations with *A*. *tumefaciens* harboring the p19 T-DNA vector, a viral-encoded gene-silencing suppressor. 20 µM acetosyringone were added before infiltration of the constructs into leaves of 4-5 week old *N. benthamiana* plants. Infiltration was done by pressing a 1 mL syringe onto abaxial side of the leaves followed by slow infiltration of *A*. *tumefaciens* in the leaf. After complete infiltration of 3-4 leaves the plants were brought back into growth chambers for an additional 5 days before n-hexane extraction and GC-MS measurement analysis.

### Establishing a backcross population

Pollen from the wild tomato species (*S*. *habrochaites* LA2167) was collected on a glass slide. Flowers from the cultivated tomato *S*. *lycopersicum* accession LA4024 were emasculated and manually pollinated with the harvested pollen of LA2167. This procedure was repeated several times with each of a half a dozen flowers in the next 7 days to ensure successful pollination. After fruit development all seeds were harvested and sowed after 1 week of drying at room temperature. The upcoming F1 plants were then analyzed by GC-MS analysis and HRM mapping for at least 6 independent loci. Upon flowering, pollen of the F1 plants were collected and transferred again on to the stigma of the cultivated tomato (LA4024). This procedure was carried out as described above. Seeds were harvested and each line of the F2 back-cross generation was then individually characterized, genotyped and phenotyped following the same procedure as for the F1 hybrid plants.

### GC-MS analysis

To measure the metabolite profile of leaf extracts done with n-hexane, 1 µL sample volume was injected directly into the GC-MS, a coupled Trace GC ultra-gas chromatograph with EI ionization coupled to an ISQ mass spectrometer from Thermo Fisher Scientific. The exudates were separated by a 30-m x 0.32 mm diameter capillary, with 0.25 µm film of ZS-5 ms from Phenomenex (Aschaffenburg, Germany). Splitless mode was applied to inject with an inlet temperature of 250°C. The GC oven was programmed to hold a start temperature of 50°C for 1 min before increasing the temperature to 300°C at a rate of 7°C/min. Afterwards heating was continued to 330°C with 20°C/min. Helium was used as a carrier gas with a flow rate of 1 ml/min and electron impact was recorded at 70 eV. The MS data was recorded from 50 to 450 m/z in parallel to the performed temperature ramp.

### Isolation of genomic DNA

Genomic DNA of each line was extracted from around 50-100 mg leave material with the Qiagen DNeasy plant (Hilden, Germany) or the NucleoSpin Plant II Kit from Machery-Nagel (Düren, Germany) according to the manufacturer's specifications. The concentration was determined via the PeqLab NanoDrop 1000 spectrophotometer (Erlangen, Germany). An aliquot of the stock solution was then diluted to 10 ng/µL and 1 µL was then used for further PCR and qRT-PCR reactions.

### HRM mapping

The backcross lines were mapped with at least 12 different oligonucleotide markers designed to detect single nucleotide polymorphism (SNPs) between the parental wild and cultivated accessions using the annotated tomato genome for the cultivated tomato (Tomato-Genome-Consortium 2012) and the Solanum trichome EST library (McDowell, Kapteyn et al. 2011) plus internal unpublished RNA sequencing data for LA2167. The oligonucleotides were designed using primer 3 (Rozen and Skaletsky 2000) based tools and synthesized by Eurofins Genomics (Ebersberg, Germany). High-resolution melt curves were recorded in 96 well format with my-Budget 5x EvaGreen QPCR Mix II from Bio&Sell (Feucht, Germany) using a Bio-Rad Connect 96x Real-Time PCR system (München, Germany). The data was analyzed with the Bio-Rad Precision Melt Analysis Software V1.1.

### IPLEX SNP genotyping mapping

For 99 markers an iPlex Gold multiplex genotyping assay was performed by ATLAS Biolabs (Berlin, Germany).

### RNA-Isolation

RNA-isolation of leave and trichome material from tomato was done using the RNeasy Mini Kit. The RNA quality was controlled with the QIAxcel Advanced system (both Qiagen) following the manufacturer's specifications.

### First strand cDNA synthesis

RNA reverse transcription into complementary DNA (cDNA) was performed following manufacturer's recommendations. After preparative DNase I Digest was done using the Ambion Kit (Life Technologies, Carlsbad, USA) around 1 µg of total RNA and 1 µL of oligo-18dT primer was used for cDNA synthesis. Nuclease-free H₂O was added to a final reaction volume of 12 µL. Then 4 µL 5 x reaction buffer, 1 uL Ribolock RNase inhibitor, 2 µL desoxynucleotides (dNTPs) and 1 µL of RevertAid H Minus-MuLV reverse transcriptase were added to a final volume of 20 µL. The mixture was then incubated at 42°C for 1 h and heated to 70°C for 10 min. The resulting cDNA was ready-to-use for PCR reaction and for qRT-PCR assays.

### Example 1: GC-MS analysis of S. habrochaites LA2167

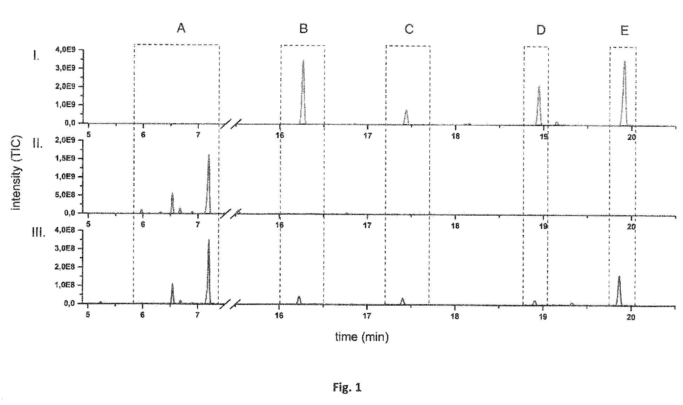

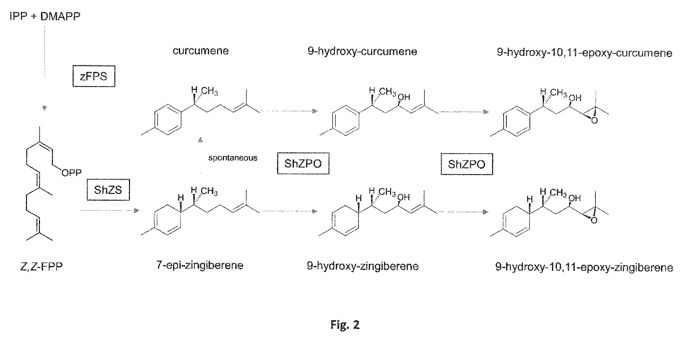

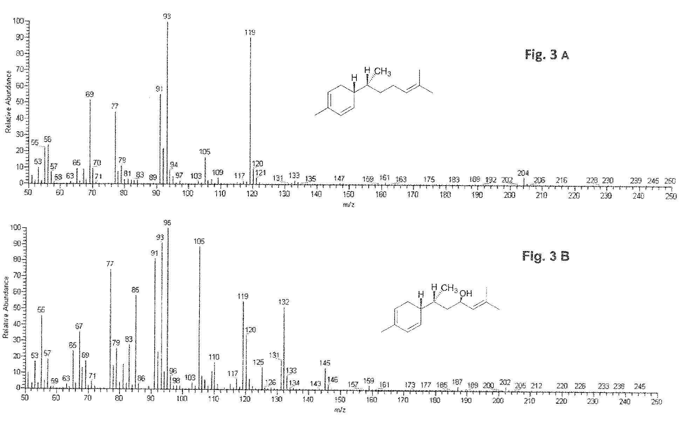

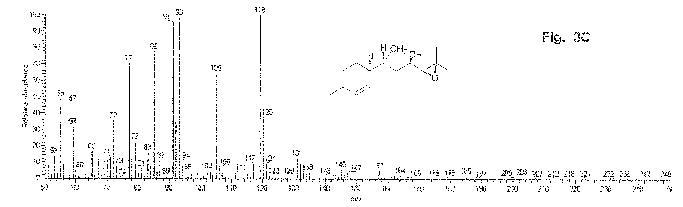

Leaf surface extracts of LA2167 were produced by dipping leaves in hexane and analyzed by GC-MS. In addition to zingiberene, the aromatic derivative of zingiberene, *ar-*curcumene can also be detected. Also, two major peaks corresponding to ZG9OH and ZG9OH10Epoxy can be easily detected. The corresponding oxidation products of *ar*curcumene could also be identified, namely 9-hydroxy-curcumene (C90H) and 9-hydroxy-(10,11)-epoxy-curcumene (C9OH10Epoxy) (Figure 1 and 2). Their mass spectra are shown in Figure 3.

### Example 2: Transcriptome analysis of glandular trichomes from LA2167

To identify potential candidate genes responsible for the oxidation of zingiberene and curcumene, we performed transcriptome analysis by next-generation RNA sequencing (Illumina) of several *S*. *habrochaites* accessions (LA1777, LA1731, LA1753, LA2650 and LA2167), of which only LA2167 produces zingiberene and its derivatives. Since cytochrome P450 monooxygenases are typically involved in terpene oxidation, we selected 5 P450 encoding genes whose expression was specifically upregulated in LA2167. These were Solyc01g008670, Solyc02g090340, Solyc03g111940, Solyc06g0662490 and Solyc06g0066280 (see Table 2). For the sake of clarity, we name the corresponding allele from *S*. *habrochaites* of those genes with the Sohab prefix. These results were confirmed by quantitative RT-PCR (Figure 3). Of these genes Sohab01g008670 was the most promising candidate with a more than 40-fold over-expression in LA2167 compared to all other lines.

**Table 2: P450 encoding genes with the highest differential expression levels in LA2167. The column "number of reads" gives the ratio of expression in LA2167 compared to the average of the other lines.**

| attributes | Human-readable description | number of reads* | CYP Clan | Closest homolog | Function | # |
|---|---|---|---|---|---|---|
| Solyc01g008670.2.1 | Cytochrome P450 | 40x | CYP71 | CYP71 D55 (Hyoasciamus muticus) | Premnaspirodiene oxygenase | P450-1 |
| Solyc02g090340.2.1 | Cytochrome P450 | 10x | CYP76 | CYP76B6 (Catharanthus roseus) | geraniol 10-hydroxylase | P450-2 |
| Solyc03g111940.2.1 | Cytochrome P450 | 10x | CYP71 | CYP71A4 (solanum melongena) | unknown | P450-3 |
| Solyc06g066240.2.1 | Cytochrome P450 | 5x | CYP71 | CYP71 D48v1 (Nicotiana tabacum) | unknown | P450-4 |
| Solyc06g066280.2.1 | Cytochrome P450 | 5x | CYP76 | CYP76C4-like (Vitis vinifera) | unknown | P450-5 |

### Example 3: Genetic analysis of the production of zingiberene derivatives

Pollen from LA2167 plants was used to fertilize emasculated flowers of *S*. *lycopersicum* LA4024. Seeds from the resulting fruits were collected and germinated. The putative F1 plants were verified by GC-MS analysis, since the presence of zingiberene and its derivatives is indicative of a successful cross (see Figure 1). F1 plants were then back-crossed to *S*. *lycopersicum* LA4024. 173 plants could then be recovered and characterized. To ascertain that these plants were indeed derived from a cross between the F1 plants and *S*. *lycopersicum,* the plants were genotyped for 8 independent markers by high resolution melt curves. The probability that a back-cross plant has all markers from the cultivated parent is 3.9/1000. After this screening, 149 plants remained which were analyzed by GC-MS (see Table 3). 77 plants from 150 produced zingiberene, which is within the range of expectation for a single locus trait. Interestingly, all the lines that produced ZG9OH also produced ZG9OH10Epoxy indicating that either a single gene is sufficient for the two oxidations or that two genes are required but they are tightly linked. 36 lines produced the zingiberene derivatives indicating that the corresponding locus is independent (= unlinked) from the zingiberene locus. The BC1 population was then mapped for the best three P450 candidates identified by transcriptomics using high-resolution melding (HRM) markers. This analysis indicated a perfect linkage of the candidate of chromosome 1 (Sohab01g008670) with the presence of the zingiberene derivatives. These data confirmed that Sohab01g008670 was the best and most likely candidate.

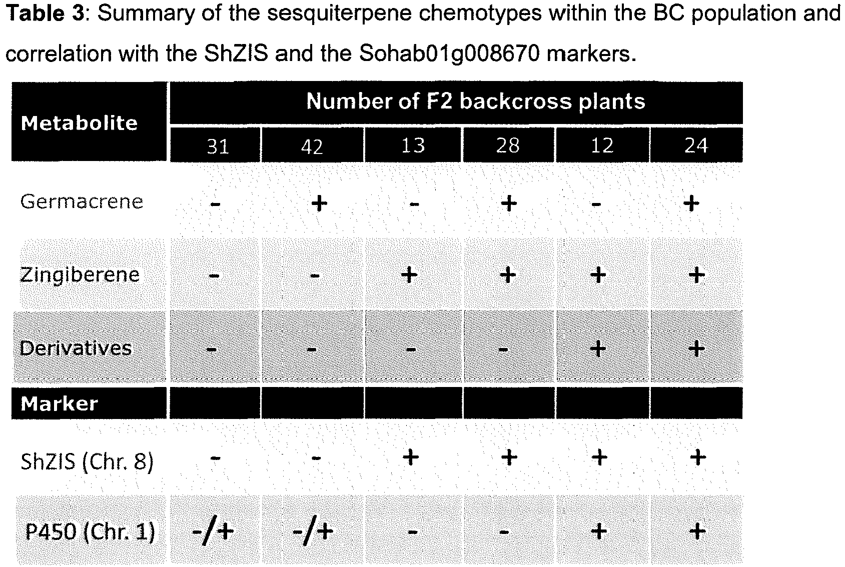

### Example 4: Transient expression in N. benthamiana

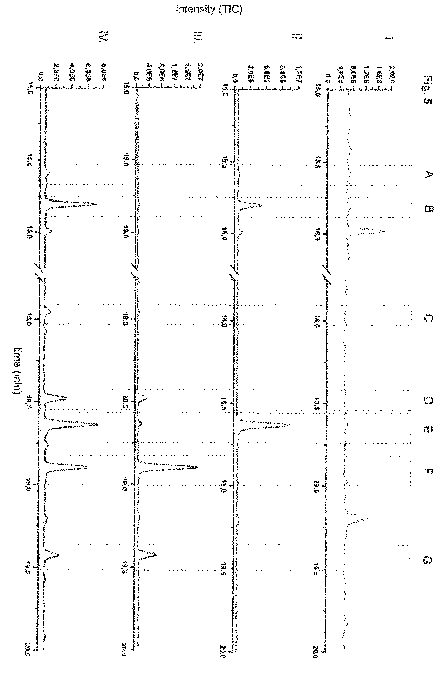

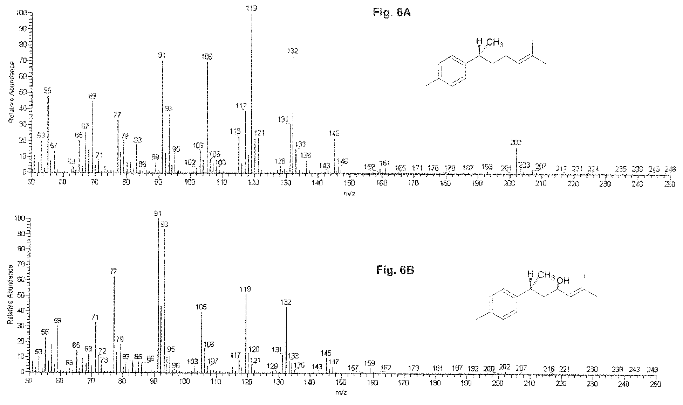

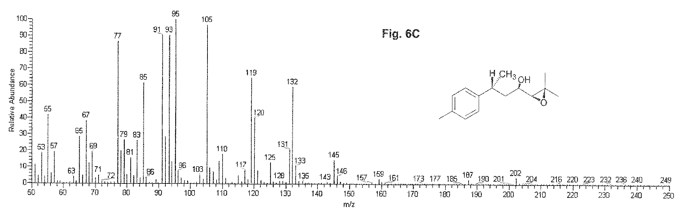

The cDNA for Sohab01g008670 was amplified, and its sequence verified. The sequence of the protein is provided as SEQ ID NO: 4. It was then cloned in a T-DNA vector under the control of the 35S promoter and with the Ocs terminator. In parallel, the genes for the biosynthesis of zingiberene, i.e. the Z,Z-FPP and synthase (zFPS) and the zingiberene synthase (ShZIS), were also cloned into a binary vector under the control of the 35S promoter. All these constructs were transformed into *Agrobacterium tumefaciens* and leaves of *N. benthamiana* were then infiltrated with combination of those genes. Infiltrated leaves of *N. benthamiana* were then either extracted with hexane or directly measured by headspace. The hexane extracts were analyzed by GC-MS as described above. For headspace measurements, infiltrated leaves were placed in 20 mL headspace vials (for example Sigma catalog number 27306). The headspace vials were then heated to 60°C for 15 min, and 1 mL of headspace was collected with a 2.5 mL syringe pre-heated to 80°C. The whole content of the syringe was then injected into the GC and the analysis proceeded then as for the liquid injection. The handling of the headspace vials and the collection of the headspace was operated on an automated PAL autosampler (from the company CTC Analytics) equipped with an headspace sampling option. As expected, co-expression of zFPS and ShZIS resulted in the production of zingiberene and small amounts curcumene. Co-expression of Sohab01g008670 resulted in the production of ZG9OH and ZG9OH10Epoxy. Two additional compounds could be detected. Based on their mass spectra and their retention times, they are 9-hydroxy-curcumene and 9-hydroxy-(10,11)-epoxy-curcumene (see Figures 5 and 6). Co-expression of the *Arabidopsis* P450 reductase (CPR) did not improve the yield of the products, indicating that the endogenous reductase activity of *N*. *benthamiana* was sufficient to support the reaction. Thus, Sohab01g008670 is sufficient to oxidize zingiberene and curcumene to their respective 9-hydroxyalcohol and 9-hydroxy-10,11-epoxide. Consequently, we name this enzyme zingiberene poly-oxidase (ZPO).

### Example 5: Expression in yeast

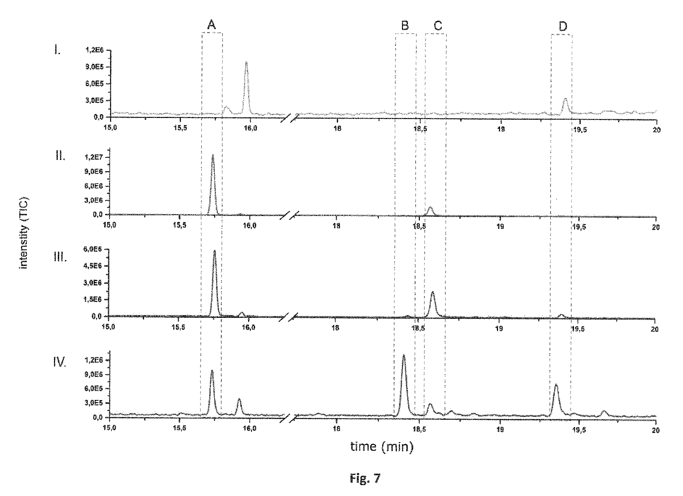

The cDNAs coding for zFPS, ShZIS and ZPO as well as the *Arabidopsis* cytochrome P450 reductase were cloned in a yeast expression vector with different GAL inducible promoters. Because zFPS and ShZIS are targeted to the chloroplasts, their transit peptide was removed for a better expression in yeast. The resulting proteins are named trc-zFPS (truncated zFPS) and trc-ShZIS (truncated ShZIS). The constructs were assembled in various combinations and transformed into yeast strain INVSc1 (Thermo Fisher). After induction with galactose, hexane extracts of the cultures were measured by GC-MS. As with *N. benthamiana,* expression of zFPS and ShZIS resulted in the production of zingiberene and small amounts of curcumene. Oxidation of zingiberene by ZPO required the co-expression of CPR since without it, no oxidation could be detected (Figure 7).

### Example 6: NMR based structure elucidation of 9-hydroxy-zingiberene and 10,11-epoxy-9-hydroxy-7-epizingiberene

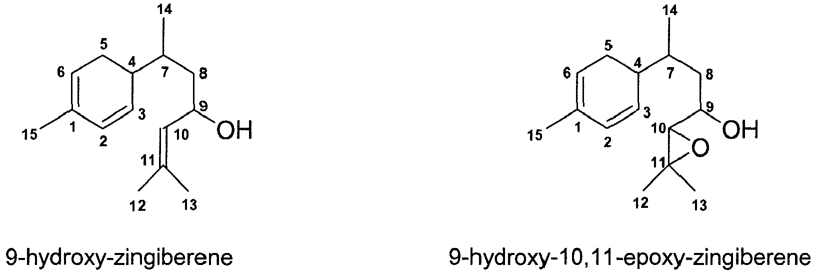

The structures of 9-hydroxy-zingiberene (ZG9OH) and 10,11-epoxy-9-hydroxy-zingiberene (ZG9OH10Epoxy) were elucidated on the basis of extensive 1D (¹H, ¹³C) and 2D (COSY, HSQC, HMBC,) NMR spectroscopic analysis. NMR spectra were recorded on an Agilent (Varian) VNMRS 600 NMR spectrometer at 599.832 MHz (1H, 2D) and 150.826 MHz (13C) using a 5 mm inverse detection cryoprobe. The samples were solved in C₆D₆. Chemical shifts were referenced to internal TMS (δ 1 H 0 ppm) and internal C₆D₆ (δ 13C 128.0 ppm). The ¹³C and DEPT spectra (Tab. 1) of ZG9OH exhibited signals for four methyl, two methylene and seven methine groups as well as two quaternary carbons. As can be seen from their low-field ¹³C chemical shifts, the quaternary (δ ¹³C 133.9, 131.2 ppm) and four of the tertiary carbons (δ ¹³C 129.8, 129.6, 128.8, 120.9 ppm) belong to double bond units. One of the remaining two methine groups shows a hydroxyl substituent (δ ¹³C 67.0 ppm; δ ¹H 4.356 ppm). Detailed analysis of ¹H,¹H COSY and ¹H,¹³C HMBC 2D spectra (Fig. 1, Table 4) and comparison with literature data (Ishii et al., 2011) revealed ZG9OH to be 9-hydroxy-zingiberene. Key ¹H, ¹³C HMBC (H to C) and ¹H,¹H COSY correlations of ZG9OH are shown in the following figure:

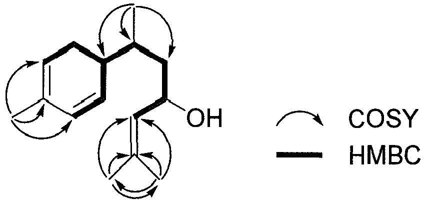

In comparison to ZG9OH, the molecular formula of ZG9OH10EPOXY of C₁₅H₂₄O₂, based on mass spectrometry analysis, indicated one additional oxygen atom but the same number of four double bond equivalents. The NMR spectra of ZG9OH10EPOXY largely resemble those of ZG9OH. However, the signals of CH-10 (δ ¹³C 128.9 ppm; δ ¹H 5.110 ppm) and C-11 (δ ¹³C 133.9 ppm) in ZG9OH are replaced for ZG9OH10EPOXY by signals at δ ¹³C 68.1 ppm; δ ¹H 2.558 ppm and δ ¹³C 58.9 ppm, respectively. The coupling constant of C-10/H-10 was extracted from the residual ¹J correlation peak in the HMBC spectrum. Its size of 170 Hz clearly shows CH-10 to be involved in an epoxy unit. The structure of ZG9OH10EPOXY was thus established as 9-hydroxy-10,11-epoxy-zingiberene.

**Table 4. NMR data of ZG9OH (solvent: C₆D₆)**

| Pos. | δ¹³C [ppm] | δ ¹H [ppm] m (J [Hz]) | HMBC to C |
|---|---|---|---|
| 1 | 131.2 | --- | |
| 2 | 128.8 | 5.829 ddd (9.7/3.4/1.7) | 3, 4, 6 |
| 3 | 129.6 | 5.684 dd (9.7/3.4) | 1, 2, 4, 5, 7 |
| 4 | 38.7 | 2.377 m | |
| 5 | 26.5 | 2.04^{a} | 1, 3, 4, 6, 7 |
| 6 | 120.9 | 5.427 m | 15 |
| 7 | 33.5 | 1.66^{a} | 3, 4, 5, 8, 14 |
| 8 | 42.3 | 1.553 ddd (13.4/7.2/5.2); 1.496 ddd (13.4/8.6/6.7) | 4, 7 ,9, 10, 14 |
| 9 | 67.0 | 4.356 ddd (8.7/7.2/6.7 | 11 |
| 10 | 129.8 | 5.110 d sept(8.7/1.4) | 12, 13 |
| 11 | 133.9 | ---- | |
| 12 | 25.7 | 1.557 d (1.4) | 10, 11, 13 |
| 13 | 18.0 | 1.475 d (1.4) | 10, 11, 12 |
| 14 | 17.6 | 0.910 d (6.8) | 4, 7, 8 |
| 15 | 21.2 | 1.663 br s | 1, 2, 6 |

| | | | |
|---|---|---|---|
| ^{a 1}H chemical shift extracted from the HSQC spectrum | | | |

**Table 5. NMR data of ZG9OH10EPOXY (solvent: C₆D₆)**

| Pos. | δ¹³C [ppm] | δ¹H [ppm] m (J [Hz]) | HMBC to C |
|---|---|---|---|
| 1 | 131.2 | --- | |
| 2 | 129.0 | 5.822 ddd (9.8/3.4/1.7) | 1, 4, 6, 15 |
| 3 | 129.0 | 5.618 dd (9.8/3.3) | 1, 4, 5, 6, 7 |
| 4 | 37.8 | 2.346 d | 3, 9 |
| 5 | 26.6 | 2.02^{a} | 1, 3, 4, 6, 7 |
| 6 | 121.0 | 5.419 m | 4, 5, 15 |
| 7 | 32.9 | 1.748 m | 4, 5, 8, 9, 14 |
| 8 | 38.4 | 1.476 ddd (14.0/6.6/5.2); 1.435 ddd (14.0/8.3/7.0) | 4, 7 ,9, 10, 14 |
| 9 | 69.0 | 3.442 ddd (8.3/7.8/5.2) | 7, 8, 10, 11 |
| 10 | 68.1 | 2.558 d (7.8) | 8, 9, 11, 12 |
| 11 | 58.9 | --- | |
| 12^{b} | 24.8 | 1.058 s | 10, 11, 13 |
| 13^{b} | 19.4 | 1.046 s | 10, 11, 12 |
| 14 | 18.2 | 0.906 d (6.9) | 4, 7, 8 |
| 15 | 21.2 | 1.663 br s | 1, 2, 6 |

| | | | |
|---|---|---|---|
| ^{a 1}H chemical shift extracted from the HSQC spectrum; ^{b} may be interchanged | | | |

### Polymorphisms and molecular markers

In an alignment of the cDNA sequences of Solyc01g008670 and Solhab01g008670 several polymorphisms can be identified (see Figure 6). We used one of these in our mapping (see above), other polymorphisms could also be used for the same purpose.

### NUCLEIC ACID AND PROTEIN SEQUENCES

SEQ ID NO: 1 Nucleotide sequence (cDNA) encoding zingiberene polyoxidase lacking membrane anchor (1398 bases): 
ATGTTGCCTCCTGGTCCATGGAAATTACCTTTAATTGGAAGCATGTTTCATTTGTTAGGTGGACTT
CCACATCATGTTTTTAGAGATTTAGCGAAAAAATATGGACCAATTATGCACCTTCAACTAGGTGAA
GTTTCTCTAGTTGTTATTACTTCTCCTGACATGGCCAAACAAATACTAAGAACTCATGATCTCGCT
TTTGCATCTAGGCCCAAGTTCCTAGTTGGGGAGATTCTCTTTTATAAGTGGACTGATATTCTTTTT
TCTCCCTATGGTGATTACTGGAAACAAATGCGTAAAATATGTCTATTGGAATTGCTTAGCGCAAAA
AATGTTAAGTCATTCAACTCTATACGACAAGATGAAGTTCATCATATGATTGAGTTTTTCGATCA
TCTTCTGGTGAAACAATTAATGTAACAAAAAGGATTTATCAATTCTCGAGCTCTATGACATGTAGA
TCAGCATTTGGGAAAGTATTCAAAGAGCAAAACAAACTCATAGAACTCGTTAAAGAAGCGTCAGG
CTTTTTTAAAGGATTTGATGTAGCTGATATGTTTCCATCACTAAAATTTCTTCATGTGTTATGTGGA
ATGAAGGGTAAAATTATGGATGCTCACCATGAGTTAGATGCCATTCTTGAAAACATCATCAATGA
GCACAAGAACAATGGTGAATTAGGAGGCGAAGGTTTACTTGTTGCATTGCTAAGATTAATGAAGG
 
AGGGAGGCTTTCAATTTCCAATCACCAATGACAATATCAAAGCTATTATTTTTGACATGTTTGCTG
GGGGAACAGATACTTCATCAACATTAATTGATTGGGCCATGGTGGAAATGATGAGGAATCCAAGT
GTACTTTTGAAAGCTCAAGTTGAGATAAGAAATGCTTTCACAGGAAAAGAAACTTTTAATGAAAAC
GATGTTGAAGAATTGAAATACCTTAAGTTGGTTGTTAAAGAAACTTTCAGACTCCATCCTCCAGGA
CCCCTTTTGATGGCAAGGGAATGTAGAGAAGAAGTAGACATAAATGGCTATAATATTCCTTTGAA
AACAAAAGTTATGGTTAATGCCTACGCAATCGGAAGAGATCCTAAATATTGGATTGACGCAGAAT
GTTTCAAACCTGAGAGATTTGAGCAGATTTCAGTAGATTTTATTGGCAATAACTTTGAATTTCTTC
CGTTTGGTAGTGGAAGGAGGAGTTGTCCGGGAATATCATTTGGTCTAGCTAATATTTTTTTCCCA
CTAGCTAAATTATTGTATCACTTTGATTGGAAACTTCCTATTGGAATCAATCCAAGTGATTTGGAC
TTAACTGAATCTGCTGGAATAACTTGTCCTAGAAAGAGTAACTTATACTTGATCGCGACTCCTCAT
GAACCTAGTCAAGAATGA
SEQ ID NO: 2: Solhab01g008670.2.1_translation zingiberene polyoxidase lacking membrane anchor (465 amino acid residues): 
MLPPGPWKLPLIGSMFHLLGGLPHHVFRDLAKKYGPIMHLQLGEVSLVVITSPDMAKQILRTHDLAFAS
RPKFLVGEILFYKWTDILFSPYGDYWKQMRKICLLELLSAKNVKSFNSIRQDEVHHMIEFFRSSSGETI
NVTKRIYQFSSSMTCRSAFGKVFKEQNKLIELVKEASGFFKGFDVADMFPSLKFLHVLCGMKGKIMDA
HHELDAILENIINEHKNNGELGGEGLLVALLRLMKEGGFQFPITNDNIKAIIFDMFAGGTDTSSTLIDWA
MVEMMRNPSVLLKAQVEIRNAFTGKETFNENDVEELKYLKLVVKETFRLHPPGPLLMARECREEVDIN
GYNIPLKTKVMVNAYAIGRDPKYWIDAECFKPERFEQISVDFIGNNFEFLPFGSGRRSCPGISFGLANIF
FPLAKLLYHFDWKLPIGINPSDLDLTESAGITCPRKSNLYLIATPHEPSQE
SEQ ID NO: 3: Solhab01g008670.2.1 encoding zingiberene polyoxidase (1488 bases): 
ATGCAGTTCTTCAACATCTTTTCCCTTTTCCTTTTATGTCTTTCCTCTTTTTGTTAATTAAATGGA
AGAATTCCAATAGCAAAACCAAAAGATTGCCTCCTGGTCCATGGAAATTACCTTTAATTGGAAGC
ATGTTTCATTTGTTAGGTGGACTTCCACATCATGTTTTTAGAGATTTAGCGAAAAAATATGGACCA
ATTATGCACCTTCAACTAGGTGAAGTTTCTCTAGTTGTTATT
ACTTCTCCTGACATGGCCAAACAAATACTAAGAACTCATGATCTCGCTTTTGCATCTAGGCCCAA
GTTCCTAGTTGGGGAGATTCTCTTTTATAAGTGGACTGATATTCTTTTTCTCCCTATGGTGATTA
CTGGAAACAAATGCGTAAAATATGTCTATTGGAATTGCTTAGCGCAAAAAATGTTAAGTCATTCAA
CTCTATACGACAAGATGAAGTTCATCATATGATTGAGTTTTTCGATCATCTTCTGGTGAAACAAT
TAATGTAACAAAAAGGATTTATCAATTCTCGAGCTCTATGACATGTAGATCAGCATTTGGGAAAGT
ATTCAAAGAGCAAAACAAACTCATAGAACTCGTTAAAGAAGCGTCAGGCTTTTTAAAGGATTTGA
TGTAGCTGATATGTTTCCATCACTAAAATTTCTTCATGTGTTATGTGGAATGAAGGGTAAAATTAT
GGATGCTCACCATGAGTTAGATGCCATTCTTGAAAACATCATCAATGAGCACAAGAACAATGGTG
AATTAGGAGGCGAAGGTTTACTTGTTGCATTGCTAAGATTAATGAAGGAGGGAGGCTTTCAATTT
CCAATCACCAATGACAATATCAAAGCTATTATTTTTGACATGTTTGCTGGGGGAACAGATACTTCA
TCAACATTAATTGATTGGGCCATGGTGGAAATGATGAGGAATCCAAGTGTACTTTTGAAAGCTCA
AGTTGAGATAAGAAATGCTTTCACAGGAAAAGAAACTTTTAATGAAAACGATGTTGAAGAATTGAA
ATACCTTAAGTTGGTTGTTAAAGAAACTTTCAGACTCCATCCTCCAGGACCCCTTTTGATGGCAA
 
GGGAATGTAGAGAAGAAGTAGACATAAATGGCTATAATATTCCTTTGAAAACAAAAGTTATGGTTA
ATGCCTACGCAATCGGAAGAGATCCTAAATATTGGATTGACGCAGAATGTTTCAAACCTGAGAGA
TTTGAGCAGATTTCAGTAGATTTTATTGGCAATAACTTTGAATTTCTTCCGTTTGGTAGTGGAAGG
AGGAGTTGTCCGGGAATATCATTTGGTCTAGCTAATATTTTTTTCCCACTAGCTAAATTATTGTAT
CACTTTGATTGGAAACTTCCTATTGGAATCAATCCAAGTGATTTGGACTTAACTGAATCTGCTGGA
ATAACTTGTCCTAGAAAGAGTAACTTATACTTGATCGCGACTCCTCATGAACCTAGTCAAGAATGA
SEQ ID NO: 4: Solhab01g008670.2.1_translation zingiberene polyoxidase (495 amino acid residues) 
MQFFNIFSLFLFMSFLFLLIKWKNSNSKTKRLPPGPWKLPLIGSMFHLLGGLPHHVFRDLAKKYGPIMH
LQLGEVSLVVITSPDMAKQILRTHDLAFASRPKFLVGEILFYKWTDILFSPYGDYWKQMRKICLLELLSA
KNVKSFNSIRQDEVHHMIEFFRSSSGETINVTKRIYQFSSSMTCRSAFGKVFKEQNKLIELVKEASGFF
KGFDVADMFPSLKFLHVLCGMKGKIMDAHHELDAILENIINEHKNNGELGGEGLLVALLRLMKEGGFQ
FPITNDNIKAIIFDMFAGGTDTSSTLIDWAMVEMMRNPSVLLKAQVEIRNAFTGKETFNENDVEELKYL
KLVVKETFRLHPPGPLLMARECREEVDINGYNIPLKTKVMVNAYAIGRDPKYWIDAECFKPERFEQISV
DFIGNNFEFLPFGSGRRSCPGISFGLANIFFPLAKLLYHFDWKLPIGINPSDLDLTESAGITCPRKSNLYL
IATPHEPSQE
SEQ ID NO: 5: coding sequence of Solyc01g008670 (Fig. 8).
SEQ ID NOs: 6 to 11: oligonucleotides (see Table 1).
SEQ ID NOs: 12 to 19: sequence of primers used for the cloning of synthetic galactose inducible promoters (Fig. 11).
SEQ ID NOs 20 to 36: nucleotide squences of the following plasmids: pAGM2925, pICH75721, pICH75716, pICH75603, pICH42100, pAGT864, pAGT865, pAGT866, pAGT529, pAGT537, pAGT538, pAGT539, pAGT540, pAGT1372, pAGT1041, pAGT938, pAGM1011, respectively.

### REFERENCES

Bleeker PM, Mirabella R, Diergaarde PJ, VanDoorn A, Tissier A, Kant MR, Prins M, de Vos M, Haring MA, Schuurink RC. 2012. Improved herbivore resistance in cultivated tomato with the sesquiterpene biosynthetic pathway from a wild relative. Proc Natl Acad Sci U S A, 109: 20124-9.
Borts, Rhona H. (1996) Yeast protocols: Methods in cell and molecular biology. Methods in Molecular Biology (Book 53) Humana Press.
Breeden DC, Coates RM. 1994. 7-Epizingiberene, a Novel Bisabolane Sesquiterpene from Wild Tomato Leaves. Tetrahedron, 50: 11123-11132.
Coates RM, Denissen JF, Juvik JA, Babka BA. 1988. Identification of Alpha-Santalenoic and Endo-Beta-Bergamotenoic Acids as Moth Oviposition Stimulants from Wild Tomato Leaves. Journal of Organic Chemistry, 53: 2186-2192.
Engler C, Youles M, Gruetzner R, Ehnert TM, Werner S, Jones JD, Patron NJ, Marillonnet S. 2014. A Golden Gate Modular Cloning Toolbox for Plants. ACS Synth Biol*,* Epub ahead of print.
Glas JJ, Schimmel BC, Alba JM, Escobar-Bravo R, Schuurink RC, Kant MR. 2012. Plant glandular trichomes as targets for breeding or engineering of resistance to herbivores. Int J Mol Sci, 13: 17077-103.
Gonzales-Vigil E, Hufnagel DE, Kim J, Last RL, Barry CS. 2012. Evolution of TPS20-related terpene synthases influences chemical diversity in the glandular trichomes of the wild tomato relative Solanum habrochaites. Plant Journal, 71: 921-35.
Ishii T, Matsuura H, Kaya K, Vairappan CS. 2011. A new bisabolane-type sesquiterpenoid from Curcuma domestica. Biochem. Syst. Ecol. 39, 864-867.
Jennewein, S., Rithner, C.D., Williams, R.M., and Croteau, R.B. (2001). Taxol biosynthesis: Taxane 13α-hydroxylase is a cytochrome P450-dependent monooxygenase. Proc. Natl. Acad. Sci. USA 98: 13595-13600**.**
Kumar S. 2010. Engineering cytochrome P450 biocatalysts for biotechnology, medicine and bioremediation. Expert Opin Drug Metab Toxicol. 2010 Feb;6(2):115-31.
Rasala and Mayfield (2015). Photosynthetic biomanufacturing in green algae; production of recombinant proteins for industrial, nutritional, and medical uses. PHOTOSYNTHESIS RESEARCH Volume: 123 Issue: 3, Special Issue: SI, pages: 227-239.
Scaife et al. (2015). Establishing Chlamydomonas reinhardtii as an industrial biotechnology host. The PLANT JOURNAL Volume: 82 Issue: 3, pages 532-546.
Schuurink R, Haring, M., Bleeker, P. 2012. PEST RESISTANT PLANTS. WO 2012/165961 A1.
van Der Hoeven RS, Monforte AJ, Breeden D, Tanksley SD, Steffens JC. 2000. Genetic control and evolution of sesquiterpene biosynthesis in Lycopersicon esculentum and L. hirsutum. Plant Cell, 12: 2283-94.
Scott, E.E., Spatzenegger, M., and Halpert, J.R. (2001). A truncation of 2B subfamily cytochromes P450 yields increased expression levels, increased solubility, and decreased aggregation while retaining function. Arch. Biochem. Biophys. 395: 57-68**.**
Swaminathan et al. 2009. CYP76M7 is an ent-cassadiene C11alpha-hydroxylase defining a second multifunctional diterpenoid biosynthetic gene cluster in rice. Plant Cell. 2009 Oct;21(10):3315-25.
Thompson JD, Higgins DG, Gibson TJ. (1994). CLUSTAL W: improving the sensitivity of progressive multiple sequence alignment through sequence weighting, position-specific gap penalties and weight matrix choice. Nucleic Acids Res., 22, 4673-4680.
Urban et al. 1997. J Biol Chem. 1997 Aug 1;272(31):19176-86
Urlacher 2004. Microbial P450 enzymes in biotechnology. Appl Microbiol Biotechnol. 64(3):317-25.
Weber E, Engler C, Gruetzner R, Werner S, Marillonnet S. 2011. A modular cloning system for standardized assembly of multigene constructs. PLoS One. 6:e16765.
Werner S, Engler C, Weber E, Gruetzner R, Marillonnet S. 2012. Fast track assembly of multigene constructs using Golden Gate cloning and the MoClo system. Bioeng Bugs. 3:38-43.

## Claims

1. A nucleic acid comprising
(i) a nucleotide sequence as defined in SEQ ID NO: 1, optionally with inserted intron(s);
(ii) a nucleotide sequence having a sequence identity of at least 85% to the nucleotide sequence as defined in SEQ ID NO: 1, optionally with inserted intron(s);
(iii) a nucleotide sequence encoding a protein comprising an amino acid sequence as defined in SEQ ID NO: 2;
(iv) a nucleotide sequence encoding a protein having an amino acid sequence identity to the amino acid sequence as defined in SEQ ID NO: 2 of at least 85%;
(v) a nucleotide sequence encoding a protein having an amino acid sequence similarity to the amino acid sequence as defined in SEQ ID NO: 2 of at least 90%;
(vi) a nucleotide sequence encoding a protein having from 1 to 30 amino acid substitutions, insertions or additions to the amino acid sequence as defined in SEQ ID NO: 2.

2. A nucleic acid comprising
(i) a nucleotide sequence as defined in SEQ ID NO: 1;
(ii) a nucleotide sequence having a sequence identity of at least 97% to the nucleotide sequence as defined in SEQ ID NO: 1;
(iii) a nucleotide sequence encoding a protein comprising an amino acid sequence as defined in SEQ ID NO: 2;
(iv) a nucleotide sequence encoding a protein having an amino acid sequence identity to the amino acid sequence as defined in SEQ ID NO: 2 of at least 94.5 %;
(v) a nucleotide sequence encoding a protein having an amino acid sequence similarity to the amino acid sequence as defined in SEQ ID NO: 2 of at least 97.0%;
(vi) a nucleotide sequence encoding a protein having from 1 to 25 amino acid substitutions to the amino acid sequence as defined in SEQ ID NO: 2, and/or having from 1 to 15 non-conservative amino acid substitutions or additions to the amino acid sequence as defined in SEQ ID NO: 2.

3. The nucleic acid according to claim 1 or 2, encoding a protein functional for the conversion of 7-epizingiberene to 9-hydroxy-7-epi-zingiberene.

4. A protein encoded by the nucleic acid of any one of claims 1 to 3 that is functional for the conversion of 7-epizingiberene to 9-hydroxy-7-epi-zingiberene.

5. A recombinant construct comprising the nucleic acid according to any one claims 1 to 3.

6. A vector or plasmid comprising the nucleic acid according to any one of claims 1 to 3, or comprising the recombinant construct according to claim 5.

7. A prokaryotic cell or a eukaryotic non-plant cell, cell line or strain comprising the nucleic acid according to any one of claims 1 to 3, such as a yeast cell, cell line or strain; or
a prokaryotic cell or a eukaryotic cell, cell line or strain, such as a yeast cell, cell line or strain, comprising the recombinant construct according to claim 5, or a vector or plasmid according to claim 5.

8. A process of generating a plant or plant cell capable of producing 9-hydroxy-zingiberene and/or 9-hydroxy-10,11-epoxy-zingiberene, comprising inserting the recombinant construct according to claim 5, or a vector or plasmid according to claim 6 into a plant or plant cell.

9. A process of generating a plant capable of producing 9-hydroxy-zingiberene and/or 9-hydroxy-10,11-epoxy-zingiberene, comprising crossing a plant (A) not capable of producing 9-hydroxy-zingiberene and/or 9-hydroxy-10,11-epoxy-zingiberene or producing insufficient amounts of 9-hydroxy-zingiberene and/or 9-hydroxy-10,11-epoxy-zingiberene with a plant (B) capable of producing 9-hydroxy-zingiberene and/or 9-hydroxy-10,11-epoxy-zingiberene, and selecting progeny capable of producing 9-hydroxy-zingiberene and/or 9-hydroxy-10,11-epoxy-zingiberene.

10. A plant, plant cell, or material of said plant, obtainable according to the process of claim 8; or
a plant or material of said plant comprising the recombinant construct according to claim 5, or a vector or plasmid according to claim 6; or
a plant or plant material thereof, obtainable according to the process of claims 9; or
a *Solanum lycopersicum* plant comprising a nucleic acid according to claim 2, said plant optionally further comprising a gene construct expressibly encoding a *Z,Z-*farnesyl diphosphate synthase and/or a gene expressibly encoding a 7-epi-zingiberene synthase.

11. A method of determining the capability of a plant for producing 9-hydroxy-zingiberene and/or 9-hydroxy-10,11-epoxy-zingiberene, comprising:
(a) obtaining a sample from said plant and analyzing said sample for the presence or absence of a nucleic acid as defined in claim 1 or 2; or
(b) obtaining a sample from said plant and analyzing said sample for the presence or absence, or the amount of, expression of an mRNA encoded by a nucleic acid as defined in claim 1 or 2; and/or
(c) obtaining a sample from said plant and analyzing said sample for its content of 9-hydroxy-zingiberene and/or 9-hydroxy-10,11-epoxy-zingiberene, and/or 9-hydroxy curcumene and/or 9-hydroxy-10,11-epoxy curcumene.

12. A method of selecting, from a set of plants, a plant having increased pest resistance compared to another plant or other plants in said set, comprising:
(a) analyzing a plurality of plants in said set of plants for the presence of a nucleic acid as defined in any one of claims 1 to 3, and selecting a plant or plants containingsaid nucleic acid; or
(b) analyzing a plurality of plants in said set of plants for 9-hydroxy-zingiberene and/or 9-hydroxy-10,11-epoxy-zingiberene, and selecting a plant or plants having higher content or production of 9-hydroxy-zingiberene and/or 9-hydroxy-10,11-epoxy-zingiberene than one or more other plants in said set; or
(c) analyzing a plurality of plants in said set of plants for one or more polymorphisms at any one or more of positions 59, 85, 168, 243, 247, 295, 307, 309, 337, 349, 408, 444, 445, 557, 590, 602, 616, 624, 708, 776, 804, 824, 904, 965, 1060, 1076, 1153, 1218, 1295, 1376, 1404, 1405, 1432, and/or 1447 of SEQ ID NO: 3, and selecting a plant or plants having one or more of the nucleotides of SEQ ID NO: 3 at any one or more of these positions of SEQ ID NO:3.

13. A zingiberene derivative selected from 9-hydroxy-zingiberene and 9-hydroxy-10,11-epoxy-zingiberene.

14. A method of increasing pest resistance of a plant, comprising spraying said plant or the vicinity where it grows with a composition comprising 9-hydroxy-zingiberene and/or 9-hydroxy-10,11-epoxy-zingiberene.

15. Pesticidal composition comprising a zingiberene derivative selected from 9-hydroxy-zingiberene and 9-hydroxy-10,11-epoxy-zingiberene.
